# EUROPÄISCHE PATENTSCHRIFT

(11) **EP 2 736 897 B1**
(45) Veröffentlichungstag und Bekanntmachung des Hinweises auf die Patenterteilung: **25.11.2015**
(21) Anmeldenummer: 12737842.0
(22) Anmeldetag: 24.07.2012
(51) Int. Cl.: C07D 401/04, C07D 403/04, C07D 405/04, C07D 409/04, C07D 413/04, C07D 417/04, A01N 43/40

(54) **SUBSTITUIERTE PICOLINSÄUREN UND PYRIMIDIN-4-CARBONSÄUREN, VERFAHREN ZU DEREN HERSTELLUNG SOWIE DEREN VERWENDUNG ALS HERBIZIDE UND PFLANZENWACHSTUMSREGULATOREN**
SUBSTITUTED PICOLINIC ACIDS AND PYRIMIDINE-4-CARBOXYLIC ACIDS, METHOD FOR THE PRODUCTION THEREOF, AND USE THEREOF AS HERBICIDES AND PLANT GROWTH REGULATORS
ACIDES PICOLINIQUES ET ACIDES PYRIMIDIN-4-CARBOXYLIQUES SUBSTITUÉS, LEUR PROCÉDÉ DE PRODUCTION ET LEUR UTILISATION COMME HERBICIDES ET RÉGULATEURS DE CROISSANCE VÉGÉTALE

(30) Priorität: 27.07.2011 EP 11175511
(43) Veröffentlichungstag der Anmeldung: 04.06.2014
(73) Patentinhaber: Bayer Intellectual Property GmbH, 40789 Monheim (DE)
(72) Erfinder: HOFFMANN, Michael Gerhard, 65439 Flörsheim (DE); BRÜNJES, Marco, 65719 Hofheim (DE); DÖLLER, Uwe, 63110 Rodgau (DE); DIETRICH, Hans-Jörg, 65835 Liederbach am Taunus (DE); HÄUSER-HAHN, Isolde, 51375 Leverkusen (DE); ROSINGER, Christopher Hugh, 65719 Hofheim (DE); GATZWEILER, Elmar, 61231 Bad Nauheim (DE); HEINEMANN, Ines, 65719 Hofheim (DE)
(74) Vertreter: BIP Patents
(86) Internationale Anmeldenummer: PCT/EP2012/064519
(87) Internationale Veröffentlichungsnummer: WO 2013/014165

(56) Entgegenhaltungen:
- WO-A1-03/011853
- WO-A2-2009/138712
- WO-A2-2010/060581

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bekannt, dass substituierte Picolinsäurederivate und Pyrimidin-4-carbonsäurederivate herbizide Eigenschaften aufweisen: In WO 2003/011853 A1 werden poly-substituierte 6-Phenylpicolinsäurederivate mit herbizider Wirkung beschrieben. WO2009/029735 A1 und WO2010/125332 A1 beschreiben herbizide Wirkungen für polysubstituierte 2-Phenyl-4-pyrimidin-carbonsäurederivate. Heteroaromatisch-substituierte Picolin- und Pyrimidincarbonsäuren mit herbiziden Eigenschaften werden in WO 2009/138712 A2 offenbart. In WO 2007/080382 A1 und WO 2009/007751 A2 werden heteroaromatisch-substituierte Picolin- und Pyrimidincarbonsäuren mit pharmakologischen Wirkungen beschrieben.

Die dort beschriebenen Verbindungen zeigen jedoch häufig eine nicht ausreichende herbizide Wirksamkeit und/oder eine nicht ausreichende Selektivität in Nutzpflanzenkulturen.

Es wurden substituierte Picolinsäuren und Pyrimidin-4-carbonsäuren gefunden, die besonders gut als Herbizide geeignet sind.

Ein Gegenstand der vorliegenden Erfindung sind Picolinsäuren und Pyrimidin-4-carbonsäuren der allgemeinen Formel (I), deren N-Oxide oder deren Salze 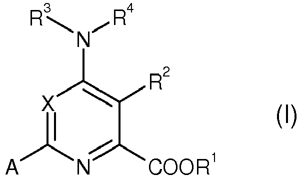 worin
- A: bedeutet einen Rest der Gruppe bestehend aus A1 bis A20, 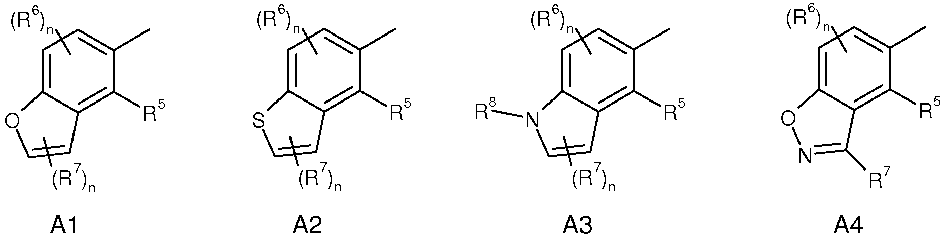 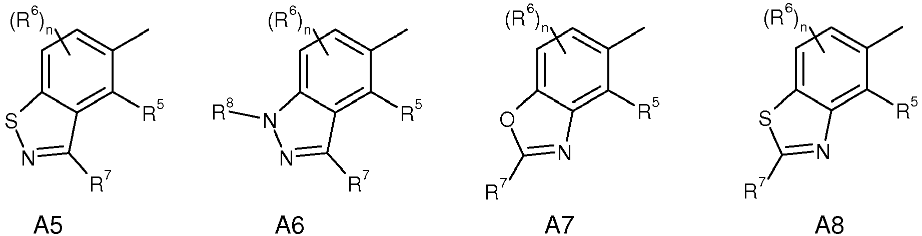 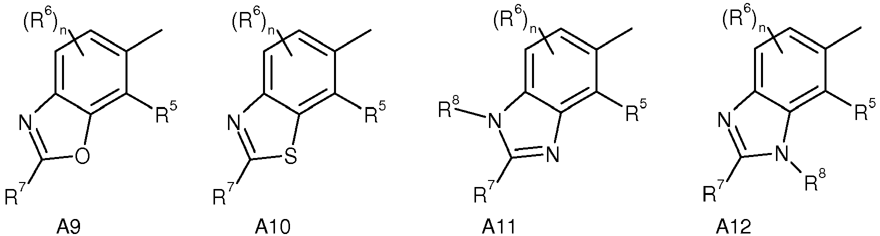 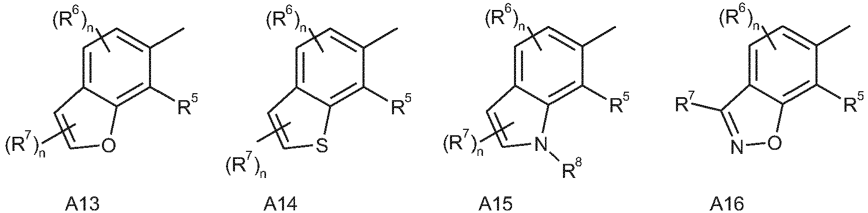 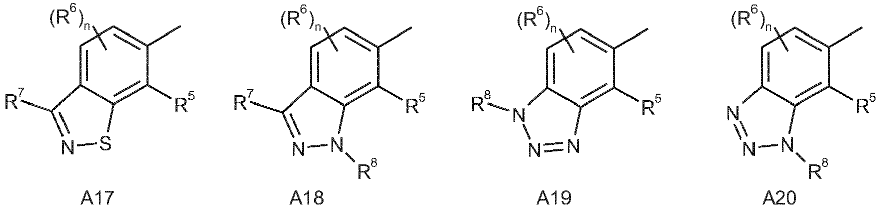
- R¹: bedeutet Wasserstoff oder (C₁-C₄)-Alkyl,
- R²: bedeutet Chlor,
- R³: bedeutet Wasserstoff,
- R⁴: bedeutet Wasserstoff,
- R⁵: bedeutet Wasserstoff, Halogen, OH, NH₂, CN, (C₁-C₃)-Alkyl, (C₁-C₃)-Alkoxy, C₁-C₃)-Alkylamino oder Cyclopropyl,
- R⁶: bedeutet Wasserstoff, Halogen, OH, NH₂, CN, (C₁-C₃)-Alkyl, (C₁-C₃)-Alkoxy, Cyclopropyl oder Vinyl,
- R⁷: bedeutet Wasserstoff, Halogen, (C₁-C₃)-Alkyl, (C₁-C₃)-Alkoxy, (C₁-C₃)-Alkylthio, Cyclopropyl, (C₁-C₃)-Alkylamino oder Phenyl,
- R⁸: bedeutet Wasserstoff, (C₁-C₃)-Alkyl, Phenyl oder (C₁-C₃)-Alkylcarbonyl,
- X: bedeutet N, CH, CCI, CF oder CBr,
- n: bedeutet 0, 1 oder 2.

Alkyl bedeutet gesättigte, geradkettige oder verzweigte Kohlenwasserstoffreste mit 1 bis 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methyl-propyl.

Halogenalkyl bedeutet geradkettige oder verzweigte Alkylgruppen mit 1 bis 8 Kohlenstoffatomen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit 2 bis 8 Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy; Halogenalkoxy bedeutet geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy.

Alkylthio bedeutet gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
Halogenalkylthio bedeutet geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio.

Aryl bedeutet Phenyl oder Naphthyl.

Die Verbindungen der Formel (I) können, auch in Abhängigkeit von der Art der Substituenten, als geometrische und/oder optische Isomere oder Isomeren-gemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ein Metallionäquivalent bedeutet ein Metallion mit einer positiven Ladung wie Na⁺, K⁺, (Mg²⁺)_{1/2}, (Ca²⁺)_{1/2}, MgH⁺, CaH⁺, (Al³⁺)_{1/3} (Fe²⁺)_{1/2} oder (Fe³⁺)_{1/3}.

Halogen bedeutet Fluor, Chlor, Brom und Jod.

Ist eine Gruppe mehrfach durch Reste substituiert, so ist darunter zu verstehen, daß diese Gruppe durch ein oder mehrere gleiche oder verschiedene der genannten Reste substituiert ist.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.

Beispiele für anorganische Säuren sind Halogenwasserstoffsäuren wie Fluorwasserstoff, Chlorwasserstoff, Bromwasserstoff und lodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.
Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Die Verbindungen der allgemeinen Formel (I) können je nach Art und Verknüpfung der Substituenten als Stereoisomere vorliegen. Sind beispielsweise ein oder mehrere asymmetrisch substiuierte Kohlenstoffatome oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

In allen nachfolgend genannten Formeln haben die Substituenten und Symbole, sofern nicht anders definiert, dieselbe Bedeutung wie unter Formel (I) beschrieben.

Verbindungen der Formel (I), können beispielsweise gemäß nachfolgendem Schema durch palladium-katalysierte Kupplungsreaktion einer Halogenverbindung (II) mit einem Boronsäure-Derivat (III) hergestellt werden. In diesem Schema steht Het für die an den Phenylring ankondensierten Heterocyclen der Gruppen A1 bis A24. R steht für Wasserstoff oder für (C₁-C₃)-Alkyl.

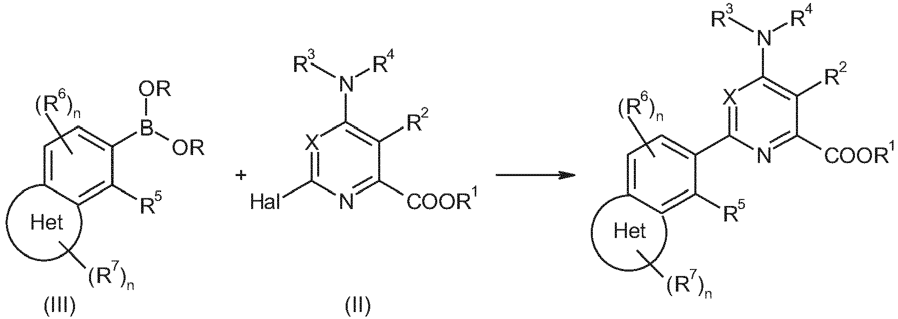

Die Halogenverbindungen der Formel (II) sind beispielsweise aus WO2001051468 A1 und WO2007082076 A1 bekannt, oder können nach dem Fachmann an sich bekannten Methoden hergestellt werden. Die Boronsäure-Derivate der Formel (III) sind kommerziell erhältlich oder können nach dem Fachmann an sich bekannten Methoden hergestellt werden.

Bevorzugt sind die in den Tabellen 1 bis 44 genannten Verbindungen der Formel (I), die analog der hier genannten Methoden hergestellt werden können. Die verwendeten Abkürzungen bedeuten:

| | | | |
|---|---|---|---|
| Et = Ethyl | Me = Methyl | Pr = Propyl | Ph = Phenyl |

**Tabelle 1: Erfindungsgemäße Verbindungen der Formel (I), worin X für CH, A für A1, R³ und R⁴ jeweils für Wasserstoff sowie R² für Chlor steht:**

| | | | | | | |
|---|---|---|---|---|---|---|
| 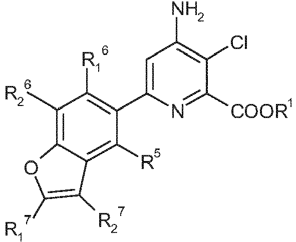 | | | | | | |

| Bsp. Nr. | R¹ | R⁵ | R₁⁶ | R₂⁶ | R₁⁷ | R₂⁷ |
|---|---|---|---|---|---|---|
| 1.001 | H | H | H | H | H | H |
| 1.002 | H | F | H | H | H | H |
| 1.003 | H | F | H | H | Cl | H |
| 1.004 | H | F | H | H | H | Cl |
| 1.005 | H | F | H | H | Cl | Cl |
| 1.006 | H | F | H | H | Ph | H |
| 1.007 | H | F | H | H | Ph | Cl |
| 1.008 | H | F | H | H | Ph | F |
| 1.009 | H | F | H | H | Me | H |
| 1.010 | H | F | H | H | Me | Cl |
| 1.011 | H | F | H | H | Me | F |
| 1.012 | Me | H | H | H | H | H |
| 1.013 | Me | F | H | H | H | H |
| 1.014 | Me | F | H | H | Cl | H |
| 1.015 | Me | F | H | H | H | Cl |
| 1.016 | Me | F | H | H | Cl | Cl |
| 1.017 | Me | F | H | H | H | F |
| 1.018 | Me | F | H | H | Ph | H |
| 1.019 | Me | F | H | H | Ph | Cl |
| 1.020 | Me | F | H | H | Ph | F |
| 1.021 | Me | F | H | H | Me | H |
| 1.022 | Me | F | H | H | Me | Cl |
| 1.023 | Me | F | H | H | Me | F |
| 1.024 | Et | H | H | H | H | H |
| 1.025 | Et | Cl | H | H | H | H |
| 1.026 | Et | Cl | H | H | Cl | H |
| 1.027 | Et | Cl | H | H | H | Cl |
| 1.028 | Et | Cl | H | H | Cl | Cl |
| 1.029 | Et | Cl | H | H | H | F |
| 1.030 | Et | Cl | H | H | Ph | H |
| 1.031 | Et | Cl | H | H | Ph | Cl |
| 1.032 | Et | Cl | H | H | Ph | F |
| 1.033 | Et | Cl | H | H | Me | H |
| 1.034 | Et | Cl | H | H | Me | Cl |
| 1.035 | Et | Cl | H | H | Me | F |
| 1.036 | H | F | F | H | H | H |
| 1.037 | H | F | H | F | H | H |
| 1.038 | Me | H | F | H | H | H |
| 1.039 | H | H | F | H | H | H |
| 1.040 | K | H | F | H | H | H |
| 1.041 | Me | H | Cl | H | H | H |
| 1.042 | H | H | Cl | H | H | H |
| 1.043 | K | H | Cl | H | H | H |
| 1.044 | Me | H | H | F | H | H |
| 1.045 | H | H | H | F | H | H |
| 1.046 | K | H | H | F | H | H |
| 1.047 | Me | H | H | Cl | H | H |
| 1.048 | H | H | H | Cl | H | H |
| 1.049 | K | H | H | Cl | H | H |
| 1.050 | K | F | H | H | H | H |
| 1.051 | Me | Cl | H | H | H | H |
| 1.052 | H | Cl | H | H | H | H |
| 1.053 | Na | Cl | H | H | H | H |

**Tabelle 2: Erfindungsgemäße Verbindungen der Formel (I), worin X für CH, A für A2, R³ und R⁴ jeweils für Wasserstoff sowie R² für Chlor steht:**

| | | | | | | |
|---|---|---|---|---|---|---|
| 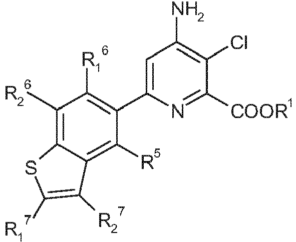 | | | | | | |

| Bsp. Nr. | R¹ | R⁵ | R₁⁶ | R₂⁶ | R₁⁷ | R₂⁷ |
|---|---|---|---|---|---|---|
| 2.001 | H | H | H | H | H | H |
| 2.002 | H | F | H | H | H | H |
| 2.003 | H | F | H | H | Cl | H |
| 2.004 | H | F | H | H | H | Cl |
| 2.005 | H | F | H | H | Cl | Cl |
| 2.006 | H | F | H | H | Ph | H |
| 2.007 | H | F | H | H | Ph | Cl |
| 2.008 | H | F | H | H | Ph | F |
| 2.009 | H | F | H | H | Me | H |
| 2.010 | H | F | H | H | Me | Cl |
| 2.011 | H | F | H | H | Me | F |
| 2.012 | Me | H | H | H | H | H |
| 2.013 | Me | F | H | H | H | H |
| 2.014 | Me | F | H | H | Cl | H |
| 2.015 | Me | F | H | H | H | Cl |
| 2.016 | Me | F | H | H | Cl | Cl |
| 2.017 | Me | F | H | H | H | F |
| 2.018 | Me | F | H | H | Ph | H |
| 2.019 | Me | F | H | H | Ph | Cl |
| 2.020 | Me | F | H | H | Ph | F |
| 2.021 | Me | F | H | H | Me | H |
| 2.022 | Me | F | H | H | Me | Cl |
| 2.023 | Me | F | H | H | Me | F |
| 2.024 | Et | H | H | H | H | H |
| 2.025 | Et | Cl | H | H | H | H |
| 2.026 | Et | Cl | H | H | Cl | H |
| 2.027 | Et | Cl | H | H | H | Cl |
| 2.028 | Et | Cl | H | H | Cl | Cl |
| 2.029 | Et | Cl | H | H | H | F |
| 2.030 | Et | Cl | H | H | Ph | H |
| 2.031 | Et | Cl | H | H | Ph | Cl |
| 2.032 | Et | Cl | H | H | Ph | F |
| 2.033 | Et | Cl | H | H | Me | H |
| 2.034 | Et | Cl | H | H | Me | Cl |
| 2.035 | Et | Cl | H | H | Me | F |
| 2.036 | H | F | F | H | H | H |
| 2.037 | H | F | H | F | H | H |
| 2.038 | Me | H | F | H | H | H |
| 2.039 | H | H | F | H | H | H |
| 2.040 | K | H | F | H | H | H |
| 2.041 | Me | H | Cl | H | H | H |
| 2.042 | H | H | Cl | H | H | H |
| 2.043 | K | H | Cl | H | H | H |
| 2.044 | Me | H | H | F | H | H |
| 2.045 | H | H | H | F | H | H |
| 2.046 | K | H | H | F | H | H |
| 2.047 | Me | H | H | Cl | H | H |
| 2.048 | H | H | H | Cl | H | H |
| 2.049 | K | H | H | Cl | H | H |
| 2.050 | K | F | H | H | H | H |
| 2.051 | Me | Cl | H | H | H | H |
| 2.052 | H | Cl | H | H | H | H |
| 2.053 | K | Cl | H | H | H | H |

**Tabelle 3: Erfindungsgemäße Verbindungen der Formel (I), worin X für CH, A für A3, R³ und R⁴ jeweils für Wasserstoff sowie R² für Chlor steht:**

| | | | | | | | |
|---|---|---|---|---|---|---|---|
| 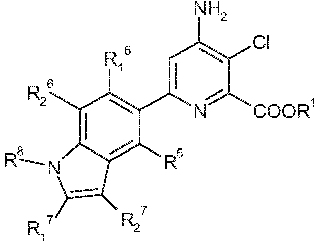 | | | | | | | |

| Bsp. Nr. | R¹ | R⁵ | R₁⁶ | R₂⁶ | R₁⁷ | R₂⁷ | R⁸ |
|---|---|---|---|---|---|---|---|
| 3.001 | H | H | H | H | H | H | H |
| 3.002 | H | F | H | H | H | H | Me |
| 3.003 | H | F | H | H | Cl | H | H |
| 3.004 | H | F | H | H | H | Cl | Me |
| 3.005 | H | F | H | H | Cl | Cl | Me |
| 3.006 | H | F | H | H | Ph | H | Me |
| 3.007 | H | F | H | H | Ph | Cl | H |
| 3.008 | H | F | H | H | Ph | F | H |
| 3.009 | H | F | H | H | Me | H | H |
| 3.010 | H | F | H | H | Me | Cl | Me |
| 3.011 | H | F | H | H | Me | F | Me |
| 3.012 | Me | H | H | H | H | H | Me |
| 3.013 | Me | F | H | H | H | H | H |
| 3.014 | Me | F | H | H | Cl | H | H |
| 3.015 | Me | F | H | H | H | Cl | Me |
| 3.016 | Me | F | H | H | Cl | Cl | Me |
| 3.017 | Me | F | H | H | H | F | Me |
| 3.018 | Me | F | H | H | Ph | H | H |
| 3.019 | Me | F | H | H | Ph | Cl | H |
| 3.020 | Me | F | H | H | Ph | F | H |
| 3.021 | Me | F | H | H | Me | H | H |
| 3.022 | Me | F | H | H | Me | Cl | Me |
| 3.023 | Me | F | H | H | Me | F | H |
| 3.024 | Et | H | H | H | H | H | Me |
| 3.025 | Et | Cl | H | H | H | H | Me |
| 3.026 | Et | Cl | H | H | Cl | H | Me |
| 3.027 | Et | Cl | H | H | H | Cl | Me |
| 3.028 | Et | Cl | H | H | Cl | Cl | Me |
| 3.029 | Et | Cl | H | H | H | F | Me |
| 3.030 | Et | Cl | H | H | Ph | H | H |
| 3.031 | Et | Cl | H | H | Ph | Cl | H |
| 3.032 | Et | Cl | H | H | Ph | F | H |
| 3.033 | Et | Cl | H | H | Me | H | H |
| 3.034 | Et | Cl | H | H | Me | Cl | H |
| 3.035 | Et | Cl | H | H | Me | F | Me |
| 3.036 | H | F | F | H | H | H | H |
| 3.037 | H | F | H | F | H | H | H |

**Tabelle 4: Erfindungsgemäße Verbindungen der Formel (I), worin X für CH, A für A4, R³ und R⁴ jeweils für Wasserstoff sowie R² für Chlor steht:**

| | | | | | |
|---|---|---|---|---|---|
| 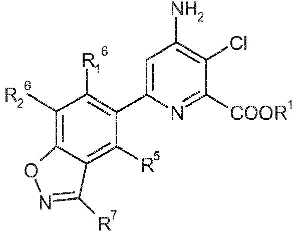 | | | | | |

| Bsp. Nr. | R¹ | R⁵ | R₁⁶ | R₂⁶ | R⁷ |
|---|---|---|---|---|---|
| 4.001 | H | H | H | H | H |
| 4.002 | H | F | H | H | H |
| 4.003 | H | F | H | H | F |
| 4.004 | H | F | H | H | Cl |
| 4.005 | H | F | H | H | Me |
| 4.006 | H | Cl | H | H | H |
| 4.007 | H | Cl | H | H | F |
| 4.008 | H | Cl | H | H | Cl |
| 4.009 | H | Cl | H | H | Me |
| 4.010 | Me | H | H | H | H |
| 4.011 | Me | F | H | H | H |
| 4.012 | Me | F | H | H | F |
| 4.013 | Me | F | H | H | Cl |
| 4.014 | Me | Cl | H | H | H |
| 4.015 | Me | Cl | H | H | F |
| 4.016 | Me | Cl | H | H | Cl |
| 4.017 | Me | Cl | H | H | Me |
| 4.018 | Et | H | H | H | H |
| 4.019 | Et | F | H | H | H |
| 4.020 | Et | Cl | H | H | H |
| 4.021 | Et | F | H | H | Me |
| 4.022 | Et | Cl | H | H | Me |
| 4.023 | H | F | F | H | H |
| 4.024 | H | F | H | F | H |

**Tabelle 5: Erfindungsgemäße Verbindungen der Formel (I), worin X für CH, A für A5, R³ und R⁴ jeweils für Wasserstoff sowie R² für Chlor steht:**

| | | | | | |
|---|---|---|---|---|---|
| 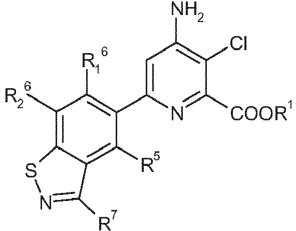 | | | | | |

| Bsp. Nr. | R¹ | R⁵ | R₁⁶ | R₂⁶ | R⁷ |
|---|---|---|---|---|---|
| 5.001 | H | H | H | H | H |
| 5.002 | H | F | H | H | H |
| 5.003 | H | F | H | H | F |
| 5.004 | H | F | H | H | Cl |
| 5.005 | H | F | H | H | F |
| 5.006 | H | Cl | H | H | H |
| 5.007 | H | Cl | H | H | F |
| 5.008 | H | Cl | H | H | Cl |
| 5.009 | H | Cl | H | H | Me |
| 5.010 | Me | H | H | H | H |
| 5.011 | Me | F | H | H | H |
| 5.012 | Me | F | H | H | Cl |
| 5.013 | Me | Cl | H | H | H |
| 5.014 | Me | Cl | H | H | F |
| 5.015 | Me | Cl | H | H | Cl |
| 5.016 | Me | Cl | H | H | Me |
| 5.017 | Et | H | H | H | H |
| 5.018 | Et | F | H | H | H |
| 5.019 | Et | Cl | H | H | H |
| 5.020 | Et | F | H | H | Me |
| 5.021 | Et | Cl | H | H | Me |
| 5.022 | H | F | F | H | H |
| 5.023 | H | F | H | F | H |

**Tabelle 6: Erfindungsgemäße Verbindungen der Formel (I), worin X für CH, A für A6, R⁸, R³ und R⁴ jeweils für Wasserstoff sowie R² für Chlor steht:**

| | | | | | |
|---|---|---|---|---|---|
| 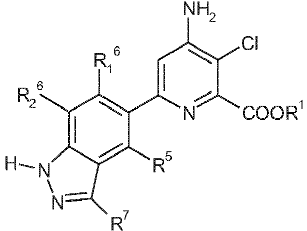 | | | | | |

| Bsp. Nr. | R¹ | R⁵ | R₁⁶ | R₂⁶ | R⁷ |
|---|---|---|---|---|---|
| 6.001 | H | H | H | H | H |
| 6.002 | H | F | H | H | H |
| 6.003 | H | F | H | H | Me |
| 6.004 | H | F | H | H | Et |
| 6.005 | H | H | H | H | Me |
| 6.006 | H | Cl | H | H | H |
| 6.007 | H | Cl | H | H | Me |
| 6.008 | H | Cl | H | H | Et |
| 6.009 | Me | H | H | H | H |
| 6.010 | Me | H | H | H | Me |
| 6.011 | Me | F | H | H | H |
| 6.012 | Me | F | H | H | Me |
| 6.013 | Me | F | H | H | Et |
| 6.014 | Me | Cl | H | H | H |
| 6.015 | Me | Cl | H | H | Me |
| 6.016 | Me | Cl | H | H | Et |
| 6.017 | Me | H | H | H | Me |
| 6.018 | Et | H | H | H | H |
| 6.019 | Et | H | H | H | Me |
| 6.020 | Et | H | H | H | Et |
| 6.021 | Et | F | H | H | H |
| 6.022 | Et | F | H | H | Me |
| 6.023 | Et | F | H | H | Et |
| 6.024 | Et | Cl | H | H | H |
| 6.025 | Et | Cl | H | H | Me |
| 6.026 | Et | Cl | H | H | Et |
| 6.027 | H | F | F | H | H |
| 6.028 | H | F | H | F | H |

**Tabelle 7: Erfindungsgemäße Verbindungen der Formel (I), worin X für CH, A für A6, R³ und R⁴ jeweils für Wasserstoff, R⁸ für Methyl sowie R² für Chlor steht:**

| | | | | | |
|---|---|---|---|---|---|
| 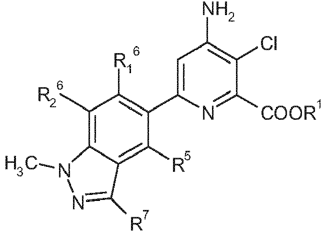 | | | | | |

| Bsp. Nr. | R¹ | R⁵ | R₁⁶ | R₂⁶ | R⁷ |
|---|---|---|---|---|---|
| 7.001 | H | H | H | H | H |
| 7.002 | H | F | H | H | H |
| 7.003 | H | F | H | H | Me |
| 7.004 | H | F | H | H | Et |
| 7.005 | H | H | H | H | Me |
| 7.006 | H | Cl | H | H | H |
| 7.007 | H | Cl | H | H | Me |
| 7.008 | H | Cl | H | H | Et |
| 7.009 | Me | H | H | H | H |
| 7.010 | Me | H | H | H | Me |
| 7.011 | Me | F | H | H | H |
| 7.012 | Me | F | H | H | Me |
| 7.013 | Me | F | H | H | Et |
| 7.014 | Me | Cl | H | H | H |
| 7.015 | Me | Cl | H | H | Me |
| 7.016 | Me | Cl | H | H | Et |
| 7.017 | Me | H | H | H | Me |
| 7.018 | Et | H | H | H | H |
| 7.019 | Et | H | H | H | Me |
| 7.020 | Et | H | H | H | Et |
| 7.021 | Et | F | H | H | H |
| 7.022 | Et | F | H | H | Me |
| 7.023 | Et | F | H | H | Et |
| 7.024 | Et | Cl | H | H | H |
| 7.025 | Et | Cl | H | H | Me |
| 7.026 | Et | Cl | H | H | Et |
| 7.027 | H | F | F | H | H |
| 7.028 | H | F | H | F | H |

**Tabelle 8: Erfindungsgemäße Verbindungen der Formel (I), worin X für CH, A für A7, R³ und R⁴ jeweils für Wasserstoff sowie R² für Chlor steht:**

| | | | | | |
|---|---|---|---|---|---|
| 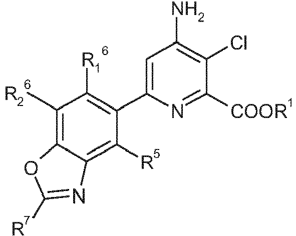 | | | | | |

| Bsp. Nr. | R¹ | R⁵ | R₁⁶ | R₂⁶ | R⁷ |
|---|---|---|---|---|---|
| 8.001 | H | H | H | H | H |
| 8.002 | H | H | H | H | Me |
| 8.003 | H | H | H | H | Ph |
| 8.004 | H | H | H | H | SMe |
| 8.005 | H | F | H | H | H |
| 8.006 | H | F | H | H | Me |
| 8.007 | H | F | H | H | Ph |
| 8.008 | H | F | H | H | SMe |
| 8.009 | H | Cl | H | H | H |
| 8.010 | H | Cl | H | H | Me |
| 8.011 | H | Cl | H | H | Ph |
| 8.012 | H | Cl | H | H | SMe |
| 8.013 | Me | H | H | H | H |
| 8.014 | Me | H | H | H | Me |
| 8.015 | Me | H | H | H | Ph |
| 8.016 | Me | H | H | H | SMe |
| 8.017 | Me | F | H | H | H |
| 8.018 | Me | F | H | H | Me |
| 8.019 | Me | F | H | H | Ph |
| 8.020 | Me | F | H | H | SMe |
| 8.021 | Me | Cl | H | H | H |
| 8.022 | Me | Cl | H | H | Me |
| 8.023 | Me | Cl | H | H | Ph |
| 8.024 | Me | Cl | H | H | SMe |
| 8.025 | Et | H | H | H | H |
| 8.026 | Et | H | H | H | Me |
| 8.027 | Et | H | H | H | Ph |
| 8.028 | Et | H | H | H | SMe |
| 8.029 | Et | F | H | H | H |
| 8.030 | Et | F | H | H | Me |
| 8.031 | Et | F | H | H | Ph |
| 8.032 | Et | F | H | H | SMe |
| 8.033 | Et | Cl | H | H | H |
| 8.034 | Et | Cl | H | H | Me |
| 8.035 | Et | Cl | H | H | Ph |
| 8.036 | Et | Cl | H | H | SMe |
| 8.037 | H | F | F | H | H |
| 8.038 | H | F | H | F | H |

**Tabelle 9: Erfindungsgemäße Verbindungen der Formel (I), worin X für CH, A für A8, R³ und R⁴ jeweils für Wasserstoff sowie R² für Chlor steht:**

| | | | | | |
|---|---|---|---|---|---|
| 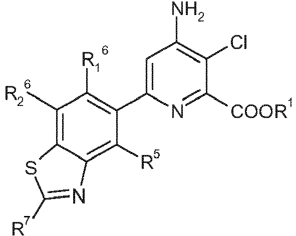 | | | | | |

| Bsp. Nr. | R¹ | R⁵ | R₁⁶ | R₂⁶ | R⁷ |
|---|---|---|---|---|---|
| 9.001 | H | H | H | H | H |
| 9.002 | H | H | H | H | Me |
| 9.003 | H | H | H | H | Ph |
| 9.004 | H | H | H | H | SMe |
| 9.005 | H | F | H | H | H |
| 9.006 | H | F | H | H | Me |
| 9.007 | H | F | H | H | Ph |
| 9.008 | H | F | H | H | SMe |
| 9.009 | H | Cl | H | H | H |
| 9.010 | H | Cl | H | H | Me |
| 9.011 | H | Cl | H | H | Ph |
| 9.012 | H | Cl | H | H | SMe |
| 9.013 | Me | H | H | H | H |
| 9.014 | Me | H | H | H | Me |
| 9.015 | Me | H | H | H | Ph |
| 9.016 | Me | H | H | H | SMe |
| 9.017 | Me | F | H | H | H |
| 9.018 | Me | F | H | H | Me |
| 9.019 | Me | F | H | H | Ph |
| 9.020 | Me | F | H | H | SMe |
| 9.021 | Me | Cl | H | H | H |
| 9.022 | Et | H | H | H | H |
| 9.023 | Et | H | H | H | Me |
| 9.024 | Et | H | H | H | Ph |
| 9.025 | Et | H | H | H | SMe |
| 9.026 | Et | F | H | H | H |
| 9.027 | Et | F | H | H | Me |
| 9.028 | Et | F | H | H | Ph |
| 9.029 | Et | F | H | H | SMe |
| 9.030 | Et | Cl | H | H | H |
| 9.031 | Et | Cl | H | H | Me |
| 9.032 | Et | Cl | H | H | Ph |
| 9.033 | Et | Cl | H | H | SMe |
| 9.034 | H | F | F | H | H |
| 9.035 | H | F | H | F | H |

**Tabelle 10: Erfindungsgemäße Verbindungen der Formel (I), worin X für CH, A für A9, R³ und R⁴ jeweils für Wasserstoff sowie R² für Chlor steht:**

| | | | | | |
|---|---|---|---|---|---|
| 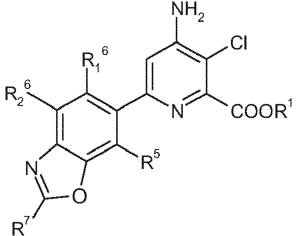 | | | | | |

| Bsp. Nr. | R¹ | R⁵ | R₁⁶ | R₂⁶ | R⁷ |
|---|---|---|---|---|---|
| 10.001 | H | H | H | H | H |
| 10.002 | H | H | H | H | Me |
| 10.003 | H | H | H | H | Ph |
| 10.004 | H | H | H | H | SMe |
| 10.005 | H | F | H | H | H |
| 10.006 | H | F | H | H | Me |
| 10.007 | H | F | H | H | Ph |
| 10.008 | H | F | H | H | SMe |
| 10.009 | H | Cl | H | H | H |
| 10.010 | H | Cl | H | H | Me |
| 10.011 | H | Cl | H | H | Ph |
| 10.012 | H | Cl | H | H | SMe |
| 10.013 | Me | H | H | H | H |
| 10.014 | Me | H | H | H | Me |
| 10.015 | Me | H | H | H | Ph |
| 10.016 | Me | H | H | H | SMe |
| 10.017 | Me | F | H | H | H |
| 10.018 | Me | F | H | H | Me |
| 10.019 | Me | F | H | H | Ph |
| 10.020 | Me | F | H | H | SMe |
| 10.021 | Me | Cl | H | H | H |
| 10.022 | Me | Cl | H | H | Me |
| 10.023 | Me | Cl | H | H | Ph |
| 10.024 | Me | Cl | H | H | SMe |
| 10.025 | Et | H | H | H | H |
| 10.026 | Et | H | H | H | Me |
| 10.027 | Et | H | H | H | Ph |
| 10.028 | Et | H | H | H | SMe |
| 10.029 | Et | F | H | H | H |
| 10.030 | Et | F | H | H | Me |
| 10.031 | Et | F | H | H | Ph |
| 10.032 | Et | F | H | H | SMe |
| 10.033 | Et | Cl | H | H | H |
| 10.034 | Et | Cl | H | H | Me |
| 10.035 | Et | Cl | H | H | Ph |
| 10.036 | Et | Cl | H | H | SMe |
| 10.037 | H | F | F | H | H |
| 10.038 | H | F | H | F | H |

**Tabelle 11: Erfindungsgemäße Verbindungen der Formel (I), worin X für CH, A für A10, R³ und R⁴ jeweils für Wasserstoff sowie R² für Chlor steht:**

| | | | | | |
|---|---|---|---|---|---|
| 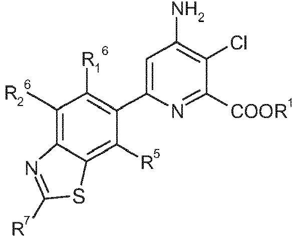 | | | | | |

| Bsp. Nr. | R¹ | R⁵ | R₁⁶ | R₂⁶ | R⁷ |
|---|---|---|---|---|---|
| 11.001 | H | H | H | H | H |
| 11.002 | H | H | H | H | Me |
| 11.003 | H | H | H | H | Ph |
| 11.004 | H | H | H | H | SMe |
| 11.005 | H | F | H | H | H |
| 11.006 | H | F | H | H | Me |
| 11.007 | H | F | H | H | Ph |
| 11.008 | H | F | H | H | SMe |
| 11.009 | H | Cl | H | H | H |
| 11.010 | H | Cl | H | H | Me |
| 11.011 | H | Cl | H | H | Ph |
| 11.012 | H | Cl | H | H | SMe |
| 11.013 | Me | H | H | H | H |
| 11.014 | Me | H | H | H | Me |
| 11.015 | Me | H | H | H | Ph |
| 11.016 | Me | H | H | H | SMe |
| 11.017 | Me | F | H | H | H |
| 11.018 | Me | F | H | H | Me |
| 11.019 | Me | F | H | H | Ph |
| 11.020 | Me | F | H | H | SMe |
| 11.021 | Me | Cl | H | H | H |
| 11.022 | Et | H | H | H | H |
| 11.023 | Et | H | H | H | Me |
| 11.024 | Et | H | H | H | Ph |
| 11.025 | Et | H | H | H | SMe |
| 11.026 | Et | F | H | H | H |
| 11.027 | Et | F | H | H | Me |
| 11.028 | Et | F | H | H | Ph |
| 11.029 | Et | F | H | H | SMe |
| 11.030 | Et | Cl | H | H | H |
| 11.031 | Et | Cl | H | H | Me |
| 11.032 | Et | Cl | H | H | Ph |
| 11.033 | Et | Cl | H | H | SMe |
| 11.034 | H | F | F | H | H |
| 11.035 | H | F | H | F | H |

**Tabelle 12: Erfindungsgemäße Verbindungen der Formel (I), worin X für CH, A für A11, R³ und R⁴ jeweils für Wasserstoff sowie R² für Chlor steht:**

| | | | | | | |
|---|---|---|---|---|---|---|
| 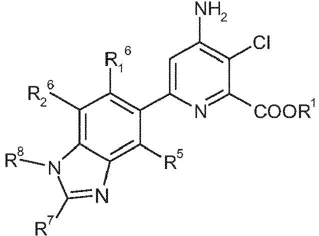 | | | | | | |

| Bsp. Nr. | R¹ | R⁵ | R₁⁶ | R₂⁶ | R⁷ | R⁸ |
|---|---|---|---|---|---|---|
| 12.001 | H | H | H | H | H | H |
| 12.002 | H | F | H | H | H | H |
| 12.003 | H | F | H | H | H | Me |
| 12.004 | H | F | H | H | Me | H |
| 12.005 | H | F | H | H | Me | Me |
| 12.006 | H | Cl | H | H | H | H |
| 12.007 | H | Cl | H | H | H | Me |
| 12.008 | H | Cl | H | H | Me | H |
| 12.009 | H | Cl | H | H | Me | Me |
| 12.010 | H | H | H | H | Ph | H |
| 12.011 | H | H | H | H | Ph | Me |
| 12.012 | H | F | H | H | Ph | H |
| 12.013 | H | F | H | H | Ph | Me |
| 12.014 | H | Cl | H | H | Ph | H |
| 12.015 | H | Cl | H | H | Ph | Me |
| 12.016 | H | H | H | H | Me | H |
| 12.017 | H | H | H | H | H | Me |
| 12.018 | H | H | H | H | Me | Me |
| 12.019 | Me | H | H | H | H | H |
| 12.020 | Me | F | H | H | H | H |
| 12.021 | Me | F | H | H | H | Me |
| 12.022 | Me | F | H | H | Me | H |
| 12.023 | Me | F | H | H | Me | Me |
| 12.024 | Me | Cl | H | H | H | H |
| 12.025 | Me | Cl | H | H | H | Me |
| 12.026 | Me | Cl | H | H | Me | H |
| 12.027 | Me | Cl | H | H | Me | Me |
| 12.028 | Me | H | H | H | Ph | H |
| 12.029 | Me | H | H | H | Ph | Me |
| 12.030 | Me | F | H | H | Ph | H |
| 12.031 | Me | F | H | H | Ph | Me |
| 12.032 | Me | Cl | H | H | Ph | H |
| 12.033 | Me | Cl | H | H | Ph | Me |
| 12.034 | Me | H | H | H | Me | H |
| 12.035 | Me | H | H | H | H | Me |
| 12.036 | Me | H | H | H | Me | Me |
| 12.037 | Et | H | H | H | H | H |
| 12.038 | Et | F | H | H | H | H |
| 12.039 | Et | F | H | H | H | Me |
| 12.040 | Et | F | H | H | Me | H |
| 12.041 | Et | F | H | H | Me | Me |
| 12.042 | Et | Cl | H | H | H | H |
| 12.043 | Et | Cl | H | H | H | Me |
| 12.044 | Et | Cl | H | H | Me | H |
| 12.045 | Et | H | H | H | Ph | H |
| 12.046 | Et | H | H | H | Ph | Me |
| 12.047 | Et | F | H | H | Ph | H |
| 12.048 | Et | F | H | H | Ph | Me |
| 12.049 | Et | Cl | H | H | Ph | H |
| 12.050 | Et | Cl | H | H | Ph | Me |
| 12.051 | Et | H | H | H | Me | H |
| 12.052 | Et | H | H | H | H | Me |
| 12.053 | Me | H | H | H | Me | Me |
| 12.054 | H | F | F | H | H | H |
| 12.055 | H | F | H | F | H | H |

**Tabelle 13: Erfindungsgemäße Verbindungen der Formel (I), worin X für CH, A für A12, R³ und R⁴ jeweils für Wasserstoff sowie R² für Chlor steht:**

| | | | | | | |
|---|---|---|---|---|---|---|
| 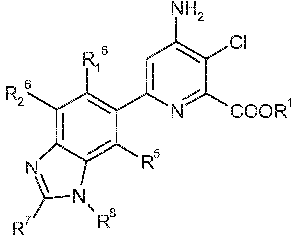 | | | | | | |

| Bsp. Nr. | R¹ | R⁵ | R₁⁶ | R₂⁶ | R⁷ | R⁸ |
|---|---|---|---|---|---|---|
| 13.001 | H | H | H | H | H | Me |
| 13.002 | H | F | H | H | H | Me |
| 13.003 | H | F | H | H | Me | Me |
| 13.004 | H | Cl | H | H | H | Me |
| 13.005 | H | Cl | H | H | Me | Me |
| 13.006 | H | H | H | H | Ph | Me |
| 13.007 | H | F | H | H | Ph | Me |
| 13.008 | H | Cl | H | H | Ph | Me |
| 13.009 | H | H | H | H | H | Me |
| 13.010 | Me | H | H | H | H | Me |
| 13.011 | Me | F | H | H | H | Me |
| 13.012 | Me | H | H | H | Me | Me |
| 13.013 | Me | F | H | H | Me | Me |
| 13.014 | Me | Cl | H | H | H | Me |
| 13.015 | Me | Cl | H | H | Me | Me |
| 13.016 | Me | H | H | H | Ph | Me |
| 13.017 | Me | F | H | H | Ph | Me |
| 13.018 | Me | Cl | H | H | Ph | Me |
| 13.019 | Et | F | H | H | H | Me |
| 13.020 | Et | F | H | H | Me | Me |
| 13.021 | Et | Cl | H | H | H | Me |
| 13.022 | Et | H | H | H | Ph | Me |
| 13.023 | Et | F | H | H | Ph | Me |
| 13.024 | Et | Cl | H | H | Ph | Me |
| 13.025 | Et | H | H | H | H | Me |
| 13.026 | Me | H | H | H | Me | Me |
| 13.027 | Et | Cl | H | H | Me | Me |
| 13.028 | H | F | F | H | H | H |
| 13.029 | H | F | H | F | H | H |

**Tabelle 14: Erfindungsgemäße Verbindungen der Formel (I), worin X für CH, A für A13, R³ und R⁴ jeweils für Wasserstoff sowie R² für Chlor steht:**

| | | | | | | |
|---|---|---|---|---|---|---|
| 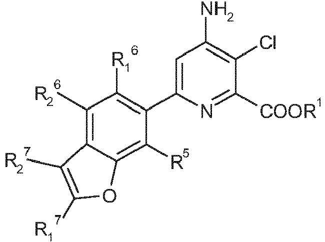 | | | | | | |

| Bsp. Nr. | R¹ | R⁵ | R₁⁶ | R₂⁶ | R₁⁷ | R₂⁷ |
|---|---|---|---|---|---|---|
| 14.001 | H | H | H | H | H | H |
| 14.002 | H | H | H | H | H | Cl |
| 14.003 | H | H | H | H | H | F |
| 14.004 | H | H | H | H | H | Me |
| 14.005 | H | H | H | H | F | H |
| 14.006 | H | H | H | H | F | Cl |
| 14.007 | H | H | H | H | F | F |
| 14.008 | H | H | H | H | F | Me |
| 14.009 | H | H | H | H | Cl | H |
| 14.010 | H | H | H | H | Cl | F |
| 14.011 | H | H | H | H | Cl | Cl |
| 14.012 | H | H | H | H | Cl | Me |
| 14.013 | H | H | H | H | Me | H |
| 14.014 | H | H | H | H | Me | Cl |
| 14.015 | H | H | H | H | Me | F |
| 14.016 | H | H | H | H | Me | Me |
| 14.017 | H | H | H | H | Ph | H |
| 14.018 | H | H | H | H | Ph | Cl |
| 14.019 | H | H | H | H | Ph | F |
| 14.020 | H | H | H | H | Ph | Me |
| 14.021 | H | F | H | H | H | H |
| 14.022 | H | F | H | H | H | Cl |
| 14.023 | H | F | H | H | H | F |
| 14.024 | H | F | H | H | H | Me |
| 14.025 | H | F | H | H | F | H |
| 14.026 | H | F | H | H | F | F |
| 14.027 | H | F | H | H | F | Cl |
| 14.028 | H | F | H | H | F | Me |
| 14.029 | H | F | H | H | Cl | H |
| 14.030 | H | F | H | H | Cl | Cl |
| 14.031 | H | F | H | H | Cl | F |
| 14.032 | H | F | H | H | Cl | Me |
| 14.033 | H | F | H | H | Me | H |
| 14.034 | H | F | H | H | Me | Cl |
| 14.035 | H | F | H | H | Me | F |
| 14.036 | H | F | H | H | Me | Me |
| 14.037 | H | F | H | H | Ph | H |
| 14.038 | H | F | H | H | Ph | Cl |
| 14.039 | H | F | H | H | Ph | F |
| 14.040 | H | F | H | H | Ph | Me |
| 14.041 | H | Cl | H | H | H | H |
| 14.042 | H | Cl | H | H | H | Cl |
| 14.043 | H | Cl | H | H | H | F |
| 14.044 | H | Cl | H | H | H | Me |
| 14.045 | H | Cl | H | H | F | H |
| 14.046 | H | Cl | H | H | F | Cl |
| 14.047 | H | Cl | H | H | F | F |
| 14.048 | H | Cl | H | H | F | Me |
| 14.049 | H | Cl | H | H | Cl | H |
| 14.050 | H | Cl | H | H | Cl | F |
| 14.051 | H | Cl | H | H | Cl | Cl |
| 14.052 | H | Cl | H | H | Cl | Me |
| 14.053 | H | Cl | H | H | Me | H |
| 14.054 | H | Cl | H | H | Me | Cl |
| 14.055 | H | Cl | H | H | Me | F |
| 14.056 | H | Cl | H | H | Me | Me |
| 14.057 | H | Cl | H | H | Ph | H |
| 14.058 | H | Cl | H | H | Ph | Cl |
| 14.059 | H | Cl | H | H | Ph | F |
| 14.060 | H | Cl | H | H | Ph | Me |
| 14.061 | Me | H | H | H | H | H |
| 14.062 | Me | H | H | H | H | Cl |
| 14.063 | Me | H | H | H | H | F |
| 14.064 | Me | H | H | H | H | Me |
| 14.065 | Me | H | H | H | F | H |
| 14.066 | Me | H | H | H | F | Cl |
| 14.067 | Me | H | H | H | F | F |
| 14.068 | Me | H | H | H | F | Me |
| 14.069 | Me | H | H | H | Cl | H |
| 14.070 | Me | H | H | H | Cl | F |
| 14.071 | Me | H | H | H | Cl | Cl |
| 14.072 | Me | H | H | H | Cl | Me |
| 14.073 | Me | H | H | H | Me | H |
| 14.074 | Me | H | H | H | Me | Cl |
| 14.075 | Me | H | H | H | Me | F |
| 14.076 | Me | H | H | H | Me | Me |
| 14.077 | Me | H | H | H | Ph | H |
| 14.078 | Me | H | H | H | Ph | Cl |
| 14.079 | Me | H | H | H | Ph | F |
| 14.080 | Me | H | H | H | Ph | Me |
| 14.081 | Me | F | H | H | H | H |
| 14.082 | Me | F | H | H | H | Cl |
| 14.083 | Me | F | H | H | H | F |
| 14.084 | Me | F | H | H | H | Me |
| 14.085 | Me | F | H | H | F | H |
| 14.086 | Me | F | H | H | F | Cl |
| 14.087 | Me | F | H | H | F | F |
| 14.088 | Me | F | H | H | F | Me |
| 14.089 | Me | F | H | H | Cl | H |
| 14.090 | Me | F | H | H | Cl | F |
| 14.091 | Me | F | H | H | Cl | Cl |
| 14.092 | Me | F | H | H | Cl | Me |
| 14.093 | Me | F | H | H | Me | H |
| 14.094 | Me | F | H | H | Me | Cl |
| 14.095 | Me | F | H | H | Me | F |
| 14.096 | Me | F | H | H | Me | Me |
| 14.097 | Me | F | H | H | Ph | H |
| 14.098 | Me | F | H | H | Ph | Cl |
| 14.099 | Me | F | H | H | Ph | F |
| 14.100 | Me | F | H | H | Ph | Me |
| 14.101 | Me | Cl | H | H | H | H |
| 14.102 | Me | Cl | H | H | H | Cl |
| 14.103 | Me | Cl | H | H | H | F |
| 14.104 | Me | Cl | H | H | H | Me |
| 14.105 | Me | Cl | H | H | F | H |
| 14.106 | Me | Cl | H | H | F | Cl |
| 14.107 | Me | Cl | H | H | F | F |
| 14.108 | Me | Cl | H | H | F | Me |
| 14.109 | Me | Cl | H | H | Cl | H |
| 14.110 | Me | Cl | H | H | Cl | F |
| 14.111 | Me | Cl | H | H | Cl | Cl |
| 14.112 | Me | Cl | H | H | Cl | Me |
| 14.113 | Me | Cl | H | H | Me | H |
| 14.114 | Me | Cl | H | H | Me | Cl |
| 14.115 | Me | Cl | H | H | Me | F |
| 14.116 | Me | Cl | H | H | Me | Me |
| 14.117 | Me | Cl | H | H | Ph | H |
| 14.118 | Me | Cl | H | H | Ph | Cl |
| 14.119 | Me | Cl | H | H | Ph | F |
| 14.120 | Me | Cl | H | H | Ph | Me |
| 14.121 | Et | H | H | H | H | H |
| 14.122 | Et | H | H | H | H | Cl |
| 14.123 | Et | H | H | H | H | F |
| 14.124 | Et | H | H | H | H | Me |
| 14.125 | Et | H | H | H | F | H |
| 14.126 | Et | H | H | H | F | Cl |
| 14.127 | Et | H | H | H | F | F |
| 14.128 | Et | H | H | H | F | Me |
| 14.129 | Et | H | H | H | Cl | H |
| 14.130 | Et | H | H | H | Cl | F |
| 14.131 | Et | H | H | H | Cl | Cl |
| 14.132 | Et | H | H | H | Cl | Me |
| 14.133 | Et | H | H | H | Me | H |
| 14.134 | Et | H | H | H | Me | Cl |
| 14.135 | Et | H | H | H | Me | F |
| 14.136 | Et | H | H | H | Me | Me |
| 14.137 | Et | H | H | H | Ph | H |
| 14.138 | Et | H | H | H | Ph | Cl |
| 14.139 | Et | H | H | H | Ph | F |
| 14.140 | Et | H | H | H | Ph | Me |
| 14.141 | Et | F | H | H | H | H |
| 14.142 | Et | F | H | H | H | Cl |
| 14.143 | Et | F | H | H | H | F |
| 14.144 | Et | F | H | H | H | Me |
| 14.145 | Et | F | H | H | F | H |
| 14.146 | Et | F | H | H | F | Cl |
| 14.147 | Et | F | H | H | F | F |
| 14.148 | Et | F | H | H | F | Me |
| 14.149 | Et | F | H | H | Cl | H |
| 14.150 | Et | F | H | H | Cl | F |
| 14.151 | Et | F | H | H | Cl | Cl |
| 14.152 | Et | F | H | H | Cl | Me |
| 14.153 | Et | F | H | H | Me | H |
| 14.154 | Et | F | H | H | Me | Cl |
| 14.155 | Et | F | H | H | Me | F |
| 14.156 | Et | F | H | H | Me | Me |
| 14.157 | Et | F | H | H | Ph | H |
| 14.158 | Et | F | H | H | Ph | Cl |
| 14.159 | Et | F | H | H | Ph | F |
| 14.160 | Et | F | H | H | Ph | Me |
| 14.161 | Et | Cl | H | H | H | H |
| 14.162 | Et | Cl | H | H | H | Cl |
| 14.163 | Et | Cl | H | H | H | F |
| 14.164 | Et | Cl | H | H | H | Me |
| 14.165 | Et | Cl | H | H | F | H |
| 14.166 | Et | Cl | H | H | F | Cl |
| 14.167 | Et | Cl | H | H | F | F |
| 14.168 | Et | Cl | H | H | F | Me |
| 14.169 | Et | Cl | H | H | Cl | H |
| 14.170 | Et | Cl | H | H | Cl | F |
| 14.171 | Et | Cl | H | H | Cl | Cl |
| 14.172 | Et | Cl | H | H | Cl | Me |
| 14.173 | Et | Cl | H | H | Me | H |
| 14.174 | Et | Cl | H | H | Me | Cl |
| 14.175 | Et | Cl | H | H | Me | F |
| 14.176 | Et | Cl | H | H | Me | Me |
| 14.177 | Et | Cl | H | H | Ph | H |
| 14.178 | Et | Cl | H | H | Ph | Cl |
| 14.179 | Et | Cl | H | H | Ph | F |
| 14.180 | Et | Cl | H | H | Ph | Me |
| 14.181 | H | F | F | H | H | H |
| 14.182 | H | F | H | F | H | H |
| 14.183 | Me | H | F | H | H | H |
| 14.184 | H | H | F | H | H | H |
| 14.185 | K | H | F | H | H | H |
| 14.186 | Me | H | Cl | H | H | H |
| 14.187 | H | H | Cl | H | H | H |
| 14.188 | K | H | Cl | H | H | H |
| 14.189 | Me | H | H | F | H | H |
| 14.190 | H | H | H | F | H | H |
| 14.191 | K | H | H | F | H | H |
| 14.192 | Me | H | H | Cl | H | H |
| 14.193 | H | H | H | Cl | H | H |
| 14.194 | K | H | H | Cl | H | H |
| 14.195 | K | F | H | H | H | H |
| 14.196 | K | Cl | H | H | H | H |

**Tabelle 15: Erfindungsgemäße Verbindungen der Formel (I), worin X für CH, A für A14, R³ und R⁴ jeweils für Wasserstoff sowie R² für Chlor steht:**

| | | | | | | |
|---|---|---|---|---|---|---|
| 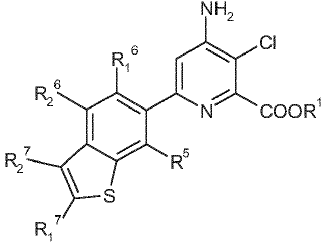 | | | | | | |

| Bsp. Nr. | R¹ | R⁵ | R₁⁶ | R₂⁶ | R₁⁷ | R₂⁷ |
|---|---|---|---|---|---|---|
| 15.001 | H | H | H | H | H | H |
| 15.002 | H | H | H | H | H | Cl |
| 15.003 | H | H | H | H | H | F |
| 15.004 | H | H | H | H | H | Me |
| 15.005 | H | H | H | H | F | H |
| 15.006 | H | H | H | H | F | Cl |
| 15.007 | H | H | H | H | F | F |
| 15.008 | H | H | H | H | F | Me |
| 15.009 | H | H | H | H | Cl | H |
| 15.010 | H | H | H | H | Cl | F |
| 15.011 | H | H | H | H | Cl | Cl |
| 15.012 | H | H | H | H | Cl | Me |
| 15.013 | H | H | H | H | Me | H |
| 15.014 | H | H | H | H | Me | Cl |
| 15.015 | H | H | H | H | Me | F |
| 15.016 | H | H | H | H | Me | Me |
| 15.017 | H | H | H | H | Ph | H |
| 15.018 | H | H | H | H | Ph | Cl |
| 15.019 | H | H | H | H | Ph | F |
| 15.020 | H | H | H | H | Ph | Me |
| 15.021 | H | F | H | H | H | H |
| 15.022 | H | F | H | H | H | Cl |
| 15.023 | H | F | H | H | H | F |
| 15.024 | H | F | H | H | H | Me |
| 15.025 | H | F | H | H | F | H |
| 15.026 | H | F | H | H | F | Cl |
| 15.027 | H | F | H | H | F | F |
| 15.028 | H | F | H | H | F | Me |
| 15.029 | H | F | H | H | Cl | H |
| 15.030 | H | F | H | H | Cl | F |
| 15.031 | H | F | H | H | Cl | Cl |
| 15.032 | H | F | H | H | Cl | Me |
| 15.033 | H | F | H | H | Me | H |
| 15.034 | H | F | H | H | Me | Cl |
| 15.035 | H | F | H | H | Me | F |
| 15.036 | H | F | H | H | Me | Me |
| 15.037 | H | F | H | H | Ph | H |
| 15.038 | H | F | H | H | Ph | Cl |
| 15.039 | H | F | H | H | Ph | F |
| 15.040 | H | F | H | H | Ph | Me |
| 15.041 | H | Cl | H | H | H | H |
| 15.042 | H | Cl | H | H | H | Cl |
| 15.043 | H | Cl | H | H | H | F |
| 15.044 | H | Cl | H | H | H | Me |
| 15.045 | H | Cl | H | H | F | H |
| 15.046 | H | Cl | H | H | F | Cl |
| 15.047 | H | Cl | H | H | F | F |
| 15.048 | H | Cl | H | H | F | Me |
| 15.049 | H | Cl | H | H | Cl | H |
| 15.050 | H | Cl | H | H | Cl | F |
| 15.051 | H | Cl | H | H | Cl | Cl |
| 15.052 | H | Cl | H | H | Cl | Me |
| 15.053 | H | Cl | H | H | Me | H |
| 15.054 | H | Cl | H | H | Me | Cl |
| 15.055 | H | Cl | H | H | Me | F |
| 15.056 | H | Cl | H | H | Me | Me |
| 15.057 | H | Cl | H | H | Ph | H |
| 15.058 | H | Cl | H | H | Ph | Cl |
| 15.059 | H | Cl | H | H | Ph | F |
| 15.060 | H | Cl | H | H | Ph | Me |
| 15.061 | Me | H | H | H | H | H |
| 15.062 | Me | H | H | H | H | Cl |
| 15.063 | Me | H | H | H | H | F |
| 15.064 | Me | H | H | H | H | Me |
| 15.065 | Me | H | H | H | F | H |
| 15.066 | Me | H | H | H | F | Cl |
| 15.067 | Me | H | H | H | F | F |
| 15.068 | Me | H | H | H | F | Me |
| 15.069 | Me | H | H | H | Cl | H |
| 15.070 | Me | H | H | H | Cl | F |
| 15.071 | Me | H | H | H | Cl | Cl |
| 15.072 | Me | H | H | H | Cl | Me |
| 15.073 | Me | H | H | H | Me | H |
| 15.074 | Me | H | H | H | Me | Cl |
| 15.075 | Me | H | H | H | Me | F |
| 15.076 | Me | H | H | H | Me | Me |
| 15.077 | Me | H | H | H | Ph | H |
| 15.078 | Me | H | H | H | Ph | Cl |
| 15.079 | Me | H | H | H | Ph | F |
| 15.080 | Me | H | H | H | Ph | Me |
| 15.081 | Me | F | H | H | H | H |
| 15.082 | Me | F | H | H | H | Cl |
| 15.083 | Me | F | H | H | H | F |
| 15.084 | Me | F | H | H | H | Me |
| 15.085 | Me | F | H | H | F | H |
| 15.086 | Me | F | H | H | F | Cl |
| 15.087 | Me | F | H | H | F | F |
| 15.088 | Me | F | H | H | F | Me |
| 15.089 | Me | F | H | H | Cl | H |
| 15.090 | Me | F | H | H | Cl | F |
| 15.091 | Me | F | H | H | Cl | Cl |
| 15.092 | Me | F | H | H | Cl | Me |
| 15.093 | Me | F | H | H | Me | H |
| 15.094 | Me | F | H | H | Me | Cl |
| 15.095 | Me | F | H | H | Me | F |
| 15.096 | Me | F | H | H | Me | Me |
| 15.097 | Me | F | H | H | Ph | H |
| 15.098 | Me | F | H | H | Ph | Cl |
| 15.099 | Me | F | H | H | Ph | F |
| 15.100 | Me | F | H | H | Ph | Me |
| 15.101 | Me | Cl | H | H | H | H |
| 15.102 | Me | Cl | H | H | H | Cl |
| 15.103 | Me | Cl | H | H | H | F |
| 15.104 | Me | Cl | H | H | H | Me |
| 15.105 | Me | Cl | H | H | F | H |
| 15.106 | Me | Cl | H | H | F | Cl |
| 15.107 | Me | Cl | H | H | F | F |
| 15.108 | Me | Cl | H | H | F | Me |
| 15.109 | Me | Cl | H | H | Cl | H |
| 15.110 | Me | Cl | H | H | Cl | F |
| 15.111 | Me | Cl | H | H | Cl | Cl |
| 15.112 | Me | Cl | H | H | Cl | Me |
| 15.113 | Me | Cl | H | H | Me | H |
| 15.114 | Me | Cl | H | H | Me | Cl |
| 15.115 | Me | Cl | H | H | Me | F |
| 15.116 | Me | Cl | H | H | Me | Me |
| 15.117 | Me | Cl | H | H | Ph | H |
| 15.118 | Me | Cl | H | H | Ph | Cl |
| 15.119 | Me | Cl | H | H | Ph | F |
| 15.120 | Me | Cl | H | H | Ph | Me |
| 15.121 | Et | H | H | H | H | H |
| 15.122 | Et | H | H | H | H | Cl |
| 15.123 | Et | H | H | H | H | F |
| 15.124 | Et | H | H | H | H | Me |
| 15.125 | Et | H | H | H | F | H |
| 15.126 | Et | H | H | H | F | Cl |
| 15.127 | Et | H | H | H | F | F |
| 15.128 | Et | H | H | H | F | Me |
| 15.129 | Et | H | H | H | Cl | H |
| 15.130 | Et | H | H | H | Cl | F |
| 15.131 | Et | H | H | H | Cl | Cl |
| 15.132 | Et | H | H | H | Cl | Me |
| 15.133 | Et | H | H | H | Me | H |
| 15.134 | Et | H | H | H | Me | Cl |
| 15.135 | Et | H | H | H | Me | F |
| 15.136 | Et | H | H | H | Me | Me |
| 15.137 | Et | H | H | H | Ph | H |
| 15.138 | Et | H | H | H | Ph | Cl |
| 15.139 | Et | H | H | H | Ph | F |
| 15.140 | Et | H | H | H | Ph | Me |
| 15.141 | Et | F | H | H | H | H |
| 15.142 | Et | F | H | H | H | Cl |
| 15.143 | Et | F | H | H | H | F |
| 15.144 | Et | F | H | H | H | Me |
| 15.145 | Et | F | H | H | F | H |
| 15.146 | Et | F | H | H | F | Cl |
| 15.147 | Et | F | H | H | F | F |
| 15.148 | Et | F | H | H | F | Me |
| 15.149 | Et | F | H | H | Cl | H |
| 15.150 | Et | F | H | H | Cl | F |
| 15.151 | Et | F | H | H | Cl | Cl |
| 15.152 | Et | F | H | H | Cl | Me |
| 15.153 | Et | F | H | H | Me | H |
| 15.154 | Et | F | H | H | Me | Cl |
| 15.155 | Et | F | H | H | Me | F |
| 15.156 | Et | F | H | H | Me | Me |
| 15.157 | Et | F | H | H | Ph | H |
| 15.158 | Et | F | H | H | Ph | Cl |
| 15.159 | Et | F | H | H | Ph | F |
| 15.160 | Et | F | H | H | Ph | Me |
| 15.161 | Et | Cl | H | H | H | H |
| 15.162 | Et | Cl | H | H | H | Cl |
| 15.163 | Et | Cl | H | H | H | F |
| 15.164 | Et | Cl | H | H | H | Me |
| 15.165 | Et | Cl | H | H | F | H |
| 15.166 | Et | Cl | H | H | F | Cl |
| 15.167 | Et | Cl | H | H | F | F |
| 15.168 | Et | Cl | H | H | F | Me |
| 15.169 | Et | Cl | H | H | Cl | H |
| 15.170 | Et | Cl | H | H | Cl | F |
| 15.171 | Et | Cl | H | H | Cl | Cl |
| 15.172 | Et | Cl | H | H | Cl | Me |
| 15.173 | Et | Cl | H | H | Me | H |
| 15.174 | Et | Cl | H | H | Me | Cl |
| 15.175 | Et | Cl | H | H | Me | F |
| 15.176 | Et | Cl | H | H | Me | Me |
| 15.177 | Et | Cl | H | H | Ph | H |
| 15.178 | Et | Cl | H | H | Ph | Cl |
| 15.179 | Et | Cl | H | H | Ph | F |
| 15.180 | Et | Cl | H | H | Ph | Me |
| 15.181 | H | F | F | H | H | H |
| 15.182 | H | F | H | F | H | H |
| 15.183 | Me | H | F | H | H | H |
| 15.184 | H | H | F | H | H | H |
| 15.185 | K | H | F | H | H | H |
| 15.186 | Me | H | Cl | H | H | H |
| 15.187 | H | H | Cl | H | H | H |
| 15.188 | K | H | Cl | H | H | H |
| 15.189 | Me | H | H | F | H | H |
| 15.190 | H | H | H | F | H | H |
| 15.191 | K | H | H | F | H | H |
| 15.192 | Me | H | H | Cl | H | H |
| 15.193 | H | H | H | Cl | H | H |
| 15.194 | K | H | H | Cl | H | H |
| 15.195 | K | F | H | H | H | H |
| 15.196 | K | Cl | H | H | H | H |

**Tabelle 16: Erfindungsgemäße Verbindungen der Formel (I), worin X für CH, A für A15, R⁸, R³ und R⁴ jeweils für Wasserstoff sowie R² für Chlor steht:**

| | | | | | | |
|---|---|---|---|---|---|---|
| 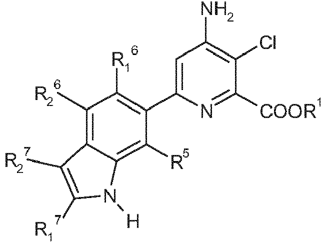 | | | | | | |

| Bsp. Nr. | R¹ | R⁵ | R₁⁶ | R₂⁶ | R₁⁷ | R₂⁷ |
|---|---|---|---|---|---|---|
| 16.001 | H | H | H | H | H | H |
| 16.002 | H | H | H | H | H | Cl |
| 16.003 | H | H | H | H | H | F |
| 16.004 | H | H | H | H | H | Me |
| 16.005 | H | H | H | H | F | H |
| 16.006 | H | H | H | H | F | Cl |
| 16.007 | H | H | H | H | F | F |
| 16.008 | H | H | H | H | F | Me |
| 16.009 | H | H | H | H | Cl | H |
| 16.010 | H | H | H | H | Cl | F |
| 16.011 | H | H | H | H | Cl | Cl |
| 16.012 | H | H | H | H | Cl | Me |
| 16.013 | H | H | H | H | Me | H |
| 16.014 | H | H | H | H | Me | Cl |
| 16.015 | H | H | H | H | Me | F |
| 16.016 | H | H | H | H | Me | Me |
| 16.017 | H | H | H | H | Ph | H |
| 16.018 | H | H | H | H | Ph | Cl |
| 16.019 | H | H | H | H | Ph | F |
| 16.020 | H | H | H | H | Ph | Me |
| 16.021 | H | F | H | H | H | H |
| 16.022 | H | F | H | H | H | Cl |
| 16.023 | H | F | H | H | H | F |
| 16.024 | H | F | H | H | H | Me |
| 16.025 | H | F | H | H | F | H |
| 16.026 | H | F | H | H | F | Cl |
| 16.027 | H | F | H | H | F | F |
| 16.028 | H | F | H | H | F | Me |
| 16.029 | H | F | H | H | Cl | H |
| 16.030 | H | F | H | H | Cl | F |
| 16.031 | H | F | H | H | Cl | Cl |
| 16.032 | H | F | H | H | Cl | Me |
| 16.033 | H | F | H | H | Me | H |
| 16.034 | H | F | H | H | Me | Cl |
| 16.035 | H | F | H | H | Me | F |
| 16.036 | H | F | H | H | Me | Me |
| 16.037 | H | F | H | H | Ph | H |
| 16.038 | H | F | H | H | Ph | Cl |
| 16.039 | H | F | H | H | Ph | F |
| 16.040 | H | F | H | H | Ph | Me |
| 16.041 | H | Cl | H | H | H | H |
| 16.042 | H | Cl | H | H | H | Cl |
| 16.043 | H | Cl | H | H | H | F |
| 16.044 | H | Cl | H | H | H | Me |
| 16.045 | H | Cl | H | H | F | H |
| 16.046 | H | Cl | H | H | F | Cl |
| 16.047 | H | Cl | H | H | F | F |
| 16.048 | H | Cl | H | H | F | Me |
| 16.049 | H | Cl | H | H | Cl | H |
| 16.050 | H | Cl | H | H | Cl | F |
| 16.051 | H | Cl | H | H | Cl | Cl |
| 16.052 | H | Cl | H | H | Cl | Me |
| 16.053 | H | Cl | H | H | Me | H |
| 16.054 | H | Cl | H | H | Me | Cl |
| 16.055 | H | Cl | H | H | Me | F |
| 16.056 | H | Cl | H | H | Me | Me |
| 16.057 | H | Cl | H | H | Ph | H |
| 16.058 | H | Cl | H | H | Ph | Cl |
| 16.059 | H | Cl | H | H | Ph | F |
| 16.060 | H | Cl | H | H | Ph | Me |
| 16.061 | Me | H | H | H | H | H |
| 16.062 | Me | H | H | H | H | Cl |
| 16.063 | Me | H | H | H | H | F |
| 16.064 | Me | H | H | H | H | Me |
| 16.065 | Me | H | H | H | F | H |
| 16.066 | Me | H | H | H | F | Cl |
| 16.067 | Me | H | H | H | F | F |
| 16.068 | Me | H | H | H | F | Me |
| 16.069 | Me | H | H | H | Cl | H |
| 16.070 | Me | H | H | H | Cl | F |
| 16.071 | Me | H | H | H | Cl | Cl |
| 16.072 | Me | H | H | H | Cl | Me |
| 16.073 | Me | H | H | H | Me | H |
| 16.074 | Me | H | H | H | Me | Cl |
| 16.075 | Me | H | H | H | Me | F |
| 16.076 | Me | H | H | H | Me | Me |
| 16.077 | Me | H | H | H | Ph | H |
| 16.078 | Me | H | H | H | Ph | Cl |
| 16.079 | Me | H | H | H | Ph | F |
| 16.080 | Me | H | H | H | Ph | Me |
| 16.081 | Me | F | H | H | H | H |
| 16.082 | Me | F | H | H | H | Cl |
| 16.083 | Me | F | H | H | H | F |
| 16.084 | Me | F | H | H | H | Me |
| 16.085 | Me | F | H | H | F | H |
| 16.086 | Me | F | H | H | F | Cl |
| 16.087 | Me | F | H | H | F | F |
| 16.088 | Me | F | H | H | F | Me |
| 16.089 | Me | F | H | H | Cl | H |
| 16.090 | Me | F | H | H | Cl | F |
| 16.091 | Me | F | H | H | Cl | Cl |
| 16.092 | Me | F | H | H | Cl | Me |
| 16.093 | Me | F | H | H | Me | H |
| 16.094 | Me | F | H | H | Me | Cl |
| 16.095 | Me | F | H | H | Me | F |
| 16.096 | Me | F | H | H | Me | Me |
| 16.097 | Me | F | H | H | Ph | H |
| 16.098 | Me | F | H | H | Ph | Cl |
| 16.099 | Me | F | H | H | Ph | F |
| 16.100 | Me | F | H | H | Ph | Me |
| 16.101 | Me | Cl | H | H | H | H |
| 16.102 | Me | Cl | H | H | H | Cl |
| 16.103 | Me | Cl | H | H | H | F |
| 16.104 | Me | Cl | H | H | H | Me |
| 16.105 | Me | Cl | H | H | F | H |
| 16.106 | Me | Cl | H | H | F | Cl |
| 16.107 | Me | Cl | H | H | F | F |
| 16.108 | Me | Cl | H | H | F | Me |
| 16.109 | Me | Cl | H | H | Cl | H |
| 16.110 | Me | Cl | H | H | Cl | F |
| 16.111 | Me | Cl | H | H | Cl | Cl |
| 16.112 | Me | Cl | H | H | Cl | Me |
| 16.113 | Me | Cl | H | H | Me | H |
| 16.114 | Me | Cl | H | H | Me | Cl |
| 16.115 | Me | Cl | H | H | Me | F |
| 16.116 | Me | Cl | H | H | Me | Me |
| 16.117 | Me | Cl | H | H | Ph | H |
| 16.118 | Me | Cl | H | H | Ph | Cl |
| 16.119 | Me | Cl | H | H | Ph | F |
| 16.120 | Me | Cl | H | H | Ph | Me |
| 16.121 | Et | H | H | H | H | H |
| 16.122 | Et | H | H | H | H | Cl |
| 16.123 | Et | H | H | H | H | F |
| 16.124 | Et | H | H | H | H | Me |
| 16.125 | Et | H | H | H | F | H |
| 16.126 | Et | H | H | H | F | Cl |
| 16.127 | Et | H | H | H | F | F |
| 16.128 | Et | H | H | H | F | Me |
| 16.129 | Et | H | H | H | Cl | H |
| 16.130 | Et | H | H | H | Cl | F |
| 16.131 | Et | H | H | H | Cl | Cl |
| 16.132 | Et | H | H | H | Cl | Me |
| 16.133 | Et | H | H | H | Me | H |
| 16.134 | Et | H | H | H | Me | Cl |
| 16.135 | Et | H | H | H | Me | F |
| 16.136 | Et | H | H | H | Me | Me |
| 16.137 | Et | H | H | H | Ph | H |
| 16.138 | Et | H | H | H | Ph | Cl |
| 16.139 | Et | H | H | H | Ph | F |
| 16.140 | Et | H | H | H | Ph | Me |
| 16.141 | Et | F | H | H | H | H |
| 16.142 | Et | F | H | H | H | Cl |
| 16.143 | Et | F | H | H | H | F |
| 16.144 | Et | F | H | H | H | Me |
| 16.145 | Et | F | H | H | F | H |
| 16.146 | Et | F | H | H | F | Cl |
| 16.147 | Et | F | H | H | F | F |
| 16.148 | Et | F | H | H | F | Me |
| 16.149 | Et | F | H | H | Cl | H |
| 16.150 | Et | F | H | H | Cl | F |
| 16.151 | Et | F | H | H | Cl | Cl |
| 16.152 | Et | F | H | H | Cl | Me |
| 16.153 | Et | F | H | H | Me | H |
| 16.154 | Et | F | H | H | Me | Cl |
| 16.155 | Et | F | H | H | Me | F |
| 16.156 | Et | F | H | H | Me | Me |
| 16.157 | Et | F | H | H | Ph | H |
| 16.158 | Et | F | H | H | Ph | Cl |
| 16.159 | Et | F | H | H | Ph | F |
| 16.160 | Et | F | H | H | Ph | Me |
| 16.161 | Et | Cl | H | H | H | H |
| 16.162 | Et | Cl | H | H | H | Cl |
| 16.163 | Et | Cl | H | H | H | F |
| 16.164 | Et | Cl | H | H | H | Me |
| 16.165 | Et | Cl | H | H | F | H |
| 16.166 | Et | Cl | H | H | F | Cl |
| 16.167 | Et | Cl | H | H | F | F |
| 16.168 | Et | Cl | H | H | F | Me |
| 16.169 | Et | Cl | H | H | Cl | H |
| 16.170 | Et | Cl | H | H | Cl | F |
| 16.171 | Et | Cl | H | H | Cl | Cl |
| 16.172 | Et | Cl | H | H | Cl | Me |
| 16.173 | Et | Cl | H | H | Me | H |
| 16.174 | Et | Cl | H | H | Me | Cl |
| 16.175 | Et | Cl | H | H | Me | F |
| 16.176 | Et | Cl | H | H | Me | Me |
| 16.177 | Et | Cl | H | H | Ph | H |
| 16.178 | Et | Cl | H | H | Ph | Cl |
| 16.179 | Et | Cl | H | H | Ph | F |
| 16.180 | Et | Cl | H | H | Ph | Me |
| 16.181 | H | F | F | H | H | H |
| 16.182 | H | F | H | F | H | H |

**Tabelle 17: Erfindungsgemäße Verbindungen der Formel (I), worin X für CH, A für A15, R⁸ für Methyl, R³ und R⁴ jeweils für Wasserstoff sowie R² für Chlor steht:**

| | | | | | | |
|---|---|---|---|---|---|---|
| 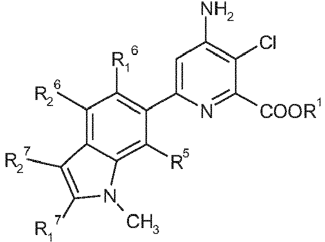 | | | | | | |

| Bsp. Nr. | R¹ | R⁵ | R₁⁶ | R₂⁶ | R₁⁷ | R₂⁷ |
|---|---|---|---|---|---|---|
| 17.001 | H | H | H | H | H | H |
| 17.002 | H | H | H | H | H | Cl |
| 17.003 | H | H | H | H | H | F |
| 17.004 | H | H | H | H | H | Me |
| 17.005 | H | H | H | H | F | H |
| 17.006 | H | H | H | H | F | Cl |
| 17.007 | H | H | H | H | F | F |
| 17.008 | H | H | H | H | F | Me |
| 17.009 | H | H | H | H | Cl | H |
| 17.010 | H | H | H | H | Cl | F |
| 17.011 | H | H | H | H | Cl | Cl |
| 17.012 | H | H | H | H | Cl | Me |
| 17.013 | H | H | H | H | Me | H |
| 17.014 | H | H | H | H | Me | Cl |
| 17.015 | H | H | H | H | Me | F |
| 17.016 | H | H | H | H | Me | Me |
| 17.017 | H | H | H | H | Ph | H |
| 17.018 | H | H | H | H | Ph | Cl |
| 17.019 | H | H | H | H | Ph | F |
| 17.020 | H | H | H | H | Ph | Me |
| 17.021 | H | F | H | H | H | H |
| 17.022 | H | F | H | H | H | Cl |
| 17.023 | H | F | H | H | H | F |
| 17.024 | H | F | H | H | H | Me |
| 17.025 | H | F | H | H | F | H |
| 17.026 | H | F | H | H | F | Cl |
| 17.027 | H | F | H | H | F | F |
| 17.028 | H | F | H | H | F | Me |
| 17.029 | H | F | H | H | Cl | H |
| 17.030 | H | F | H | H | Cl | F |
| 17.031 | H | F | H | H | Cl | Cl |
| 17.032 | H | F | H | H | Cl | Me |
| 17.033 | H | F | H | H | Me | H |
| 17.034 | H | F | H | H | Me | Cl |
| 17.035 | H | F | H | H | Me | F |
| 17.036 | H | F | H | H | Me | Me |
| 17.037 | H | F | H | H | Ph | H |
| 17.038 | H | F | H | H | Ph | Cl |
| 17.039 | H | F | H | H | Ph | F |
| 17.040 | H | F | H | H | Ph | Me |
| 17.041 | H | Cl | H | H | H | H |
| 17.042 | H | Cl | H | H | H | Cl |
| 17.043 | H | Cl | H | H | H | F |
| 17.044 | H | Cl | H | H | H | Me |
| 17.045 | H | Cl | H | H | F | H |
| 17.046 | H | Cl | H | H | F | Cl |
| 17.047 | H | Cl | H | H | F | F |
| 17.048 | H | Cl | H | H | F | Me |
| 17.049 | H | Cl | H | H | Cl | H |
| 17.050 | H | Cl | H | H | Cl | F |
| 17.051 | H | Cl | H | H | Cl | Cl |
| 17.052 | H | Cl | H | H | Cl | Me |
| 17.053 | H | Cl | H | H | Me | H |
| 17.054 | H | Cl | H | H | Me | Cl |
| 17.055 | H | Cl | H | H | Me | F |
| 17.056 | H | Cl | H | H | Me | Me |
| 17.057 | H | Cl | H | H | Ph | H |
| 17.058 | H | Cl | H | H | Ph | Cl |
| 17.059 | H | Cl | H | H | Ph | F |
| 17.060 | H | Cl | H | H | Ph | Me |
| 17.061 | Me | H | H | H | H | H |
| 17.062 | Me | H | H | H | H | Cl |
| 17.063 | Me | H | H | H | H | F |
| 17.064 | Me | H | H | H | H | Me |
| 17.065 | Me | H | H | H | F | H |
| 17.066 | Me | H | H | H | F | Cl |
| 17.067 | Me | H | H | H | F | F |
| 17.068 | Me | H | H | H | F | Me |
| 17.069 | Me | H | H | H | Cl | H |
| 17.070 | Me | H | H | H | Cl | F |
| 17.071 | Me | H | H | H | Cl | Cl |
| 17.072 | Me | H | H | H | Cl | Me |
| 17.073 | Me | H | H | H | Me | H |
| 17.074 | Me | H | H | H | Me | Cl |
| 17.075 | Me | H | H | H | Me | F |
| 17.076 | Me | H | H | H | Me | Me |
| 17.077 | Me | H | H | H | Ph | H |
| 17.078 | Me | H | H | H | Ph | Cl |
| 17.079 | Me | H | H | H | Ph | F |
| 17.080 | Me | H | H | H | Ph | Me |
| 17.081 | Me | F | H | H | H | H |
| 17.082 | Me | F | H | H | H | Cl |
| 17.083 | Me | F | H | H | H | F |
| 17.084 | Me | F | H | H | H | Me |
| 17.085 | Me | F | H | H | F | H |
| 17.086 | Me | F | H | H | F | Cl |
| 17.087 | Me | F | H | H | F | F |
| 17.088 | Me | F | H | H | F | Me |
| 17.089 | Me | F | H | H | Cl | H |
| 17.090 | Me | F | H | H | Cl | F |
| 17.091 | Me | F | H | H | Cl | Cl |
| 17.092 | Me | F | H | H | Cl | Me |
| 17.093 | Me | F | H | H | Me | H |
| 17.094 | Me | F | H | H | Me | Cl |
| 17.095 | Me | F | H | H | Me | F |
| 17.096 | Me | F | H | H | Me | Me |
| 17.097 | Me | F | H | H | Ph | H |
| 17.098 | Me | F | H | H | Ph | Cl |
| 17.099 | Me | F | H | H | Ph | F |
| 17.100 | Me | F | H | H | Ph | Me |
| 17.101 | Me | Cl | H | H | H | H |
| 17.102 | Me | Cl | H | H | H | Cl |
| 17.103 | Me | Cl | H | H | H | F |
| 17.104 | Me | Cl | H | H | H | Me |
| 17.105 | Me | Cl | H | H | F | H |
| 17.106 | Me | Cl | H | H | F | Cl |
| 17.107 | Me | Cl | H | H | F | F |
| 17.108 | Me | Cl | H | H | F | Me |
| 17.109 | Me | Cl | H | H | Cl | H |
| 17.110 | Me | Cl | H | H | Cl | F |
| 17.111 | Me | Cl | H | H | Cl | Cl |
| 17.112 | Me | Cl | H | H | Cl | Me |
| 17.113 | Me | Cl | H | H | Me | H |
| 17.114 | Me | Cl | H | H | Me | Cl |
| 17.115 | Me | Cl | H | H | Me | F |
| 17.116 | Me | Cl | H | H | Me | Me |
| 17.117 | Me | Cl | H | H | Ph | H |
| 17.118 | Me | Cl | H | H | Ph | Cl |
| 17.119 | Me | Cl | H | H | Ph | F |
| 17.120 | Me | Cl | H | H | Ph | Me |
| 17.121 | Et | H | H | H | H | H |
| 17.122 | Et | H | H | H | H | Cl |
| 17.123 | Et | H | H | H | H | F |
| 17.124 | Et | H | H | H | H | Me |
| 17.125 | Et | H | H | H | F | H |
| 17.126 | Et | H | H | H | F | Cl |
| 17.127 | Et | H | H | H | F | F |
| 17.128 | Et | H | H | H | F | Me |
| 17.129 | Et | H | H | H | Cl | H |
| 17.130 | Et | H | H | H | Cl | F |
| 17.131 | Et | H | H | H | Cl | Cl |
| 17.132 | Et | H | H | H | Cl | Me |
| 17.133 | Et | H | H | H | Me | H |
| 17.134 | Et | H | H | H | Me | Cl |
| 17.135 | Et | H | H | H | Me | F |
| 17.136 | Et | H | H | H | Me | Me |
| 17.137 | Et | H | H | H | Ph | H |
| 17.138 | Et | H | H | H | Ph | Cl |
| 17.139 | Et | H | H | H | Ph | F |
| 17.140 | Et | H | H | H | Ph | Me |
| 17.141 | Et | F | H | H | H | H |
| 17.142 | Et | F | H | H | H | Cl |
| 17.143 | Et | F | H | H | H | F |
| 17.144 | Et | F | H | H | H | Me |
| 17.145 | Et | F | H | H | F | H |
| 17.146 | Et | F | H | H | F | Cl |
| 17.147 | Et | F | H | H | F | F |
| 17.148 | Et | F | H | H | F | Me |
| 17.149 | Et | F | H | H | Cl | H |
| 17.150 | Et | F | H | H | Cl | F |
| 17.151 | Et | F | H | H | Cl | Cl |
| 17.152 | Et | F | H | H | Cl | Me |
| 17.153 | Et | F | H | H | Me | H |
| 17.154 | Et | F | H | H | Me | Cl |
| 17.155 | Et | F | H | H | Me | F |
| 17.156 | Et | F | H | H | Me | Me |
| 17.157 | Et | F | H | H | Ph | H |
| 17.158 | Et | F | H | H | Ph | Cl |
| 17.159 | Et | F | H | H | Ph | F |
| 17.160 | Et | F | H | H | Ph | Me |
| 17.161 | Et | Cl | H | H | H | H |
| 17.162 | Et | Cl | H | H | H | Cl |
| 17.163 | Et | Cl | H | H | H | F |
| 17.164 | Et | Cl | H | H | H | Me |
| 17.165 | Et | Cl | H | H | F | H |
| 17.166 | Et | Cl | H | H | F | Cl |
| 17.167 | Et | Cl | H | H | F | F |
| 17.168 | Et | Cl | H | H | F | Me |
| 17.169 | Et | Cl | H | H | Cl | H |
| 17.170 | Et | Cl | H | H | Cl | F |
| 17.171 | Et | Cl | H | H | Cl | Cl |
| 17.172 | Et | Cl | H | H | Cl | Me |
| 17.173 | Et | Cl | H | H | Me | H |
| 17.174 | Et | Cl | H | H | Me | Cl |
| 17.175 | Et | Cl | H | H | Me | F |
| 17.176 | Et | Cl | H | H | Me | Me |
| 17.177 | Et | Cl | H | H | Ph | H |
| 17.178 | Et | Cl | H | H | Ph | Cl |
| 17.179 | Et | Cl | H | H | Ph | F |
| 17.180 | Et | Cl | H | H | Ph | Me |
| 17.181 | H | F | F | H | H | H |
| 17.182 | H | F | H | F | H | H |

**Tabelle 18: Erfindungsgemäße Verbindungen der Formel (I), worin X für CH, A für A16, R³ und R⁴ jeweils für Wasserstoff sowie R² für Chlor steht:**

| | | | | | |
|---|---|---|---|---|---|
| 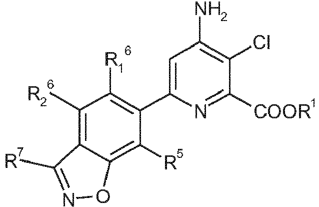 | | | | | |

| Bsp. Nr. | R¹ | R⁵ | R₁⁶ | R₂⁶ | R⁷ |
|---|---|---|---|---|---|
| 18.001 | H | H | H | H | H |
| 18.002 | H | H | H | H | Me |
| 18.003 | H | H | H | H | Ph |
| 18.004 | H | H | H | H | F |
| 18.005 | H | H | H | H | Cl |
| 18.006 | H | Cl | H | H | H |
| 18.007 | H | Cl | H | H | Me |
| 18.008 | H | Cl | H | H | Ph |
| 18.009 | H | Cl | H | H | F |
| 18.010 | H | Cl | H | H | Cl |
| 18.011 | H | F | H | H | H |
| 18.012 | H | F | H | H | Me |
| 18.013 | H | F | H | H | Ph |
| 18.014 | H | F | H | H | F |
| 18.015 | H | F | H | H | Cl |
| 18.016 | Me | H | H | H | H |
| 18.017 | Me | H | H | H | Me |
| 18.018 | Me | H | H | H | Ph |
| 18.019 | Me | H | H | H | F |
| 18.020 | Me | H | H | H | Cl |
| 18.021 | Me | Cl | H | H | H |
| 18.022 | Me | Cl | H | H | Me |
| 18.023 | Me | Cl | H | H | Ph |
| 18.024 | Me | Cl | H | H | F |
| 18.025 | Me | Cl | H | H | Cl |
| 18.026 | Et | H | H | H | H |
| 18.027 | Et | H | H | H | Me |
| 18.028 | Et | H | H | H | Ph |
| 18.029 | Et | H | H | H | F |
| 18.030 | Et | H | H | H | Cl |
| 18.031 | Et | F | H | H | H |
| 18.032 | Et | F | H | H | Me |
| 18.033 | Et | F | H | H | Ph |
| 18.034 | Et | F | H | H | F |
| 18.035 | Et | F | H | H | Cl |
| 18.036 | Et | Cl | H | H | H |
| 18.037 | Et | Cl | H | H | Me |
| 18.038 | Et | Cl | H | H | Ph |
| 18.039 | Et | Cl | H | H | F |
| 18.040 | Et | Cl | H | H | Cl |
| 18.041 | H | F | F | H | H |
| 18.042 | H | F | H | F | H |

**Tabelle 19: Erfindungsgemäße Verbindungen der Formel (I), worin X für CH, A für A17, R³ und R⁴ jeweils für Wasserstoff sowie R² für Chlor steht:**

| | | | | | |
|---|---|---|---|---|---|
| 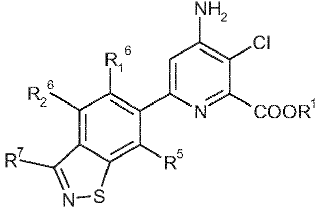 | | | | | |

| Bsp. Nr. | R¹ | R⁵ | R₁⁶ | R₂⁶ | R⁷ |
|---|---|---|---|---|---|
| 19.001 | H | H | H | H | H |
| 19.002 | H | H | H | H | Me |
| 19.003 | H | H | H | H | Ph |
| 19.004 | H | H | H | H | F |
| 19.005 | H | H | H | H | Cl |
| 19.006 | H | Cl | H | H | H |
| 19.007 | H | Cl | H | H | Me |
| 19.008 | H | Cl | H | H | Ph |
| 19.009 | H | Cl | H | H | F |
| 19.010 | H | Cl | H | H | Cl |
| 19.011 | H | F | H | H | H |
| 19.012 | H | F | H | H | Me |
| 19.013 | H | F | H | H | Ph |
| 19.014 | H | F | H | H | F |
| 19.015 | H | F | H | H | Cl |
| 19.016 | Me | H | H | H | H |
| 19.017 | Me | H | H | H | Me |
| 19.018 | Me | H | H | H | Ph |
| 19.019 | Me | H | H | H | F |
| 19.020 | Me | H | H | H | Cl |
| 19.021 | Me | Cl | H | H | H |
| 19.022 | Me | Cl | H | H | Me |
| 19.023 | Me | Cl | H | H | Ph |
| 19.024 | Me | Cl | H | H | F |
| 19.025 | Me | Cl | H | H | Cl |
| 19.026 | Et | H | H | H | H |
| 19.027 | Et | H | H | H | Me |
| 19.028 | Et | H | H | H | Ph |
| 19.029 | Et | H | H | H | F |
| 19.030 | Et | H | H | H | Cl |
| 19.031 | Et | F | H | H | H |
| 19.032 | Et | F | H | H | Me |
| 19.033 | Et | F | H | H | Ph |
| 19.034 | Et | F | H | H | F |
| 19.035 | Et | F | H | H | Cl |
| 19.036 | Et | Cl | H | H | H |
| 19.037 | Et | Cl | H | H | Me |
| 19.038 | Et | Cl | H | H | Ph |
| 19.039 | Et | Cl | H | H | F |
| 19.040 | Et | Cl | H | H | Cl |
| 19.041 | H | F | F | H | H |
| 19.042 | H | F | H | F | H |

**Tabelle 20: Erfindungsgemäße Verbindungen der Formel (I), worin X für CH, A für A18, R³ und R⁴ jeweils für Wasserstoff sowie R² für Chlor steht:**

| | | | | | | |
|---|---|---|---|---|---|---|
| 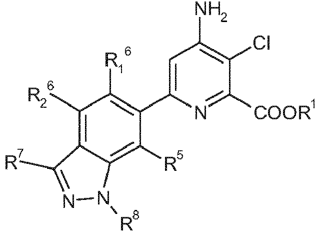 | | | | | | |

| Bsp. Nr. | R¹ | R⁵ | R₁⁶ | R₂⁶ | R⁷ | R⁸ |
|---|---|---|---|---|---|---|
| 20.001 | H | H | H | H | H | H |
| 20.002 | H | H | H | H | Me | H |
| 20.003 | H | H | H | H | Ph | H |
| 20.004 | H | H | H | H | F | Me |
| 20.005 | H | H | H | H | Cl | H |
| 20.006 | H | Cl | H | H | H | Me |
| 20.007 | H | Cl | H | H | Me | Me |
| 20.008 | H | Cl | H | H | Ph | Me |
| 20.009 | H | Cl | H | H | F | H |
| 20.010 | H | Cl | H | H | Cl | H |
| 20.011 | H | F | H | H | H | Me |
| 20.012 | H | F | H | H | Me | H |
| 20.013 | H | F | H | H | Ph | H |
| 20.014 | H | F | H | H | F | Me |
| 20.015 | H | F | H | H | Cl | Me |
| 20.016 | Me | H | H | H | H | Et |
| 20.017 | Me | H | H | H | Me | Et |
| 20.018 | Me | H | H | H | Ph | Et |
| 20.019 | Me | H | H | H | F | Et |
| 20.020 | Me | H | H | H | Cl | Et |
| 20.021 | Me | Cl | H | H | H | Me |
| 20.022 | Me | Cl | H | H | Me | H |
| 20.023 | Me | Cl | H | H | Ph | H |
| 20.024 | Me | Cl | H | H | F | H |
| 20.025 | Me | Cl | H | H | Cl | Et |
| 20.026 | Et | H | H | H | H | Me |
| 20.027 | Et | H | H | H | Me | Me |
| 20.028 | Et | H | H | H | Ph | Me |
| 20.029 | Et | H | H | H | F | H |
| 20.030 | Et | H | H | H | Cl | H |
| 20.031 | Et | F | H | H | H | Me |
| 20.032 | Et | F | H | H | Me | Me |
| 20.033 | Et | F | H | H | Ph | H |
| 20.034 | Et | F | H | H | F | Et |
| 20.035 | Et | F | H | H | Cl | Et |
| 20.036 | Et | Cl | H | H | H | H |
| 20.037 | Et | Cl | H | H | Me | H |
| 20.038 | Et | Cl | H | H | Ph | H |
| 20.039 | Et | Cl | H | H | F | Me |
| 20.040 | Et | Cl | H | H | Cl | H |
| 20.041 | H | F | F | H | H | H |
| 20.042 | H | F | H | F | H | H |

**Tabelle 21: Erfindungsgemäße Verbindungen der Formel (I), worin X für CH, A für A19, R³ und R⁴ jeweils für Wasserstoff sowie R² für Chlor steht:**

| | | | | | |
|---|---|---|---|---|---|
| 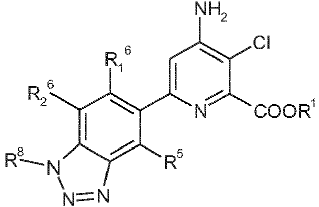 | | | | | |

| Bsp. Nr. | R¹ | R⁵ | R₁⁶ | R₂⁶ | R⁸ |
|---|---|---|---|---|---|
| 21.001 | H | H | H | H | H |
| 21.002 | H | H | H | H | Me |
| 21.003 | H | H | H | H | Et |
| 21.004 | H | F | H | H | H |
| 21.005 | H | F | H | H | Me |
| 21.006 | H | F | H | H | Et |
| 21.007 | H | Cl | H | H | H |
| 21.008 | H | Cl | H | H | Me |
| 21.009 | H | Cl | H | H | Et |
| 21.010 | Me | H | H | H | H |
| 21.011 | Me | H | H | H | Me |
| 21.012 | Me | H | H | H | Et |
| 21.013 | Me | F | H | H | H |
| 21.014 | Me | F | H | H | Me |
| 21.015 | Me | F | H | H | Et |
| 21.016 | Me | Cl | H | H | H |
| 21.017 | Me | Cl | H | H | Me |
| 21.018 | Me | Cl | H | H | Et |
| 21.019 | Et | H | H | H | H |
| 21.020 | Et | H | H | H | Me |
| 21.021 | Et | H | H | H | Et |
| 21.022 | Et | F | H | H | H |
| 21.023 | Et | F | H | H | Me |
| 21.024 | Et | F | H | H | Et |
| 21.025 | Et | Cl | H | H | H |
| 21.026 | Et | Cl | H | H | Me |
| 21.027 | Et | Cl | H | H | Et |
| 21.028 | H | F | F | H | H |
| 21.029 | H | F | H | F | H |

**Tabelle 22: Erfindungsgemäße Verbindungen der Formel (I), worin X für CH, A für A20, R³ und R⁴ jeweils für Wasserstoff sowie R² für Chlor steht:**

| | | | | | |
|---|---|---|---|---|---|
| 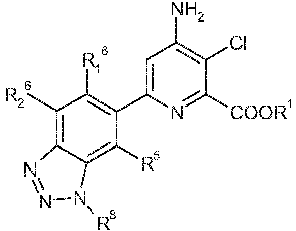 | | | | | |

| Bsp. Nr. | R¹ | R⁵ | R₁⁶ | R₂⁶ | R⁸ |
|---|---|---|---|---|---|
| 22.001 | H | H | H | H | H |
| 22.002 | H | H | H | H | Me |
| 22.003 | H | H | H | H | Et |
| 22.004 | H | F | H | H | H |
| 22.005 | H | F | H | H | Me |
| 22.006 | H | F | H | H | Et |
| 22.007 | H | Cl | H | H | H |
| 22.008 | H | Cl | H | H | Me |
| 22.009 | H | Cl | H | H | Et |
| 22.010 | Me | H | H | H | H |
| 22.011 | Me | H | H | H | Me |
| 22.012 | Me | H | H | H | Et |
| 22.013 | Me | F | H | H | H |
| 22.014 | Me | F | H | H | Me |
| 22.015 | Me | F | H | H | Et |
| 22.016 | Me | Cl | H | H | H |
| 22.017 | Me | Cl | H | H | Me |
| 22.018 | Me | Cl | H | H | Et |
| 22.019 | Et | H | H | H | H |
| 22.020 | Et | H | H | H | Me |
| 22.021 | Et | H | H | H | Et |
| 22.022 | Et | F | H | H | H |
| 22.023 | Et | F | H | H | Me |
| 22.024 | Et | F | H | H | Et |
| 22.025 | Et | Cl | H | H | H |
| 22.026 | Et | Cl | H | H | Me |
| 22.027 | Et | Cl | H | H | Et |
| 22.028 | H | F | F | H | H |
| 22.029 | H | F | H | F | H |

**Tabelle 23: Erfindungsgemäße Verbindungen der Formel (I), worin X für N, A für A1, R³ und R⁴ jeweils für Wasserstoff sowie R² für Chlor steht:**

| | | | | | | |
|---|---|---|---|---|---|---|
| 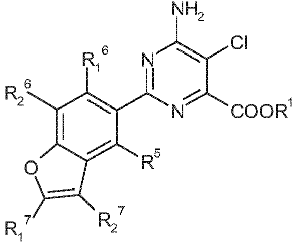 | | | | | | |

| Bsp. Nr. | R¹ | R⁵ | R₁⁶ | R₂⁶ | R₁⁷ | R₂⁷ |
|---|---|---|---|---|---|---|
| 23.001 | H | H | H | H | H | H |
| 23.002 | H | F | H | H | H | H |
| 23.003 | H | F | H | H | Cl | H |
| 23.004 | H | F | H | H | H | Cl |
| 23.005 | H | F | H | H | Cl | Cl |
| 23.006 | H | F | H | H | Ph | H |
| 23.007 | H | F | H | H | Ph | Cl |
| 23.008 | H | F | H | H | Ph | F |
| 23.009 | H | F | H | H | Me | H |
| 23.010 | H | F | H | H | Me | Cl |
| 23.011 | H | F | H | H | Me | F |
| 23.012 | Me | H | H | H | H | H |
| 23.013 | Me | F | H | H | H | H |
| 23.014 | Me | F | H | H | Cl | H |
| 23.015 | Me | F | H | H | H | Cl |
| 23.016 | Me | F | H | H | Cl | Cl |
| 23.017 | Me | F | H | H | H | F |
| 23.018 | Me | F | H | H | Ph | H |
| 23.019 | Me | F | H | H | Ph | Cl |
| 23.020 | Me | F | H | H | Ph | F |
| 23.021 | Me | F | H | H | Me | H |
| 23.022 | Me | F | H | H | Me | Cl |
| 23.023 | Me | F | H | H | Me | F |
| 23.024 | Et | H | H | H | H | H |
| 23.025 | Et | Cl | H | H | H | H |
| 23.026 | Et | Cl | H | H | Cl | H |
| 23.027 | Et | Cl | H | H | H | Cl |
| 23.028 | Et | Cl | H | H | Cl | Cl |
| 23.029 | Et | Cl | H | H | H | F |
| 23.030 | Et | Cl | H | H | Ph | H |
| 23.031 | Et | Cl | H | H | Ph | Cl |
| 23.032 | Et | Cl | H | H | Ph | F |
| 23.033 | Et | Cl | H | H | Me | H |
| 23.034 | Et | Cl | H | H | Me | Cl |
| 23.035 | Et | Cl | H | H | Me | F |
| 23.036 | H | F | F | H | H | H |
| 23.037 | H | F | H | F | H | H |

**Tabelle 24: Erfindungsgemäße Verbindungen der Formel (I), worin X für N, A für A2, R³ und R⁴ jeweils für Wasserstoff sowie R² für Chlor steht:**

| | | | | | | |
|---|---|---|---|---|---|---|
| 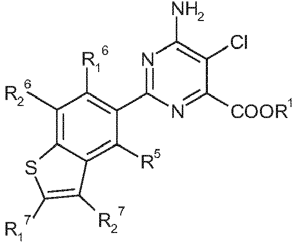 | | | | | | |

| Bsp. Nr. | R¹ | R⁵ | R₁⁶ | R₂⁶ | R₁⁷ | R₂⁷ |
|---|---|---|---|---|---|---|
| 24.001 | H | H | H | H | H | H |
| 24.002 | H | F | H | H | H | H |
| 24.003 | H | F | H | H | Cl | H |
| 24.004 | H | F | H | H | H | Cl |
| 24.005 | H | F | H | H | Cl | Cl |
| 24.006 | H | F | H | H | Ph | H |
| 24.007 | H | F | H | H | Ph | Cl |
| 24.008 | H | F | H | H | Ph | F |
| 24.009 | H | F | H | H | Me | H |
| 24.010 | H | F | H | H | Me | Cl |
| 24.011 | H | F | H | H | Me | F |
| 24.012 | Me | H | H | H | H | H |
| 24.013 | Me | F | H | H | H | H |
| 24.014 | Me | F | H | H | Cl | H |
| 24.015 | Me | F | H | H | H | Cl |
| 24.016 | Me | F | H | H | Cl | Cl |
| 24.017 | Me | F | H | H | H | F |
| 24.018 | Me | F | H | H | Ph | H |
| 24.019 | Me | F | H | H | Ph | Cl |
| 24.020 | Me | F | H | H | Ph | F |
| 24.021 | Me | F | H | H | Me | H |
| 24.022 | Me | F | H | H | Me | Cl |
| 24.023 | Me | F | H | H | Me | F |
| 24.024 | Et | H | H | H | H | H |
| 24.025 | Et | Cl | H | H | H | H |
| 24.026 | Et | Cl | H | H | Cl | H |
| 24.027 | Et | Cl | H | H | H | Cl |
| 24.028 | Et | Cl | H | H | Cl | Cl |
| 24.029 | Et | Cl | H | H | H | F |
| 24.030 | Et | Cl | H | H | Ph | H |
| 24.031 | Et | Cl | H | H | Ph | Cl |
| 24.032 | Et | Cl | H | H | Ph | F |
| 24.033 | Et | Cl | H | H | Me | H |
| 24.034 | Et | Cl | H | H | Me | Cl |
| 24.035 | Et | Cl | H | H | Me | F |
| 24.036 | H | F | F | H | H | H |
| 24.037 | H | F | H | F | H | H |

**Tabelle 25: Erfindungsgemäße Verbindungen der Formel (I), worin X für N, A für A3, R³ und R⁴ jeweils für Wasserstoff sowie R² für Chlor steht:**

| | | | | | | | |
|---|---|---|---|---|---|---|---|
| 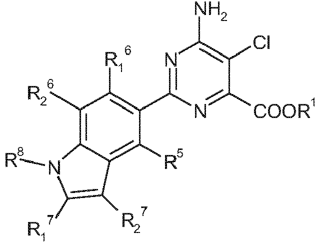 | | | | | | | |

| Bsp. Nr. | R¹ | R⁵ | R₁⁶ | R₂⁶ | R₁⁷ | R₂⁷ | R⁸ |
|---|---|---|---|---|---|---|---|
| 25.001 | H | H | H | H | H | H | H |
| 25.002 | H | F | H | H | H | H | H |
| 25.003 | H | F | H | H | Cl | H | H |
| 25.004 | H | F | H | H | H | Cl | Me |
| 25.005 | H | F | H | H | Cl | Cl | Me |
| 25.006 | H | F | H | H | Ph | H | Me |
| 25.007 | H | F | H | H | Ph | Cl | H |
| 25.008 | H | F | H | H | Ph | F | H |
| 25.009 | H | F | H | H | Me | H | H |
| 25.010 | H | F | H | H | Me | Cl | Me |
| 25.011 | H | F | H | H | Me | F | Me |
| 25.012 | Me | H | H | H | H | H | H |
| 25.013 | Me | F | H | H | H | H | H |
| 25.014 | Me | F | H | H | Cl | H | H |
| 25.015 | Me | F | H | H | H | Cl | Me |
| 25.016 | Me | F | H | H | Cl | Cl | Me |
| 25.017 | Me | F | H | H | H | F | Me |
| 25.018 | Me | F | H | H | Ph | H | H |
| 25.019 | Me | F | H | H | Ph | Cl | H |
| 25.020 | Me | F | H | H | Ph | F | H |
| 25.021 | Me | F | H | H | Me | H | H |
| 25.022 | Me | F | H | H | Me | Cl | Me |
| 25.023 | Me | F | H | H | Me | F | H |
| 25.024 | Et | H | H | H | H | H | H |
| 25.025 | Et | Cl | H | H | H | H | Me |
| 25.026 | Et | Cl | H | H | Cl | H | Me |
| 25.027 | Et | Cl | H | H | H | Cl | Me |
| 25.028 | Et | Cl | H | H | Cl | Cl | Me |
| 25.029 | Et | Cl | H | H | H | F | Me |
| 25.030 | Et | Cl | H | H | Ph | H | H |
| 25.031 | Et | Cl | H | H | Ph | Cl | H |
| 25.032 | Et | Cl | H | H | Ph | F | H |
| 25.033 | Et | Cl | H | H | Me | H | H |
| 25.034 | Et | Cl | H | H | Me | Cl | H |
| 25.035 | Et | Cl | H | H | Me | F | Me |
| 25.036 | H | F | F | H | H | H | H |
| 25.037 | H | F | H | F | H | H | H |

**Tabelle 26: Erfindungsgemäße Verbindungen der Formel (I), worin X für N, A für A4, R³ und R⁴ jeweils für Wasserstoff sowie R² für Chlor steht:**

| | | | | | |
|---|---|---|---|---|---|
| 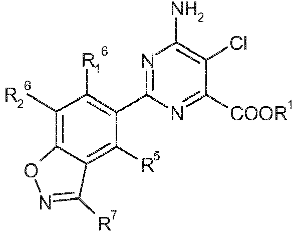 | | | | | |

| Bsp. Nr. | R¹ | R⁵ | R₁⁶ | R₂⁶ | R⁷ |
|---|---|---|---|---|---|
| 26.001 | H | H | H | H | H |
| 26.002 | H | F | H | H | H |
| 26.003 | H | F | H | H | F |
| 26.004 | H | F | H | H | Cl |
| 26.005 | H | F | H | H | Me |
| 26.006 | H | Cl | H | H | H |
| 26.007 | H | Cl | H | H | F |
| 26.008 | H | Cl | H | H | Cl |
| 26.009 | H | Cl | H | H | Me |
| 26.010 | Me | H | H | H | H |
| 26.011 | Me | F | H | H | H |
| 26.012 | Me | F | H | H | F |
| 26.013 | Me | F | H | H | Cl |
| 26.014 | Me | Cl | H | H | H |
| 26.015 | Me | Cl | H | H | F |
| 26.016 | Me | Cl | H | H | Cl |
| 26.017 | Me | Cl | H | H | Me |
| 26.018 | Et | H | H | H | H |
| 26.019 | Et | F | H | H | H |
| 26.020 | Et | Cl | H | H | H |
| 26.021 | Et | F | H | H | Me |
| 26.022 | Et | Cl | H | H | Me |
| 26.023 | H | F | F | H | H |
| 26.024 | H | F | H | F | H |

**Tabelle 27: Erfindungsgemäße Verbindungen der Formel (I), worin X für N, A für A5, R³ und R⁴ jeweils für Wasserstoff sowie R² für Chlor steht:**

| | | | | | |
|---|---|---|---|---|---|
| 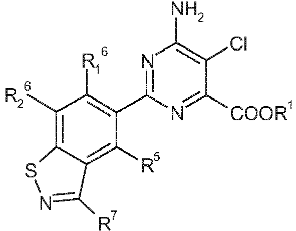 | | | | | |

| Bsp. Nr. | R¹ | R⁵ | R₁⁶ | R₂⁶ | R⁷ |
|---|---|---|---|---|---|
| 27.001 | H | H | H | H | H |
| 27.002 | H | F | H | H | H |
| 27.003 | H | F | H | H | F |
| 27.004 | H | F | H | H | Cl |
| 27.005 | H | F | H | H | F |
| 27.006 | H | Cl | H | H | H |
| 27.007 | H | Cl | H | H | F |
| 27.008 | H | Cl | H | H | Cl |
| 27.009 | H | Cl | H | H | Me |
| 27.010 | Me | H | H | H | H |
| 27.011 | Me | F | H | H | H |
| 27.012 | Me | F | H | H | Cl |
| 27.013 | Me | Cl | H | H | H |
| 27.014 | Me | Cl | H | H | F |
| 27.015 | Me | Cl | H | H | Cl |
| 27.016 | Me | Cl | H | H | Me |
| 27.017 | Et | H | H | H | H |
| 27.018 | Et | F | H | H | H |
| 27.019 | Et | Cl | H | H | H |
| 27.020 | Et | F | H | H | Me |
| 27.021 | Et | Cl | H | H | Me |
| 27.022 | H | F | F | H | H |
| 27.023 | H | F | H | F | H |

**Tabelle 28: Erfindungsgemäße Verbindungen der Formel (I), worin X für N, A für A6, R⁸, R³ und R⁴ jeweils für Wasserstoff sowie R² für Chlor steht:**

| | | | | | |
|---|---|---|---|---|---|
| 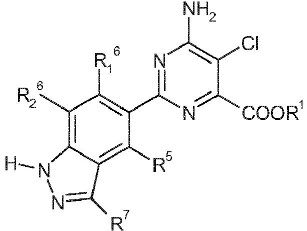 | | | | | |

| Bsp. Nr. | R¹ | R⁵ | R₁⁶ | R₂⁶ | R⁷ |
|---|---|---|---|---|---|
| 28.001 | H | H | H | H | H |
| 28.002 | H | F | H | H | H |
| 28.003 | H | F | H | H | Me |
| 28.004 | H | F | H | H | Et |
| 28.005 | H | H | H | H | Me |
| 28.006 | H | Cl | H | H | H |
| 28.007 | H | Cl | H | H | Me |
| 28.008 | H | Cl | H | H | Et |
| 28.009 | Me | H | H | H | H |
| 28.010 | Me | H | H | H | Me |
| 28.011 | Me | F | H | H | H |
| 28.012 | Me | F | H | H | Me |
| 28.013 | Me | F | H | H | Et |
| 28.014 | Me | Cl | H | H | H |
| 28.015 | Me | Cl | H | H | Me |
| 28.016 | Me | Cl | H | H | Et |
| 28.017 | Me | H | H | H | Me |
| 28.018 | Et | H | H | H | H |
| 28.019 | Et | H | H | H | Me |
| 28.020 | Et | H | H | H | Et |
| 28.021 | Et | F | H | H | H |
| 28.022 | Et | F | H | H | Me |
| 28.023 | Et | F | H | H | Et |
| 28.024 | Et | Cl | H | H | H |
| 28.025 | Et | Cl | H | H | Me |
| 28.026 | Et | Cl | H | H | Et |
| 28.027 | H | F | F | H | H |
| 28.028 | H | F | H | F | H |

**Tabelle 29: Erfindungsgemäße Verbindungen der Formel (I), worin X für N, A für A6, R³ und R⁴ jeweils für Wasserstoff, R⁸ für Methyl sowie R² für Chlor steht:**

| | | | | | |
|---|---|---|---|---|---|
| 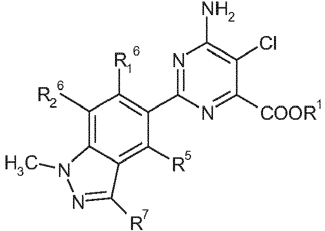 | | | | | |

| Bsp. Nr. | R¹ | R⁵ | R₁⁶ | R₂⁶ | R⁷ |
|---|---|---|---|---|---|
| 29.001 | H | H | H | H | H |
| 29.002 | H | F | H | H | H |
| 29.003 | H | F | H | H | Me |
| 29.004 | H | F | H | H | Et |
| 29.005 | H | H | H | H | Me |
| 29.006 | H | Cl | H | H | H |
| 29.007 | H | Cl | H | H | Me |
| 29.008 | H | Cl | H | H | Et |
| 29.009 | Me | H | H | H | H |
| 29.010 | Me | H | H | H | Me |
| 29.011 | Me | F | H | H | H |
| 29.012 | Me | F | H | H | Me |
| 29.013 | Me | F | H | H | Et |
| 29.014 | Me | Cl | H | H | H |
| 29.015 | Me | Cl | H | H | Me |
| 29.016 | Me | Cl | H | H | Et |
| 29.017 | Me | H | H | H | Me |
| 29.018 | Et | H | H | H | H |
| 29.019 | Et | H | H | H | Me |
| 29.020 | Et | H | H | H | Et |
| 29.021 | Et | F | H | H | H |
| 29.022 | Et | F | H | H | Me |
| 29.023 | Et | F | H | H | Et |
| 29.024 | Et | Cl | H | H | H |
| 29.025 | Et | Cl | H | H | Me |
| 29.026 | Et | Cl | H | H | Et |
| 29.027 | H | F | F | H | H |
| 29.028 | H | F | H | F | H |

**Tabelle 30: Erfindungsgemäße Verbindungen der Formel (I), worin X für N, A für A7, R³ und R⁴ jeweils für Wasserstoff sowie R² für Chlor steht:**

| | | | | | |
|---|---|---|---|---|---|
| 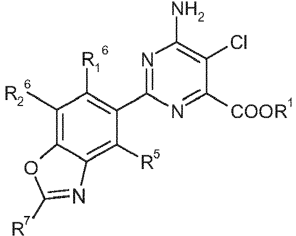 | | | | | |

| Bsp. Nr. | R¹ | R⁵ | R₁⁶ | R₂⁶ | R⁷ |
|---|---|---|---|---|---|
| 30.001 | H | H | H | H | H |
| 30.002 | H | H | H | H | Me |
| 30.003 | H | H | H | H | Ph |
| 30.004 | H | H | H | H | SMe |
| 30.005 | H | F | H | H | H |
| 30.006 | H | F | H | H | Me |
| 30.007 | H | F | H | H | Ph |
| 30.008 | H | F | H | H | SMe |
| 30.009 | H | Cl | H | H | H |
| 30.010 | H | Cl | H | H | Me |
| 30.011 | H | Cl | H | H | Ph |
| 30.012 | H | Cl | H | H | SMe |
| 30.013 | Me | H | H | H | H |
| 30.014 | Me | H | H | H | Me |
| 30.015 | Me | H | H | H | Ph |
| 30.016 | Me | H | H | H | SMe |
| 30.017 | Me | F | H | H | H |
| 30.018 | Me | F | H | H | Me |
| 30.019 | Me | F | H | H | Ph |
| 30.020 | Me | F | H | H | SMe |
| 30.021 | Me | Cl | H | H | H |
| 30.022 | Me | Cl | H | H | Me |
| 30.023 | Me | Cl | H | H | Ph |
| 30.024 | Me | Cl | H | H | SMe |
| 30.025 | Et | H | H | H | H |
| 30.026 | Et | H | H | H | Me |
| 30.027 | Et | H | H | H | Ph |
| 30.028 | Et | H | H | H | SMe |
| 30.029 | Et | F | H | H | H |
| 30.030 | Et | F | H | H | Me |
| 30.031 | Et | F | H | H | Ph |
| 30.032 | Et | F | H | H | SMe |
| 30.033 | Et | Cl | H | H | H |
| 30.034 | Et | Cl | H | H | Me |
| 30.035 | Et | Cl | H | H | Ph |
| 30.036 | Et | Cl | H | H | SMe |
| 30.037 | H | F | F | H | H |
| 30.038 | H | F | H | F | H |

**Tabelle 31: Erfindungsgemäße Verbindungen der Formel (I), worin X für N, A für A8, R³ und R⁴ jeweils für Wasserstoff sowie R² für Chlor steht:**

| | | | | | |
|---|---|---|---|---|---|
| 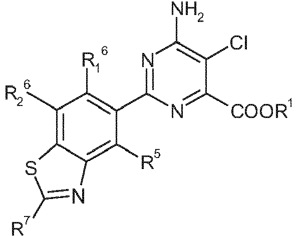 | | | | | |

| Bsp. Nr. | R¹ | R⁵ | R₁⁶ | R₂⁶ | R⁷ |
|---|---|---|---|---|---|
| 31.001 | H | H | H | H | H |
| 31.002 | H | H | H | H | Me |
| 31.003 | H | H | H | H | Ph |
| 31.004 | H | H | H | H | SMe |
| 31.005 | H | F | H | H | H |
| 31.006 | H | F | H | H | Me |
| 31.007 | H | F | H | H | Ph |
| 31.008 | H | F | H | H | SMe |
| 31.009 | H | Cl | H | H | H |
| 31.010 | H | Cl | H | H | Me |
| 31.011 | H | Cl | H | H | Ph |
| 31.012 | H | Cl | H | H | SMe |
| 31.013 | Me | H | H | H | H |
| 31.014 | Me | H | H | H | Me |
| 31.015 | Me | H | H | H | Ph |
| 31.016 | Me | H | H | H | SMe |
| 31.017 | Me | F | H | H | H |
| 31.018 | Me | F | H | H | Me |
| 31.019 | Me | F | H | H | Ph |
| 31.020 | Me | F | H | H | SMe |
| 31.021 | Me | Cl | H | H | H |
| 31.022 | Et | H | H | H | H |
| 31.023 | Et | H | H | H | Me |
| 31.024 | Et | H | H | H | Ph |
| 31.025 | Et | H | H | H | SMe |
| 31.026 | Et | F | H | H | H |
| 31.027 | Et | F | H | H | Me |
| 31.028 | Et | F | H | H | Ph |
| 31.029 | Et | F | H | H | SMe |
| 31.030 | Et | Cl | H | H | H |
| 31.031 | Et | Cl | H | H | Me |
| 31.032 | Et | Cl | H | H | Ph |
| 31.033 | Et | Cl | H | H | SMe |
| 31.034 | H | F | F | H | H |
| 31.035 | H | F | H | F | H |

**Tabelle 32: Erfindungsgemäße Verbindungen der Formel (I), worin X für N, A für A9, R³ und R⁴ jeweils für Wasserstoff sowie R² für Chlor steht:**

| | | | | | |
|---|---|---|---|---|---|
| 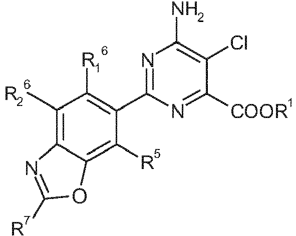 | | | | | |

| Bsp. Nr. | R¹ | R⁵ | R₁⁶ | R₂⁶ | R⁷ |
|---|---|---|---|---|---|
| 32.001 | H | H | H | H | H |
| 32.002 | H | H | H | H | Me |
| 32.003 | H | H | H | H | Ph |
| 32.004 | H | H | H | H | SMe |
| 32.005 | H | F | H | H | H |
| 32.006 | H | F | H | H | Me |
| 32.007 | H | F | H | H | Ph |
| 32.008 | H | F | H | H | SMe |
| 32.009 | H | Cl | H | H | H |
| 32.010 | H | Cl | H | H | Me |
| 32.011 | H | Cl | H | H | Ph |
| 32.012 | H | Cl | H | H | SMe |
| 32.013 | Me | H | H | H | H |
| 32.014 | Me | H | H | H | Me |
| 32.015 | Me | H | H | H | Ph |
| 32.016 | Me | H | H | H | SMe |
| 32.017 | Me | F | H | H | H |
| 32.018 | Me | F | H | H | Me |
| 32.019 | Me | F | H | H | Ph |
| 32.020 | Me | F | H | H | SMe |
| 32.021 | Me | Cl | H | H | H |
| 32.022 | Me | Cl | H | H | Me |
| 32.023 | Me | Cl | H | H | Ph |
| 32.024 | Me | Cl | H | H | SMe |
| 32.025 | Et | H | H | H | H |
| 32.026 | Et | H | H | H | Me |
| 32.027 | Et | H | H | H | Ph |
| 32.028 | Et | H | H | H | SMe |
| 32.029 | Et | F | H | H | H |
| 32.030 | Et | F | H | H | Me |
| 32.031 | Et | F | H | H | Ph |
| 32.032 | Et | F | H | H | SMe |
| 32.033 | Et | Cl | H | H | H |
| 32.034 | Et | Cl | H | H | Me |
| 32.035 | Et | Cl | H | H | Ph |
| 32.036 | Et | Cl | H | H | SMe |
| 32.037 | H | F | F | H | H |
| 32.038 | H | F | H | F | H |

**Tabelle 33: Erfindungsgemäße Verbindungen der Formel (I), worin X für N, A für A10, R³ und R⁴ jeweils für Wasserstoff sowie R² für Chlor steht:**

| | | | | | |
|---|---|---|---|---|---|
| 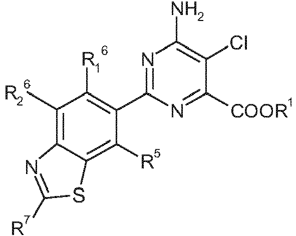 | | | | | |

| Bsp. Nr. | R¹ | R⁵ | R₁⁶ | R₂⁶ | R⁷ |
|---|---|---|---|---|---|
| 33.001 | H | H | H | H | H |
| 33.002 | H | H | H | H | Me |
| 33.003 | H | H | H | H | Ph |
| 33.004 | H | H | H | H | SMe |
| 33.005 | H | F | H | H | H |
| 33.006 | H | F | H | H | Me |
| 33.007 | H | F | H | H | Ph |
| 33.008 | H | F | H | H | SMe |
| 33.009 | H | Cl | H | H | H |
| 33.010 | H | Cl | H | H | Me |
| 33.011 | H | Cl | H | H | Ph |
| 33.012 | H | Cl | H | H | SMe |
| 33.013 | Me | H | H | H | H |
| 33.014 | Me | H | H | H | Me |
| 33.015 | Me | H | H | H | Ph |
| 33.016 | Me | H | H | H | SMe |
| 33.017 | Me | F | H | H | H |
| 33.018 | Me | F | H | H | Me |
| 33.019 | Me | F | H | H | Ph |
| 33.020 | Me | F | H | H | SMe |
| 33.021 | Me | Cl | H | H | H |
| 33.022 | Et | H | H | H | H |
| 33.023 | Et | H | H | H | Me |
| 33.024 | Et | H | H | H | Ph |
| 33.025 | Et | H | H | H | SMe |
| 33.026 | Et | F | H | H | H |
| 33.027 | Et | F | H | H | Me |
| 33.028 | Et | F | H | H | Ph |
| 33.029 | Et | F | H | H | SMe |
| 33.030 | Et | Cl | H | H | H |
| 33.031 | Et | Cl | H | H | Me |
| 33.032 | Et | Cl | H | H | Ph |
| 33.033 | Et | Cl | H | H | SMe |
| 33.034 | H | F | F | H | H |
| 33.035 | H | F | H | F | H |

**Tabelle 34: Erfindungsgemäße Verbindungen der Formel (I), worin X für N, A für A11, R³ und R⁴ jeweils für Wasserstoff sowie R² für Chlor steht:**

| | | | | | | |
|---|---|---|---|---|---|---|
| 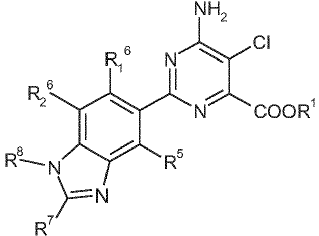 | | | | | | |

| Bsp. Nr. | R¹ | R⁵ | R₁⁶ | R₂⁶ | R⁷ | R⁸ |
|---|---|---|---|---|---|---|
| 34.001 | H | H | H | H | H | H |
| 34.002 | H | F | H | H | H | H |
| 34.003 | H | F | H | H | H | Me |
| 34.004 | H | F | H | H | Me | H |
| 34.005 | H | F | H | H | Me | Me |
| 34.006 | H | Cl | H | H | H | H |
| 34.007 | H | Cl | H | H | H | Me |
| 34.008 | H | Cl | H | H | Me | H |
| 34.009 | H | Cl | H | H | Me | Me |
| 34.010 | H | H | H | H | Ph | H |
| 34.011 | H | H | H | H | Ph | Me |
| 34.012 | H | F | H | H | Ph | H |
| 34.013 | H | F | H | H | Ph | Me |
| 34.014 | H | Cl | H | H | Ph | H |
| 34.015 | H | Cl | H | H | Ph | Me |
| 34.016 | H | H | H | H | Me | H |
| 34.017 | H | H | H | H | H | Me |
| 34.018 | H | H | H | H | Me | Me |
| 34.019 | Me | H | H | H | H | H |
| 34.020 | Me | F | H | H | H | H |
| 34.021 | Me | F | H | H | H | Me |
| 34.022 | Me | F | H | H | Me | H |
| 34.023 | Me | F | H | H | Me | Me |
| 34.024 | Me | Cl | H | H | H | H |
| 34.025 | Me | Cl | H | H | H | Me |
| 34.026 | Me | Cl | H | H | Me | H |
| 34.027 | Me | Cl | H | H | Me | Me |
| 34.028 | Me | H | H | H | Ph | H |
| 34.029 | Me | H | H | H | Ph | Me |
| 34.030 | Me | F | H | H | Ph | H |
| 34.031 | Me | F | H | H | Ph | Me |
| 34.032 | Me | Cl | H | H | Ph | H |
| 34.033 | Me | Cl | H | H | Ph | Me |
| 34.034 | Me | H | H | H | Me | H |
| 34.035 | Me | H | H | H | H | Me |
| 34.036 | Me | H | H | H | Me | Me |
| 34.037 | Et | H | H | H | H | H |
| 34.038 | Et | F | H | H | H | H |
| 34.039 | Et | F | H | H | H | Me |
| 34.040 | Et | F | H | H | Me | H |
| 34.041 | Et | F | H | H | Me | Me |
| 34.042 | Et | Cl | H | H | H | H |
| 34.043 | Et | Cl | H | H | H | Me |
| 34.044 | Et | Cl | H | H | Me | H |
| 34.045 | Et | H | H | H | Ph | H |
| 34.046 | Et | H | H | H | Ph | Me |
| 34.047 | Et | F | H | H | Ph | H |
| 34.048 | Et | F | H | H | Ph | Me |
| 34.049 | Et | Cl | H | H | Ph | H |
| 34.050 | Et | Cl | H | H | Me | Me |
| 34.051 | Et | H | H | H | Me | H |
| 34.052 | Et | H | H | H | H | Me |
| 34.053 | Et | H | H | H | Me | Me |
| 34.054 | H | F | F | H | H | H |
| 34.055 | H | F | H | F | H | H |

**Tabelle 35: Erfindungsgemäße Verbindungen der Formel (I), worin X für N, A für A12, R³ und R⁴ jeweils für Wasserstoff sowie R² für Chlor steht:**

| | | | | | | |
|---|---|---|---|---|---|---|
| 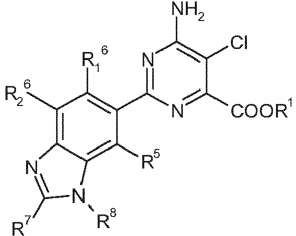 | | | | | | |

| Bsp. Nr. | R¹ | R⁵ | R₁⁶ | R₂⁶ | R⁷ | R⁸ |
|---|---|---|---|---|---|---|
| 35.001 | H | H | H | H | H | Me |
| 35.002 | H | F | H | H | H | Me |
| 35.003 | H | F | H | H | Me | Me |
| 35.004 | H | Cl | H | H | H | Me |
| 35.005 | H | Cl | H | H | Me | Me |
| 35.006 | H | H | H | H | Ph | Me |
| 35.007 | H | F | H | H | Ph | Me |
| 35.008 | H | Cl | H | H | Ph | Me |
| 35.009 | H | H | H | H | H | Me |
| 35.010 | Me | H | H | H | H | Me |
| 35.011 | Me | F | H | H | H | Me |
| 35.012 | Me | H | H | H | Me | Me |
| 35.013 | Me | F | H | H | Me | Me |
| 35.014 | Me | Cl | H | H | H | Me |
| 35.015 | Me | Cl | H | H | Me | Me |
| 35.016 | Me | H | H | H | Ph | Me |
| 35.017 | Me | F | H | H | Ph | Me |
| 35.018 | Me | Cl | H | H | Ph | Me |
| 35.019 | Et | F | H | H | H | Me |
| 35.020 | Et | F | H | H | Me | Me |
| 35.021 | Et | Cl | H | H | H | Me |
| 35.022 | Et | H | H | H | Ph | Me |
| 35.023 | Et | F | H | H | Ph | Me |
| 35.024 | Et | Cl | H | H | Ph | Me |
| 35.025 | Et | H | H | H | H | Me |
| 35.026 | Et | H | H | H | Me | Me |
| 35.027 | Et | Cl | H | H | Me | Me |
| 35.028 | H | F | F | H | H | H |
| 35.029 | H | F | H | F | H | H |

**Tabelle 36: Erfindungsgemäße Verbindungen der Formel (I), worin X für N, A für A13, R³ und R⁴ jeweils für Wasserstoff sowie R² für Chlor steht:**

| | | | | | | |
|---|---|---|---|---|---|---|
| 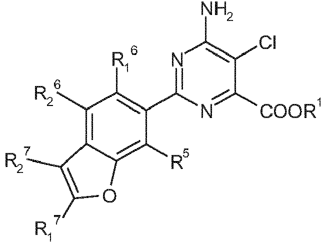 | | | | | | |

| Bsp. Nr. | R¹ | R⁵ | R₁⁶ | R₂⁶ | R₁⁷ | R₂⁷ |
|---|---|---|---|---|---|---|
| 36.001 | H | H | H | H | H | H |
| 36.002 | H | H | H | H | H | Cl |
| 36.003 | H | H | H | H | H | F |
| 36.004 | H | H | H | H | H | Me |
| 36.005 | H | H | H | H | F | H |
| 36.006 | H | H | H | H | F | Cl |
| 36.007 | H | H | H | H | F | F |
| 36.008 | H | H | H | H | F | Me |
| 36.009 | H | H | H | H | Cl | H |
| 36.010 | H | H | H | H | Cl | F |
| 36.011 | H | H | H | H | Cl | Cl |
| 36.012 | H | H | H | H | Cl | Me |
| 36.013 | H | H | H | H | Me | H |
| 36.014 | H | H | H | H | Me | Cl |
| 36.015 | H | H | H | H | Me | F |
| 36.016 | H | H | H | H | Me | Me |
| 36.017 | H | H | H | H | Ph | H |
| 36.018 | H | H | H | H | Ph | Cl |
| 36.019 | H | H | H | H | Ph | F |
| 36.020 | H | H | H | H | Ph | Me |
| 36.021 | H | F | H | H | H | H |
| 36.022 | H | F | H | H | H | Cl |
| 36.023 | H | F | H | H | H | F |
| 36.024 | H | F | H | H | H | Me |
| 36.025 | H | F | H | H | F | H |
| 36.026 | H | F | H | H | F | Cl |
| 36.027 | H | F | H | H | F | F |
| 36.028 | H | F | H | H | F | Me |
| 36.029 | H | F | H | H | Cl | H |
| 36.030 | H | F | H | H | Cl | F |
| 36.031 | H | F | H | H | Cl | Cl |
| 36.032 | H | F | H | H | Cl | Me |
| 36.033 | H | F | H | H | Me | H |
| 36.034 | H | F | H | H | Me | Cl |
| 36.035 | H | F | H | H | Me | F |
| 36.036 | H | F | H | H | Me | Me |
| 36.037 | H | F | H | H | Ph | H |
| 36.038 | H | F | H | H | Ph | Cl |
| 36.039 | H | F | H | H | Ph | F |
| 36.040 | H | F | H | H | Ph | Me |
| 36.041 | H | Cl | H | H | H | H |
| 36.042 | H | Cl | H | H | H | Cl |
| 36.043 | H | Cl | H | H | H | F |
| 36.044 | H | Cl | H | H | H | Me |
| 36.045 | H | Cl | H | H | F | H |
| 36.046 | H | Cl | H | H | F | Cl |
| 36.047 | H | Cl | H | H | F | F |
| 36.048 | H | Cl | H | H | F | Me |
| 36.049 | H | Cl | H | H | Cl | H |
| 36.050 | H | Cl | H | H | Cl | F |
| 36.051 | H | Cl | H | H | Cl | Cl |
| 36.052 | H | Cl | H | H | Cl | Me |
| 36.053 | H | Cl | H | H | Me | H |
| 36.054 | H | Cl | H | H | Me | Cl |
| 36.055 | H | Cl | H | H | Me | F |
| 36.056 | H | Cl | H | H | Me | Me |
| 36.057 | H | Cl | H | H | Ph | H |
| 36.058 | H | Cl | H | H | Ph | Cl |
| 36.059 | H | Cl | H | H | Ph | F |
| 36.060 | H | Cl | H | H | Ph | Me |
| 36.061 | Me | H | H | H | H | H |
| 36.062 | Me | H | H | H | H | Cl |
| 36.063 | Me | H | H | H | H | F |
| 36.064 | Me | H | H | H | H | Me |
| 36.065 | Me | H | H | H | F | H |
| 36.066 | Me | H | H | H | F | Cl |
| 36.067 | Me | H | H | H | F | F |
| 36.068 | Me | H | H | H | F | Me |
| 36.069 | Me | H | H | H | Cl | H |
| 36.070 | Me | H | H | H | Cl | F |
| 36.071 | Me | H | H | H | Cl | Cl |
| 36.072 | Me | H | H | H | Cl | Me |
| 36.073 | Me | H | H | H | Me | H |
| 36.074 | Me | H | H | H | Me | Cl |
| 36.075 | Me | H | H | H | Me | F |
| 36.076 | Me | H | H | H | Me | Me |
| 36.077 | Me | H | H | H | Ph | H |
| 36.078 | Me | H | H | H | Ph | Cl |
| 36.079 | Me | H | H | H | Ph | F |
| 36.080 | Me | H | H | H | Ph | Me |
| 36.081 | Me | F | H | H | H | H |
| 36.082 | Me | F | H | H | H | Cl |
| 36.083 | Me | F | H | H | H | F |
| 36.084 | Me | F | H | H | H | Me |
| 36.085 | Me | F | H | H | F | H |
| 36.086 | Me | F | H | H | F | Cl |
| 36.087 | Me | F | H | H | F | F |
| 36.088 | Me | F | H | H | F | Me |
| 36.089 | Me | F | H | H | Cl | H |
| 36.090 | Me | F | H | H | Cl | F |
| 36.091 | Me | F | H | H | Cl | Cl |
| 36.092 | Me | F | H | H | Cl | Me |
| 36.093 | Me | F | H | H | Me | H |
| 36.094 | Me | F | H | H | Me | Cl |
| 36.095 | Me | F | H | H | Me | F |
| 36.096 | Me | F | H | H | Me | Me |
| 36.097 | Me | F | H | H | Ph | H |
| 36.098 | Me | F | H | H | Ph | Cl |
| 36.099 | Me | F | H | H | Ph | F |
| 36.100 | Me | F | H | H | Ph | Me |
| 36.101 | Me | Cl | H | H | H | H |
| 36.102 | Me | Cl | H | H | H | Cl |
| 36.103 | Me | Cl | H | H | H | F |
| 36.104 | Me | Cl | H | H | H | Me |
| 36.105 | Me | Cl | H | H | F | H |
| 36.106 | Me | Cl | H | H | F | Cl |
| 36.107 | Me | Cl | H | H | F | F |
| 36.108 | Me | Cl | H | H | F | Me |
| 36.109 | Me | Cl | H | H | Cl | H |
| 36.110 | Me | Cl | H | H | Cl | F |
| 36.111 | Me | Cl | H | H | Cl | Cl |
| 36.112 | Me | Cl | H | H | Cl | Me |
| 36.113 | Me | Cl | H | H | Me | H |
| 36.114 | Me | Cl | H | H | Me | Cl |
| 36.115 | Me | Cl | H | H | Me | F |
| 36.116 | Me | Cl | H | H | Me | Me |
| 36.117 | Me | Cl | H | H | Ph | H |
| 36.118 | Me | Cl | H | H | Ph | Cl |
| 36.119 | Me | Cl | H | H | Ph | F |
| 36.120 | Me | Cl | H | H | Ph | Me |
| 36.121 | Et | H | H | H | H | H |
| 36.122 | Et | H | H | H | H | Cl |
| 36.123 | Et | H | H | H | H | F |
| 36.124 | Et | H | H | H | H | Me |
| 36.125 | Et | H | H | H | F | H |
| 36.126 | Et | H | H | H | F | Cl |
| 36.127 | Et | H | H | H | F | F |
| 36.128 | Et | H | H | H | F | Me |
| 36.129 | Et | H | H | H | Cl | H |
| 36.130 | Et | H | H | H | Cl | F |
| 36.131 | Et | H | H | H | Cl | Cl |
| 36.132 | Et | H | H | H | Cl | Me |
| 36.133 | Et | H | H | H | Me | H |
| 36.134 | Et | H | H | H | Me | Cl |
| 36.135 | Et | H | H | H | Me | F |
| 36.136 | Et | H | H | H | Me | Me |
| 36.137 | Et | H | H | H | Ph | H |
| 36.138 | Et | H | H | H | Ph | Cl |
| 36.139 | Et | H | H | H | Ph | F |
| 36.140 | Et | H | H | H | Ph | Me |
| 36.141 | Et | F | H | H | H | H |
| 36.142 | Et | F | H | H | H | Cl |
| 36.143 | Et | F | H | H | H | F |
| 36.144 | Et | F | H | H | H | Me |
| 36.145 | Et | F | H | H | F | H |
| 36.146 | Et | F | H | H | F | Cl |
| 36.147 | Et | F | H | H | F | F |
| 36.148 | Et | F | H | H | F | Me |
| 36.149 | Et | F | H | H | Cl | H |
| 36.150 | Et | F | H | H | Cl | F |
| 36.151 | Et | F | H | H | Cl | Cl |
| 36.152 | Et | F | H | H | Cl | Me |
| 36.153 | Et | F | H | H | Me | H |
| 36.154 | Et | F | H | H | Me | Cl |
| 36.155 | Et | F | H | H | Me | F |
| 36.156 | Et | F | H | H | Me | Me |
| 36.157 | Et | F | H | H | Ph | H |
| 36.158 | Et | F | H | H | Ph | Cl |
| 36.159 | Et | F | H | H | Ph | F |
| 36.160 | Et | F | H | H | Ph | Me |
| 36.161 | Et | Cl | H | H | H | H |
| 36.162 | Et | Cl | H | H | H | Cl |
| 36.163 | Et | Cl | H | H | H | F |
| 36.164 | Et | Cl | H | H | H | Me |
| 36.165 | Et | Cl | H | H | F | H |
| 36.166 | Et | Cl | H | H | F | Cl |
| 36.167 | Et | Cl | H | H | F | F |
| 36.168 | Et | Cl | H | H | F | Me |
| 36.169 | Et | Cl | H | H | Cl | H |
| 36.170 | Et | Cl | H | H | Cl | F |
| 36.171 | Et | Cl | H | H | Cl | Cl |
| 36.172 | Et | Cl | H | H | Cl | Me |
| 36.173 | Et | Cl | H | H | Me | H |
| 36.174 | Et | Cl | H | H | Me | Cl |
| 36.175 | Et | Cl | H | H | Me | F |
| 36.176 | Et | Cl | H | H | Me | Me |
| 36.177 | Et | Cl | H | H | Ph | H |
| 36.178 | Et | Cl | H | H | Ph | Cl |
| 36.179 | Et | Cl | H | H | Ph | F |
| 36.180 | Et | Cl | H | H | Ph | Me |
| 36.181 | H | F | F | H | H | H |
| 36.182 | H | F | H | F | H | H |

**Tabelle 37: Erfindungsgemäße Verbindungen der Formel (I), worin X für N, A für A14, R³ und R⁴ jeweils für Wasserstoff sowie R² für Chlor steht:**

| | | | | | | |
|---|---|---|---|---|---|---|
| 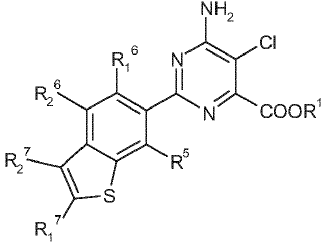 | | | | | | |

| Bsp. Nr. | R¹ | R⁵ | R₁⁶ | R₂⁶ | R₁⁷ | R₂⁷ |
|---|---|---|---|---|---|---|
| 37.001 | H | H | H | H | H | H |
| 37.002 | H | H | H | H | H | Cl |
| 37.003 | H | H | H | H | H | F |
| 37.004 | H | H | H | H | H | Me |
| 37.005 | H | H | H | H | F | H |
| 37.006 | H | H | H | H | F | Cl |
| 37.007 | H | H | H | H | F | F |
| 37.008 | H | H | H | H | F | Me |
| 37.009 | H | H | H | H | Cl | H |
| 37.010 | H | H | H | H | Cl | F |
| 37.011 | H | H | H | H | Cl | Cl |
| 37.012 | H | H | H | H | Cl | Me |
| 37.013 | H | H | H | H | Me | H |
| 37.014 | H | H | H | H | Me | Cl |
| 37.015 | H | H | H | H | Me | F |
| 37.016 | H | H | H | H | Me | Me |
| 37.017 | H | H | H | H | Ph | H |
| 37.018 | H | H | H | H | Ph | Cl |
| 37.019 | H | H | H | H | Ph | F |
| 37.020 | H | H | H | H | Ph | Me |
| 37.021 | H | F | H | H | H | H |
| 37.022 | H | F | H | H | H | Cl |
| 37.023 | H | F | H | H | H | F |
| 37.024 | H | F | H | H | H | Me |
| 37.025 | H | F | H | H | F | H |
| 37.026 | H | F | H | H | F | Cl |
| 37.027 | H | F | H | H | F | F |
| 37.028 | H | F | H | H | F | Me |
| 37.029 | H | F | H | H | Cl | H |
| 37.030 | H | F | H | H | Cl | F |
| 37.031 | H | F | H | H | Cl | Cl |
| 37.032 | H | F | H | H | Cl | Me |
| 37.033 | H | F | H | H | Me | H |
| 37.034 | H | F | H | H | Me | Cl |
| 37.035 | H | F | H | H | Me | F |
| 37.036 | H | F | H | H | Me | Me |
| 37.037 | H | F | H | H | Ph | H |
| 37.038 | H | F | H | H | Ph | Cl |
| 37.039 | H | F | H | H | Ph | F |
| 37.040 | H | F | H | H | Ph | Me |
| 37.041 | H | Cl | H | H | H | H |
| 37.042 | H | Cl | H | H | H | Cl |
| 37.043 | H | Cl | H | H | H | F |
| 37.044 | H | Cl | H | H | H | Me |
| 37.045 | H | Cl | H | H | F | H |
| 37.046 | H | Cl | H | H | F | Cl |
| 37.047 | H | Cl | H | H | F | F |
| 37.048 | H | Cl | H | H | F | Me |
| 37.049 | H | Cl | H | H | Cl | H |
| 37.050 | H | Cl | H | H | Cl | F |
| 37.051 | H | Cl | H | H | Cl | Cl |
| 37.052 | H | Cl | H | H | Cl | Me |
| 37.053 | H | Cl | H | H | Me | H |
| 37.054 | H | Cl | H | H | Me | Cl |
| 37.055 | H | Cl | H | H | Me | F |
| 37.056 | H | Cl | H | H | Me | Me |
| 37.057 | H | Cl | H | H | Ph | H |
| 37.058 | H | Cl | H | H | Ph | Cl |
| 37.059 | H | Cl | H | H | Ph | F |
| 37.060 | H | Cl | H | H | Ph | Me |
| 37.061 | Me | H | H | H | H | H |
| 37.062 | Me | H | H | H | H | Cl |
| 37.063 | Me | H | H | H | H | F |
| 37.064 | Me | H | H | H | H | Me |
| 37.065 | Me | H | H | H | F | H |
| 37.066 | Me | H | H | H | F | Cl |
| 37.067 | Me | H | H | H | F | F |
| 37.068 | Me | H | H | H | F | Me |
| 37.069 | Me | H | H | H | Cl | H |
| 37.070 | Me | H | H | H | Cl | F |
| 37.071 | Me | H | H | H | Cl | Cl |
| 37.072 | Me | H | H | H | Cl | Me |
| 37.073 | Me | H | H | H | Me | H |
| 37.074 | Me | H | H | H | Me | Cl |
| 37.075 | Me | H | H | H | Me | F |
| 37.076 | Me | H | H | H | Me | Me |
| 37.077 | Me | H | H | H | Ph | H |
| 37.078 | Me | H | H | H | Ph | Cl |
| 37.079 | Me | H | H | H | Ph | F |
| 37.080 | Me | H | H | H | Ph | Me |
| 37.081 | Me | F | H | H | H | H |
| 37.082 | Me | F | H | H | H | Cl |
| 37.083 | Me | F | H | H | H | F |
| 37.084 | Me | F | H | H | H | Me |
| 37.085 | Me | F | H | H | F | H |
| 37.086 | Me | F | H | H | F | Cl |
| 37.087 | Me | F | H | H | F | F |
| 37.088 | Me | F | H | H | F | Me |
| 37.089 | Me | F | H | H | Cl | H |
| 37.090 | Me | F | H | H | Cl | F |
| 37.091 | Me | F | H | H | Cl | Cl |
| 37.092 | Me | F | H | H | Cl | Me |
| 37.093 | Me | F | H | H | Me | H |
| 37.094 | Me | F | H | H | Me | Cl |
| 37.095 | Me | F | H | H | Me | F |
| 37.096 | Me | F | H | H | Me | Me |
| 37.097 | Me | F | H | H | Ph | H |
| 37.098 | Me | F | H | H | Ph | Cl |
| 37.099 | Me | F | H | H | Ph | F |
| 37.100 | Me | F | H | H | Ph | Me |
| 37.101 | Me | Cl | H | H | H | H |
| 37.102 | Me | Cl | H | H | H | Cl |
| 37.103 | Me | Cl | H | H | H | F |
| 37.104 | Me | Cl | H | H | H | Me |
| 37.105 | Me | Cl | H | H | F | H |
| 37.106 | Me | Cl | H | H | F | Cl |
| 37.107 | Me | Cl | H | H | F | F |
| 37.108 | Me | Cl | H | H | F | Me |
| 37.109 | Me | Cl | H | H | Cl | H |
| 37.110 | Me | Cl | H | H | Cl | F |
| 37.111 | Me | Cl | H | H | Cl | Cl |
| 37.112 | Me | Cl | H | H | Cl | Me |
| 37.113 | Me | Cl | H | H | Me | H |
| 37.114 | Me | Cl | H | H | Me | Cl |
| 37.115 | Me | Cl | H | H | Me | F |
| 37.116 | Me | Cl | H | H | Me | Me |
| 37.117 | Me | Cl | H | H | Ph | H |
| 37.118 | Me | Cl | H | H | Ph | Cl |
| 37.119 | Me | Cl | H | H | Ph | F |
| 37.120 | Me | Cl | H | H | Ph | Me |
| 37.121 | Et | H | H | H | H | H |
| 37.122 | Et | H | H | H | H | Cl |
| 37.123 | Et | H | H | H | H | F |
| 37.124 | Et | H | H | H | H | Me |
| 37.125 | Et | H | H | H | F | H |
| 37.126 | Et | H | H | H | F | Cl |
| 37.127 | Et | H | H | H | F | F |
| 37.128 | Et | H | H | H | F | Me |
| 37.129 | Et | H | H | H | Cl | H |
| 37.130 | Et | H | H | H | Cl | F |
| 37.131 | Et | H | H | H | Cl | Cl |
| 37.132 | Et | H | H | H | Cl | Me |
| 37.133 | Et | H | H | H | Me | H |
| 37.134 | Et | H | H | H | Me | Cl |
| 37.135 | Et | H | H | H | Me | F |
| 37.136 | Et | H | H | H | Me | Me |
| 37.137 | Et | H | H | H | Ph | H |
| 37.138 | Et | H | H | H | Ph | Cl |
| 37.139 | Et | H | H | H | Ph | F |
| 37.140 | Et | H | H | H | Ph | Me |
| 37.141 | Et | F | H | H | H | H |
| 37.142 | Et | F | H | H | H | Cl |
| 37.143 | Et | F | H | H | H | F |
| 37.144 | Et | F | H | H | H | Me |
| 37.145 | Et | F | H | H | F | H |
| 37.146 | Et | F | H | H | F | Cl |
| 37.147 | Et | F | H | H | F | F |
| 37.148 | Et | F | H | H | F | Me |
| 37.149 | Et | F | H | H | Cl | H |
| 37.150 | Et | F | H | H | Cl | F |
| 37.151 | Et | F | H | H | Cl | Cl |
| 37.152 | Et | F | H | H | Cl | Me |
| 37.153 | Et | F | H | H | Me | H |
| 37.154 | Et | F | H | H | Me | Cl |
| 37.155 | Et | F | H | H | Me | F |
| 37.156 | Et | F | H | H | Me | Me |
| 37.157 | Et | F | H | H | Ph | H |
| 37.158 | Et | F | H | H | Ph | Cl |
| 37.159 | Et | F | H | H | Ph | F |
| 37.160 | Et | F | H | H | Ph | Me |
| 37.161 | Et | Cl | H | H | H | H |
| 37.162 | Et | Cl | H | H | H | Cl |
| 37.163 | Et | Cl | H | H | H | F |
| 37.164 | Et | Cl | H | H | H | Me |
| 37.165 | Et | Cl | H | H | F | H |
| 37.166 | Et | Cl | H | H | F | Cl |
| 37.167 | Et | Cl | H | H | F | F |
| 37.168 | Et | Cl | H | H | F | Me |
| 37.169 | Et | Cl | H | H | Cl | H |
| 37.170 | Et | Cl | H | H | Cl | F |
| 37.171 | Et | Cl | H | H | Cl | Cl |
| 37.172 | Et | Cl | H | H | Cl | Me |
| 37.173 | Et | Cl | H | H | Me | H |
| 37.174 | Et | Cl | H | H | Me | Cl |
| 37.175 | Et | Cl | H | H | Me | F |
| 37.176 | Et | Cl | H | H | Me | Me |
| 37.177 | Et | Cl | H | H | Ph | H |
| 37.178 | Et | Cl | H | H | Ph | Cl |
| 37.179 | Et | Cl | H | H | Ph | F |
| 37.180 | Et | Cl | H | H | Ph | Me |
| 37.181 | H | F | F | H | H | H |
| 37.182 | H | F | H | F | H | H |

**Tabelle 38: Erfindungsgemäße Verbindungen der Formel (I), worin X für N, A für A15, R⁸, R³ und R⁴ jeweils für Wasserstoff sowie R² für Chlor steht:**

| | | | | | | |
|---|---|---|---|---|---|---|
| 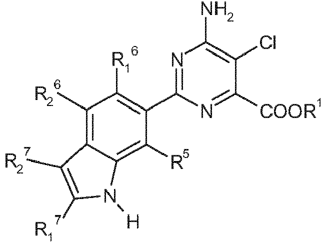 | | | | | | |

| Bsp. Nr. | R¹ | R⁵ | R₁⁶ | R₂⁶ | R₁⁷ | R₂⁷ |
|---|---|---|---|---|---|---|
| 38.001 | H | H | H | H | H | H |
| 38.002 | H | H | H | H | H | Cl |
| 38.003 | H | H | H | H | H | F |
| 38.004 | H | H | H | H | H | Me |
| 38.005 | H | H | H | H | F | H |
| 38.006 | H | H | H | H | F | Cl |
| 38.007 | H | H | H | H | F | F |
| 38.008 | H | H | H | H | F | Me |
| 38.009 | H | H | H | H | Cl | H |
| 38.010 | H | H | H | H | Cl | F |
| 38.011 | H | H | H | H | Cl | Cl |
| 38.012 | H | H | H | H | Cl | Me |
| 38.013 | H | H | H | H | Me | H |
| 38.014 | H | H | H | H | Me | Cl |
| 38.015 | H | H | H | H | Me | F |
| 38.016 | H | H | H | H | Me | Me |
| 38.017 | H | H | H | H | Ph | H |
| 38.018 | H | H | H | H | Ph | Cl |
| 38.019 | H | H | H | H | Ph | F |
| 38.020 | H | H | H | H | Ph | Me |
| 38.021 | H | F | H | H | H | H |
| 38.022 | H | F | H | H | H | Cl |
| 38.023 | H | F | H | H | H | F |
| 38.024 | H | F | H | H | H | Me |
| 38.025 | H | F | H | H | F | H |
| 38.026 | H | F | H | H | F | Cl |
| 38.027 | H | F | H | H | F | F |
| 38.028 | H | F | H | H | F | Me |
| 38.029 | H | F | H | H | Cl | H |
| 38.030 | H | F | H | H | Cl | F |
| 38.031 | H | F | H | H | Cl | Cl |
| 38.032 | H | F | H | H | Cl | Me |
| 38.033 | H | F | H | H | Me | H |
| 38.034 | H | F | H | H | Me | Cl |
| 38.035 | H | F | H | H | Me | F |
| 38.036 | H | F | H | H | Me | Me |
| 38.037 | H | F | H | H | Ph | H |
| 38.038 | H | F | H | H | Ph | Cl |
| 38.039 | H | F | H | H | Ph | F |
| 38.040 | H | F | H | H | Ph | Me |
| 38.041 | H | Cl | H | H | H | H |
| 38.042 | H | Cl | H | H | H | Cl |
| 38.043 | H | Cl | H | H | H | F |
| 38.044 | H | Cl | H | H | H | Me |
| 38.045 | H | Cl | H | H | F | H |
| 38.046 | H | Cl | H | H | F | Cl |
| 38.047 | H | Cl | H | H | F | F |
| 38.048 | H | Cl | H | H | F | Me |
| 38.049 | H | Cl | H | H | Cl | H |
| 38.050 | H | Cl | H | H | Cl | F |
| 38.051 | H | Cl | H | H | Cl | Cl |
| 38.052 | H | Cl | H | H | Cl | Me |
| 38.053 | H | Cl | H | H | Me | H |
| 38.054 | H | Cl | H | H | Me | Cl |
| 38.055 | H | Cl | H | H | Me | F |
| 38.056 | H | Cl | H | H | Me | Me |
| 38.057 | H | Cl | H | H | Ph | H |
| 38.058 | H | Cl | H | H | Ph | Cl |
| 38.059 | H | Cl | H | H | Ph | F |
| 38.060 | H | Cl | H | H | Ph | Me |
| 38.061 | Me | H | H | H | H | H |
| 38.062 | Me | H | H | H | H | Cl |
| 38.063 | Me | H | H | H | H | F |
| 38.064 | Me | H | H | H | H | Me |
| 38.065 | Me | H | H | H | F | H |
| 38.066 | Me | H | H | H | F | Cl |
| 38.067 | Me | H | H | H | F | F |
| 38.068 | Me | H | H | H | F | Me |
| 38.069 | Me | H | H | H | Cl | H |
| 38.070 | Me | H | H | H | Cl | F |
| 38.071 | Me | H | H | H | Cl | Cl |
| 38.072 | Me | H | H | H | Cl | Me |
| 38.073 | Me | H | H | H | Me | H |
| 38.074 | Me | H | H | H | Me | Cl |
| 38.075 | Me | H | H | H | Me | F |
| 38.076 | Me | H | H | H | Me | Me |
| 38.077 | Me | H | H | H | Ph | H |
| 38.078 | Me | H | H | H | Ph | Cl |
| 38.079 | Me | H | H | H | Ph | F |
| 38.080 | Me | H | H | H | Ph | Me |
| 38.081 | Me | F | H | H | H | H |
| 38.082 | Me | F | H | H | H | Cl |
| 38.083 | Me | F | H | H | H | F |
| 38.084 | Me | F | H | H | H | Me |
| 38.085 | Me | F | H | H | F | H |
| 38.086 | Me | F | H | H | F | Cl |
| 38.087 | Me | F | H | H | F | F |
| 38.088 | Me | F | H | H | F | Me |
| 38.089 | Me | F | H | H | Cl | H |
| 38.090 | Me | F | H | H | Cl | F |
| 38.091 | Me | F | H | H | Cl | Cl |
| 38.092 | Me | F | H | H | Cl | Me |
| 38.093 | Me | F | H | H | Me | H |
| 38.094 | Me | F | H | H | Me | Cl |
| 38.095 | Me | F | H | H | Me | F |
| 38.096 | Me | F | H | H | Me | Me |
| 38.097 | Me | F | H | H | Ph | H |
| 38.098 | Me | F | H | H | Ph | Cl |
| 38.099 | Me | F | H | H | Ph | F |
| 38.100 | Me | F | H | H | Ph | Me |
| 38.101 | Me | Cl | H | H | H | H |
| 38.102 | Me | Cl | H | H | H | Cl |
| 38.103 | Me | Cl | H | H | H | F |
| 38.104 | Me | Cl | H | H | H | Me |
| 38.105 | Me | Cl | H | H | F | H |
| 38.106 | Me | Cl | H | H | F | Cl |
| 38.107 | Me | Cl | H | H | F | F |
| 38.108 | Me | Cl | H | H | F | Me |
| 38.109 | Me | Cl | H | H | Cl | H |
| 38.110 | Me | Cl | H | H | Cl | F |
| 38.111 | Me | Cl | H | H | Cl | Cl |
| 38.112 | Me | Cl | H | H | Cl | Me |
| 38.113 | Me | Cl | H | H | Me | H |
| 38.114 | Me | Cl | H | H | Me | Cl |
| 38.115 | Me | Cl | H | H | Me | F |
| 38.116 | Me | Cl | H | H | Me | Me |
| 38.117 | Me | Cl | H | H | Ph | H |
| 38.118 | Me | Cl | H | H | Ph | Cl |
| 38.119 | Me | Cl | H | H | Ph | F |
| 38.120 | Me | Cl | H | H | Ph | Me |
| 38.121 | Et | H | H | H | H | H |
| 38.122 | Et | H | H | H | H | Cl |
| 38.123 | Et | H | H | H | H | F |
| 38.124 | Et | H | H | H | H | Me |
| 38.125 | Et | H | H | H | F | H |
| 38.126 | Et | H | H | H | F | Cl |
| 38.127 | Et | H | H | H | F | F |
| 38.128 | Et | H | H | H | F | Me |
| 38.129 | Et | H | H | H | Cl | H |
| 38.130 | Et | H | H | H | Cl | F |
| 38.131 | Et | H | H | H | Cl | Cl |
| 38.132 | Et | H | H | H | Cl | Me |
| 38.133 | Et | H | H | H | Me | H |
| 38.134 | Et | H | H | H | Me | Cl |
| 38.135 | Et | H | H | H | Me | F |
| 38.136 | Et | H | H | H | Me | Me |
| 38.137 | Et | H | H | H | Ph | H |
| 38.138 | Et | H | H | H | Ph | Cl |
| 38.139 | Et | H | H | H | Ph | F |
| 38.140 | Et | H | H | H | Ph | Me |
| 38.141 | Et | F | H | H | H | H |
| 38.142 | Et | F | H | H | H | Cl |
| 38.143 | Et | F | H | H | H | F |
| 38.144 | Et | F | H | H | H | Me |
| 38.145 | Et | F | H | H | F | H |
| 38.146 | Et | F | H | H | F | Cl |
| 38.147 | Et | F | H | H | F | F |
| 38.148 | Et | F | H | H | F | Me |
| 38.149 | Et | F | H | H | Cl | H |
| 38.150 | Et | F | H | H | Cl | F |
| 38.151 | Et | F | H | H | Cl | Cl |
| 38.152 | Et | F | H | H | Cl | Me |
| 38.153 | Et | F | H | H | Me | H |
| 38.154 | Et | F | H | H | Me | Cl |
| 38.155 | Et | F | H | H | Me | F |
| 38.156 | Et | F | H | H | Me | Me |
| 38.157 | Et | F | H | H | Ph | H |
| 38.158 | Et | F | H | H | Ph | Cl |
| 38.159 | Et | F | H | H | Ph | F |
| 38.160 | Et | F | H | H | Ph | Me |
| 38.161 | Et | Cl | H | H | H | H |
| 38.162 | Et | Cl | H | H | H | Cl |
| 38.163 | Et | Cl | H | H | H | F |
| 38.164 | Et | Cl | H | H | H | Me |
| 38.165 | Et | Cl | H | H | F | H |
| 38.166 | Et | Cl | H | H | F | Cl |
| 38.167 | Et | Cl | H | H | F | F |
| 38.168 | Et | Cl | H | H | F | Me |
| 38.169 | Et | Cl | H | H | Cl | H |
| 38.170 | Et | Cl | H | H | Cl | F |
| 38.171 | Et | Cl | H | H | Cl | Cl |
| 38.172 | Et | Cl | H | H | Cl | Me |
| 38.173 | Et | Cl | H | H | Me | H |
| 38.174 | Et | Cl | H | H | Me | Cl |
| 38.175 | Et | Cl | H | H | Me | F |
| 38.176 | Et | Cl | H | H | Me | Me |
| 38.177 | Et | Cl | H | H | Ph | H |
| 38.178 | Et | Cl | H | H | Ph | Cl |
| 38.179 | Et | Cl | H | H | Ph | F |
| 38.180 | Et | Cl | H | H | Ph | Me |
| 38.181 | H | F | F | H | H | H |
| 38.182 | H | F | H | F | H | H |

**Tabelle 39: Erfindungsgemäße Verbindungen der Formel (I), worin X für N, A für A15, R⁸ für Methyl, R³ und R⁴ jeweils für Wasserstoff sowie R² für Chlor steht:**

| | | | | | | |
|---|---|---|---|---|---|---|
| 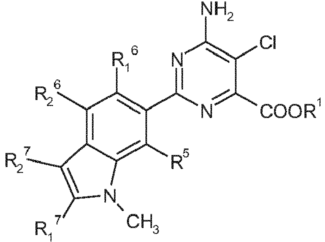 | | | | | | |

| Bsp. Nr. | R¹ | R⁵ | R₁⁶ | R₂⁶ | R₁⁷ | R₂⁷ |
|---|---|---|---|---|---|---|
| 39.001 | H | H | H | H | H | H |
| 39.002 | H | H | H | H | H | Cl |
| 39.003 | H | H | H | H | H | F |
| 39.004 | H | H | H | H | H | Me |
| 39.005 | H | H | H | H | F | H |
| 39.006 | H | H | H | H | F | Cl |
| 39.007 | H | H | H | H | F | F |
| 39.008 | H | H | H | H | F | Me |
| 39.009 | H | H | H | H | Cl | H |
| 39.010 | H | H | H | H | Cl | F |
| 39.011 | H | H | H | H | Cl | Cl |
| 39.012 | H | H | H | H | Cl | Me |
| 39.013 | H | H | H | H | Me | H |
| 39.014 | H | H | H | H | Me | Cl |
| 39.015 | H | H | H | H | Me | F |
| 39.016 | H | H | H | H | Me | Me |
| 39.017 | H | H | H | H | Ph | H |
| 39.018 | H | H | H | H | Ph | Cl |
| 39.019 | H | H | H | H | Ph | F |
| 39.020 | H | H | H | H | Ph | Me |
| 39.021 | H | F | H | H | H | H |
| 39.022 | H | F | H | H | H | Cl |
| 39.023 | H | F | H | H | H | F |
| 39.024 | H | F | H | H | H | Me |
| 39.025 | H | F | H | H | F | H |
| 39.026 | H | F | H | H | F | Cl |
| 39.027 | H | F | H | H | F | F |
| 39.028 | H | F | H | H | F | Me |
| 39.029 | H | F | H | H | Cl | H |
| 39.030 | H | F | H | H | Cl | F |
| 39.031 | H | F | H | H | Cl | Cl |
| 39.032 | H | F | H | H | Cl | Me |
| 39.033 | H | F | H | H | Me | H |
| 39.034 | H | F | H | H | Me | Cl |
| 39.035 | H | F | H | H | Me | F |
| 39.036 | H | F | H | H | Me | Me |
| 39.037 | H | F | H | H | Ph | H |
| 39.038 | H | F | H | H | Ph | Cl |
| 39.039 | H | F | H | H | Ph | F |
| 39.040 | H | F | H | H | Ph | Me |
| 39.041 | H | Cl | H | H | H | H |
| 39.042 | H | Cl | H | H | H | Cl |
| 39.043 | H | Cl | H | H | H | F |
| 39.044 | H | Cl | H | H | H | Me |
| 39.045 | H | Cl | H | H | F | H |
| 39.046 | H | Cl | H | H | F | Cl |
| 39.047 | H | Cl | H | H | F | F |
| 39.048 | H | Cl | H | H | F | Me |
| 39.049 | H | Cl | H | H | Cl | H |
| 39.050 | H | Cl | H | H | Cl | F |
| 39.051 | H | Cl | H | H | Cl | Cl |
| 39.052 | H | Cl | H | H | Cl | Me |
| 39.053 | H | Cl | H | H | Me | H |
| 39.054 | H | Cl | H | H | Me | Cl |
| 39.055 | H | Cl | H | H | Me | F |
| 39.056 | H | Cl | H | H | Me | Me |
| 39.057 | H | Cl | H | H | Ph | H |
| 39.058 | H | Cl | H | H | Ph | Cl |
| 39.059 | H | Cl | H | H | Ph | F |
| 39.060 | H | Cl | H | H | Ph | Me |
| 39.061 | Me | H | H | H | H | H |
| 39.062 | Me | H | H | H | H | Cl |
| 39.063 | Me | H | H | H | H | F |
| 39.064 | Me | H | H | H | H | Me |
| 39.065 | Me | H | H | H | F | H |
| 39.066 | Me | H | H | H | F | Cl |
| 39.067 | Me | H | H | H | F | F |
| 39.068 | Me | H | H | H | F | Me |
| 39.069 | Me | H | H | H | Cl | H |
| 39.070 | Me | H | H | H | Cl | F |
| 39.071 | Me | H | H | H | Cl | Cl |
| 39.072 | Me | H | H | H | Cl | Me |
| 39.073 | Me | H | H | H | Me | H |
| 39.074 | Me | H | H | H | Me | Cl |
| 39.075 | Me | H | H | H | Me | F |
| 39.076 | Me | H | H | H | Me | Me |
| 39.077 | Me | H | H | H | Ph | H |
| 39.078 | Me | H | H | H | Ph | Cl |
| 39.079 | Me | H | H | H | Ph | F |
| 39.080 | Me | H | H | H | Ph | Me |
| 39.081 | Me | F | H | H | H | H |
| 39.082 | Me | F | H | H | H | Cl |
| 39.083 | Me | F | H | H | H | F |
| 39.084 | Me | F | H | H | H | Me |
| 39.085 | Me | F | H | H | F | H |
| 39.086 | Me | F | H | H | F | Cl |
| 39.087 | Me | F | H | H | F | F |
| 39.088 | Me | F | H | H | F | Me |
| 39.089 | Me | F | H | H | Cl | H |
| 39.090 | Me | F | H | H | Cl | F |
| 39.091 | Me | F | H | H | Cl | Cl |
| 39.092 | Me | F | H | H | Cl | Me |
| 39.093 | Me | F | H | H | Me | H |
| 39.094 | Me | F | H | H | Me | Cl |
| 39.095 | Me | F | H | H | Me | F |
| 39.096 | Me | F | H | H | Me | Me |
| 39.097 | Me | F | H | H | Ph | H |
| 39.098 | Me | F | H | H | Ph | Cl |
| 39.099 | Me | F | H | H | Ph | F |
| 39.100 | Me | F | H | H | Ph | Me |
| 39.101 | Me | Cl | H | H | H | H |
| 39.102 | Me | Cl | H | H | H | Cl |
| 39.103 | Me | Cl | H | H | H | F |
| 39.104 | Me | Cl | H | H | H | Me |
| 39.105 | Me | Cl | H | H | F | H |
| 39.106 | Me | Cl | H | H | F | Cl |
| 39.107 | Me | Cl | H | H | F | F |
| 39.108 | Me | Cl | H | H | F | Me |
| 39.109 | Me | Cl | H | H | Cl | H |
| 39.110 | Me | Cl | H | H | Cl | F |
| 39.111 | Me | Cl | H | H | Cl | Cl |
| 39.112 | Me | Cl | H | H | Cl | Me |
| 39.113 | Me | Cl | H | H | Me | H |
| 39.114 | Me | Cl | H | H | Me | Cl |
| 39.115 | Me | Cl | H | H | Me | F |
| 39.116 | Me | Cl | H | H | Me | Me |
| 39.117 | Me | Cl | H | H | Ph | H |
| 39.118 | Me | Cl | H | H | Ph | Cl |
| 39.119 | Me | Cl | H | H | Ph | F |
| 39.120 | Me | Cl | H | H | Ph | Me |
| 39.121 | Et | H | H | H | H | H |
| 39.122 | Et | H | H | H | H | Cl |
| 39.123 | Et | H | H | H | H | F |
| 39.124 | Et | H | H | H | H | Me |
| 39.125 | Et | H | H | H | F | H |
| 39.126 | Et | H | H | H | F | Cl |
| 39.127 | Et | H | H | H | F | F |
| 39.128 | Et | H | H | H | F | Me |
| 39.129 | Et | H | H | H | Cl | H |
| 39.130 | Et | H | H | H | Cl | F |
| 39.131 | Et | H | H | H | Cl | Cl |
| 39.132 | Et | H | H | H | Cl | Me |
| 39.133 | Et | H | H | H | Me | H |
| 39.134 | Et | H | H | H | Me | Cl |
| 39.135 | Et | H | H | H | Me | F |
| 39.136 | Et | H | H | H | Me | Me |
| 39.137 | Et | H | H | H | Ph | H |
| 39.138 | Et | H | H | H | Ph | Cl |
| 39.139 | Et | H | H | H | Ph | F |
| 39.140 | Et | H | H | H | Ph | Me |
| 39.141 | Et | F | H | H | H | H |
| 39.142 | Et | F | H | H | H | Cl |
| 39.143 | Et | F | H | H | H | F |
| 39.144 | Et | F | H | H | H | Me |
| 39.145 | Et | F | H | H | F | H |
| 39.146 | Et | F | H | H | F | Cl |
| 39.147 | Et | F | H | H | F | F |
| 39.148 | Et | F | H | H | F | Me |
| 39.149 | Et | F | H | H | Cl | H |
| 39.150 | Et | F | H | H | Cl | F |
| 39.151 | Et | F | H | H | Cl | Cl |
| 39.152 | Et | F | H | H | Cl | Me |
| 39.153 | Et | F | H | H | Me | H |
| 39.154 | Et | F | H | H | Me | Cl |
| 39.155 | Et | F | H | H | Me | F |
| 39.156 | Et | F | H | H | Me | Me |
| 39.157 | Et | F | H | H | Ph | H |
| 39.158 | Et | F | H | H | Ph | Cl |
| 39.159 | Et | F | H | H | Ph | F |
| 39.160 | Et | F | H | H | Ph | Me |
| 39.161 | Et | Cl | H | H | H | H |
| 39.162 | Et | Cl | H | H | H | Cl |
| 39.163 | Et | Cl | H | H | H | F |
| 39.164 | Et | Cl | H | H | H | Me |
| 39.165 | Et | Cl | H | H | F | H |
| 39.166 | Et | Cl | H | H | F | Cl |
| 39.167 | Et | Cl | H | H | F | F |
| 39.168 | Et | Cl | H | H | F | Me |
| 39.169 | Et | Cl | H | H | Cl | H |
| 39.170 | Et | Cl | H | H | Cl | F |
| 39.171 | Et | Cl | H | H | Cl | Cl |
| 39.172 | Et | Cl | H | H | Cl | Me |
| 39.173 | Et | Cl | H | H | Me | H |
| 39.174 | Et | Cl | H | H | Me | Cl |
| 39.175 | Et | Cl | H | H | Me | F |
| 39.176 | Et | Cl | H | H | Me | Me |
| 39.177 | Et | Cl | H | H | Ph | H |
| 39.178 | Et | Cl | H | H | Ph | Cl |
| 39.179 | Et | Cl | H | H | Ph | F |
| 39.180 | Et | Cl | H | H | Ph | Me |
| 39.181 | H | F | F | H | H | H |
| 39.182 | H | F | H | F | H | H |

**Tabelle 40: Erfindungsgemäße Verbindungen der Formel (I), worin X für N, A für A16, R³ und R⁴ jeweils für Wasserstoff sowie R² für Chlor steht:**

| | | | | | |
|---|---|---|---|---|---|
| 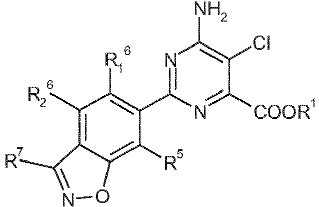 | | | | | |

| Bsp. Nr. | R¹ | R⁵ | R₁⁶ | R₂⁶ | R⁷ |
|---|---|---|---|---|---|
| 40.001 | H | H | H | H | H |
| 40.002 | H | H | H | H | Me |
| 40.003 | H | H | H | H | Ph |
| 40.004 | H | H | H | H | F |
| 40.005 | H | H | H | H | Cl |
| 40.006 | H | Cl | H | H | H |
| 40.007 | H | Cl | H | H | Me |
| 40.008 | H | Cl | H | H | Ph |
| 40.009 | H | Cl | H | H | F |
| 40.010 | H | Cl | H | H | Cl |
| 40.011 | H | F | H | H | H |
| 40.012 | H | F | H | H | Me |
| 40.013 | H | F | H | H | Ph |
| 40.014 | H | F | H | H | F |
| 40.015 | H | F | H | H | Cl |
| 40.016 | Me | H | H | H | H |
| 40.017 | Me | H | H | H | Me |
| 40.018 | Me | H | H | H | Ph |
| 40.019 | Me | H | H | H | F |
| 40.020 | Me | H | H | H | Cl |
| 40.021 | Me | Cl | H | H | H |
| 40.022 | Me | Cl | H | H | Me |
| 40.023 | Me | Cl | H | H | Ph |
| 40.024 | Me | Cl | H | H | F |
| 40.025 | Me | Cl | H | H | Cl |
| 40.026 | Et | H | H | H | H |
| 40.027 | Et | H | H | H | Me |
| 40.028 | Et | H | H | H | Ph |
| 40.029 | Et | H | H | H | F |
| 40.030 | Et | H | H | H | Cl |
| 40.031 | Et | F | H | H | H |
| 40.032 | Et | F | H | H | Me |
| 40.033 | Et | F | H | H | Ph |
| 40.034 | Et | F | H | H | F |
| 40.035 | Et | F | H | H | Cl |
| 40.036 | Et | Cl | H | H | H |
| 40.037 | Et | Cl | H | H | Me |
| 40.038 | Et | Cl | H | H | Ph |
| 40.039 | Et | Cl | H | H | F |
| 40.040 | Et | Cl | H | H | Cl |
| 40.041 | H | F | F | H | H |
| 40.042 | H | F | H | F | H |

**Tabelle 41: Erfindungsgemäße Verbindungen der Formel (I), worin X für N, A für A17, R³ und R⁴ jeweils für Wasserstoff sowie R² für Chlor steht:**

| | | | | | |
|---|---|---|---|---|---|
| 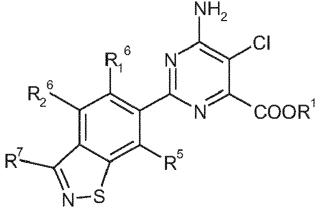 | | | | | |

| Bsp. Nr. | R¹ | R⁵ | R₁⁶ | R₂⁶ | R⁷ |
|---|---|---|---|---|---|
| 41.001 | H | H | H | H | H |
| 41.002 | H | H | H | H | Me |
| 41.003 | H | H | H | H | Ph |
| 41.004 | H | H | H | H | F |
| 41.005 | H | H | H | H | Cl |
| 41.006 | H | Cl | H | H | H |
| 41.007 | H | Cl | H | H | Me |
| 41.008 | H | Cl | H | H | Ph |
| 41.009 | H | Cl | H | H | F |
| 41.010 | H | Cl | H | H | Cl |
| 41.011 | H | F | H | H | H |
| 41.012 | H | F | H | H | Me |
| 41.013 | H | F | H | H | Ph |
| 41.014 | H | F | H | H | F |
| 41.015 | H | F | H | H | Cl |
| 41.016 | Me | H | H | H | H |
| 41.017 | Me | H | H | H | Me |
| 41.018 | Me | H | H | H | Ph |
| 41.019 | Me | H | H | H | F |
| 41.020 | Me | H | H | H | Cl |
| 41.021 | Me | Cl | H | H | H |
| 41.022 | Me | Cl | H | H | Me |
| 41.023 | Me | Cl | H | H | Ph |
| 41.024 | Me | Cl | H | H | F |
| 41.025 | Me | Cl | H | H | Cl |
| 41.026 | Et | H | H | H | H |
| 41.027 | Et | H | H | H | Me |
| 41.028 | Et | H | H | H | Ph |
| 41.029 | Et | H | H | H | F |
| 41.030 | Et | H | H | H | Cl |
| 41.031 | Et | F | H | H | H |
| 41.032 | Et | F | H | H | Me |
| 41.033 | Et | F | H | H | Ph |
| 41.034 | Et | F | H | H | F |
| 41.035 | Et | F | H | H | Cl |
| 41.036 | Et | Cl | H | H | H |
| 41.037 | Et | Cl | H | H | Me |
| 41.038 | Et | Cl | H | H | Ph |
| 41.039 | Et | Cl | H | H | F |
| 41.040 | Et | Cl | H | H | Cl |
| 41.041 | H | F | F | H | H |
| 41.042 | H | F | H | F | H |

**Tabelle 42: Erfindungsgemäße Verbindungen der Formel (I), worin X für N, A für A18, R³ und R⁴ jeweils für Wasserstoff sowie R² für Chlor steht:**

| | | | | | | |
|---|---|---|---|---|---|---|
| 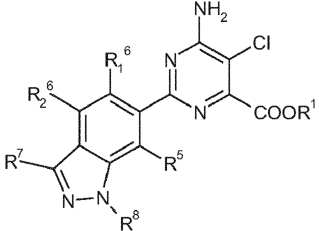 | | | | | | |

| Bsp. Nr. | R¹ | R⁵ | R₁⁶ | R₂⁶ | R⁷ | R⁸ |
|---|---|---|---|---|---|---|
| 42.001 | H | H | H | H | H | H |
| 42.002 | H | H | H | H | Me | H |
| 42.003 | H | H | H | H | Ph | H |
| 42.004 | H | H | H | H | F | Me |
| 42.005 | H | H | H | H | Cl | H |
| 42.006 | H | Cl | H | H | H | Me |
| 42.007 | H | Cl | H | H | Me | Me |
| 42.008 | H | Cl | H | H | Ph | Me |
| 42.009 | H | Cl | H | H | F | H |
| 42.010 | H | Cl | H | H | Cl | H |
| 42.011 | H | F | H | H | H | Me |
| 42.012 | H | F | H | H | Me | H |
| 42.013 | H | F | H | H | Ph | H |
| 42.014 | H | F | H | H | F | Me |
| 42.015 | H | F | H | H | Cl | Me |
| 42.016 | Me | H | H | H | H | Et |
| 42.017 | Me | H | H | H | Me | Et |
| 42.018 | Me | H | H | H | Ph | Et |
| 42.019 | Me | H | H | H | F | Et |
| 42.020 | Me | H | H | H | Cl | Et |
| 42.021 | Me | Cl | H | H | H | Me |
| 42.022 | Me | Cl | H | H | Me | H |
| 42.023 | Me | Cl | H | H | Ph | H |
| 42.024 | Me | Cl | H | H | F | H |
| 42.025 | Me | Cl | H | H | Cl | Et |
| 42.026 | Et | H | H | H | H | Me |
| 42.027 | Et | H | H | H | Me | Me |
| 42.028 | Et | H | H | H | Ph | Me |
| 42.029 | Et | H | H | H | F | H |
| 42.030 | Et | H | H | H | Cl | H |
| 42.031 | Et | F | H | H | H | Me |
| 42.032 | Et | F | H | H | Me | Me |
| 42.033 | Et | F | H | H | Ph | H |
| 42.034 | Et | F | H | H | F | Et |
| 42.035 | Et | F | H | H | Cl | Et |
| 42.036 | Et | Cl | H | H | H | H |
| 42.037 | Et | Cl | H | H | Me | H |
| 42.038 | Et | Cl | H | H | Ph | H |
| 42.039 | Et | Cl | H | H | F | Me |
| 42.040 | Et | Cl | H | H | Cl | H |
| 42.041 | H | F | F | H | H | H |
| 42.042 | H | F | H | F | H | H |

**Tabelle 43: Erfindungsgemäße Verbindungen der Formel (I), worin X für N, A für A19, R³ und R⁴ jeweils für Wasserstoff sowie R² für Chlor steht:**

| | | | | | |
|---|---|---|---|---|---|
| 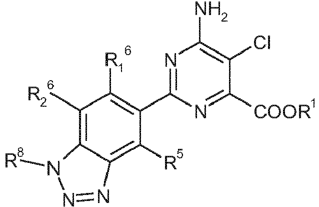 | | | | | |

| Bsp. Nr. | R¹ | R⁵ | R₁⁶ | R₂⁶ | R⁸ |
|---|---|---|---|---|---|
| 43.001 | H | H | H | H | H |
| 43.002 | H | H | H | H | Me |
| 43.003 | H | H | H | H | Et |
| 43.004 | H | F | H | H | H |
| 43.005 | H | F | H | H | Me |
| 43.006 | H | F | H | H | Et |
| 43.007 | H | Cl | H | H | H |
| 43.008 | H | Cl | H | H | Me |
| 43.009 | H | Cl | H | H | Et |
| 43.010 | Me | H | H | H | H |
| 43.011 | Me | H | H | H | Me |
| 43.012 | Me | H | H | H | Et |
| 43.013 | Me | F | H | H | H |
| 43.014 | Me | F | H | H | Me |
| 43.015 | Me | F | H | H | Et |
| 43.016 | Me | Cl | H | H | H |
| 43.017 | Me | Cl | H | H | Me |
| 43.018 | Me | Cl | H | H | Et |
| 43.019 | Et | H | H | H | H |
| 43.020 | Et | H | H | H | Me |
| 43.021 | Et | H | H | H | Et |
| 43.022 | Et | F | H | H | H |
| 43.023 | Et | F | H | H | Me |
| 43.024 | Et | F | H | H | Et |
| 43.025 | Et | Cl | H | H | H |
| 43.026 | Et | Cl | H | H | Me |
| 43.027 | Et | Cl | H | H | Et |
| 43.028 | H | F | F | H | H |
| 43.029 | H | F | H | F | H |

**Tabelle 44: Erfindungsgemäße Verbindungen der Formel (I), worin X für N, A für A20, R³ und R⁴ jeweils für Wasserstoff sowie R² für Chlor steht:**

| | | | | | |
|---|---|---|---|---|---|
| 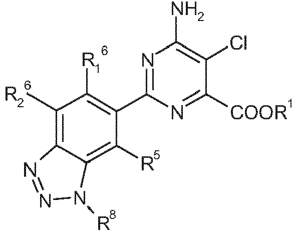 | | | | | |

| Bsp. Nr. | R¹ | R⁵ | R₁⁶ | R₂⁶ | R⁸ |
|---|---|---|---|---|---|
| 44.001 | H | H | H | H | Me |
| 44.002 | H | H | H | H | Et |
| 44.003 | H | F | H | H | Me |
| 44.004 | H | F | H | H | Et |
| 44.005 | H | Cl | H | H | Me |
| 44.006 | H | Cl | H | H | Et |
| 44.007 | Me | H | H | H | Me |
| 44.008 | Me | H | H | H | Et |
| 44.009 | Me | F | H | H | Me |
| 44.010 | Me | F | H | H | Et |
| 44.011 | Me | Cl | H | H | Me |
| 44.012 | Me | Cl | H | H | Et |
| 44.013 | Et | H | H | H | Me |
| 44.014 | Et | H | H | H | Et |
| 44.015 | Et | F | H | H | Me |
| 44.016 | Et | F | H | H | Et |
| 44.017 | Et | Cl | H | H | Me |
| 44.018 | Et | Cl | H | H | Et |
| 44.019 | H | F | F | H | Me |
| 44.020 | H | F | H | F | Me |

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum^{™} GAT^{™} (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum^{™} GAT^{™} (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.
Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Dalapon, Daimuron/Dymron, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethipin, Dimethametryn, Dimethenamid, Dimethenamid-P, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1 H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyrisopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), lodosulfuron, Iodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

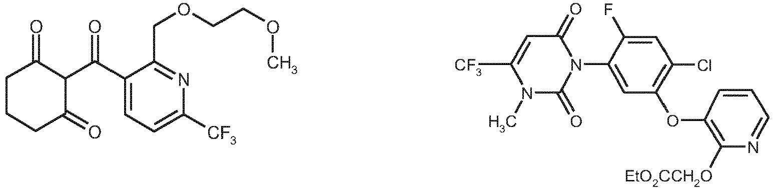

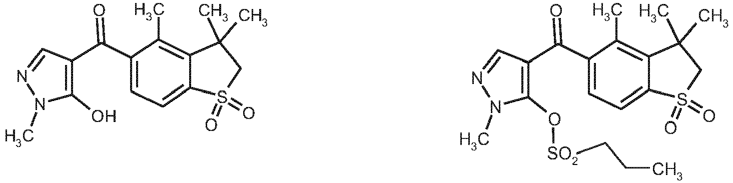

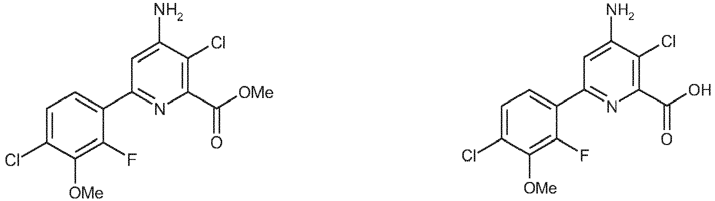

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung näher.

### A. Chemische Beispiele

### 1. Herstellung von Methyl-4-amino-3-chlor-6-(7-fluoro-2-phenyl-1,3-benzoxazol-6-yl)pyridin-2-carboxylat (Beispiel Nr. 8.019)

Zu einer Lösung von 0.3 g (1.13 mmol) Methyl-4-amino-6-brom-3-chlorpyridin-2-carboxylat in 20 ml Dioxan gibt man 0.024 g (0.03 mmol) (PPh₃)₂PdCl₂ und rührt diese Mischung 30 min bei Raumtemperatur (RT). Danach werden nacheinander 0.35 g (1.35 mmol) (7-Fluor-2-phenyl-1,3-benzoxazol-6-yl)boronsäure, 0.47 g (3,4 mmol) K₂CO₃ und 2 g (111 mmol) H₂O zu dieser Mischung hinzugefügt, die anschließend 6 h unter Rückfluss gerührt wird. Man lässt die Mischung weitere 12 h bei RT stehen und gibt sie dann auf 40 ml H₂O. Diese Mischung wird mehrmals mit CH2Cl2 extrahiert, die vereinigte organische Phase über Na₂SO₄ getrocknet und anschließend eingeengt. Chromatographische Reinigung an Kieselgel mit Heptan/Essigsäureethylester (3/7) als Laufmittel ergibt 0.16 g (36 %) Produkt. ¹H-NMR (CDCl₃): δ 8.30, 8.00, 7.55 (3m, 5H, C₆H₅), 7.60, 7.55 (2d, 2H, Benzoxazolring), 7.30 (s, 1 H, Pyridin) 4.85 (bs, 2H, NH₂), 4.00 (S, 3H, COOCH₃).

### 2. Herstellung von Methyl-4-amino-3-chlor-6-(1,3-benzoxazol-6-yl)pyridin-2-carboxylat (Beispiel Nr. 8.013)

Zu einer Lösung von 0.3 (1.13 mmol) Methyl-4-amino-6-brom-3-chlorpyridin-2-carboxylat in 20 ml Dioxan gibt man 0.024 g (0.03 mmol) (PPh₃)₂PdCl₂ und rührt diese Mischung 30 min bei RT. Danach werden nacheinander 0.28 g (1.13 mmol) 6-(4,4,5,5-Tetramethyl-1,3,2-dioxaborolan-2-yl)-1,3-benzoxazol, 0.47 g (3.4 mmol) K₂CO₃ und 2 g (111 mmol) H₂O zu dieser Mischung hinzugefügt, die dann 6 h unter Rückfluss gerührt wird. Man lässt die Mischung weitere 12 h bei RT stehen und gibt sie dann auf 40 ml H₂O. Diese Mischung wird mehrmals mit CH₂Cl₂ extrahiert, die vereinigte organische Phase über Na₂SO₄ getrocknet und anschließend eingeengt. Chromatographische Reinigung an Kieselgel mit Heptan/Essigsäureethylester (3/7) als Laufmittel ergibt 0.1 g (29 %) Produkt. ¹H-NMR (CDCl₃): δ 8.25 (s,1H, Benzoxazol), 8.15 (s, 1H, Benzoxazol), 7.90, 7.80 (2d, 2H, Benzoxazol), 7.20 (s, 1 H, Pyridin), 4.80 (bs, 2H, NH₂), 4.00 (s, 3H, COOCH₃).

### 3. Herstellung von Methyl-6-amino-5-chlor-2-(2-methyl-1,3-benzoxazol-5-yl)pyrimidin-4-carboxylat (Beispiel Nr. 30.014)

Zu einer Lösung von 0.3 (1.13 mmol) Methyl-6-amino-2-brom-5-chlorpyrimidin-4-carboxylat in 20 ml Dioxan gibt man 0.024 g (0.03 mmol) (PPh₃)₂PdCl₂ und rührt diese Mischung 30 min bei RT. Danach werden 0.29 g (1.13 mmol) 2-Methyl-5-(4,4,5,5-tetramethyl-1,3,2-dioxaborolan-2-yl)-1,3-benzoxazol, 0.47 g (3.4 mmol) K₂CO₃ und 2 g (111 mmol) H₂O hinzugefügt und anschließend 6 h unter Rückfluss gerührt. Man lässt die Reaktionsmischung weitere 12 h bei RT stehen und gibt sie dann auf 40 ml H₂O. Diese Mischung wird mehrmals mit CH₂Cl₂ extrahiert, die vereinigte organische Phase über Na₂SO₄ getrocknet und anschließend eingeengt. Chromatographische Reinigung an Kieselgel mit Heptan/Essigsäureethylester (3/7) als Laufmittel ergibt 0.06 g (17 %) Produkt. ¹H-NMR (CDCl₃): δ 8.65 (d, 1H, Benzoxazol), 8.40 (dd, 1H, Benzoxazol), 7.50 (d, 1 H, Benzoxazol), 5.55 (bs, 2H, NH₂), 4.05 (s, 3H, COOCH₃) 2.70 (s, 3H, -CH₃).

In nachfolgender Tabelle sind zur Charakterisierung die NMR-Daten einiger erfindungsgemäßer Verbindungen genannt.

| Bsp. Nr. | ¹H-NMR δ (ppm) |
|---|---|
| 1.012 | CDCl3: 8.40d, 1H; 7.83d, 1H; 7.65, 1H; 7.53d, 1H; 7.12s, 1H; 4.80bs, 2H, NH2; 4.05s, 3H, COOMe |
| 1.038 | CDCl3: 8.40d, 1H; 7.62d, 1H; 7.57d, 1H; 7.20s, 1H; 6.70d, 1H; 4.80bs, 2H, NH2; 4.00s, 3H, COOMe |
| 2.012 | CDCl₃: 8.40d, 1H; 7.92d, 1H; 7.88dd, 1H; 7.46d, 1H; 7.39d, 1H; 7.18s, 1H; 4.8bs, NH₂; 4.02s, 3H, COOMe |
| 2.038 | CDCl3: 8.40d, 1H; 7.60d, 1H; 7.40d, 1H; 7.35d, 1H; 7.25s, 1H; 4.85bs, 2H, NH2; 4.00s, 3H, COOMe |
| 8.007 | DMSO-d₆: 8.45d, 2H; 8.20m, 1H; 7.80s, 1H; 7.70m, 3H; 7.40m; 1H |
| 8.013 | CDCl₃: 8.30s, 1H; 8.13s, 1H; 8.05d, 2H; 7.62d, 1H; 7.13s, 1H; 4.82bs, NH₂; 4.0s, 3H, COOMe |
| 8.014 | CDCl₃: 8.10s, 1H; 7.90d, 1H; 7.50d, 1H; 7.10s,1H; 4.70bs, 2H, NH₂; 4.00s, 3H, COOMe; 2.65s, 3H, CH₃ |
| 8.019 | CDCl₃: 8.30m, 2H; 8.15m, 1H; 7.59m, 4H; 7.18m, 1H; 4.95bs, 2H, NH₂; 4.05s, 3H, COOMe |
| 8.039 | CDCl₃: 7.85s, 1H; 7.70d, 1H; 7.30d, 1H; 7.10s, 1H; 4.95bs, 2H, NH₂; 4.80bs, 2H, NH₂; 4.00s, 3H, COOMe |
| 9.013 | CDCl₃: 9.00s, 1H; 8.60s, 1H; 8.12d, 1H; 8.00d, 1H; 7.20s, 1H; 4.85bs, 2H, NH₂; 4.00s, 3H, COOMe |
| 9.014 | CDCl₃: 8.40s, 1H; 8.00d, 1H; 7.85d, 1H; 7.18s, 1H; 4.80bs, 2H, NH₂; 4.00s, 3H, COOMe |
| 10.013 | CDCl₃: 8.25s, 1H; 8.15s, 1H; 8.92d, 1H; 8.82d, 1H; 7.18s, 1H; 4.80bs, 2H, NH₂; 4.00s, 3H, COOMe |
| 10.014 | CDCl₃: 8.10s, 1H; 7.82d, 1H; 7.68d, 1H; 7.14s, 1H; 4.80bs, 2H, NH₂; 4.00s, 3H, COOMe; 2.68s, 3H, CH₃ |
| 10.019 | CDCl₃: 8.30m, 2H; 8.00dd, 1H; 7.60d, 1H; 7.57m, 3H; 7.30s, 1H; 4.85bs, 2H, NH₂; 4.00s 3H, COOMe |
| 10.023 | CDCl₃: 8.30m, 2H; 7.73d, 1H; 7.67s, 1H; 7.60m, 3H; 7.14s, 1 H; 4.90bs, 2H, NH₂; 4.00s, 3H, COOMe |
| 11.014 | CDCl₃: 8.50s, 1H; 7.98d, 1H; 7.90d, 1H; 7.15s, 1H; 4.85bs, 2H, NH₂; 4.00s, 3H, COOMe; 2.85s, 3H, CH₃ |
| 14.061 | CDCl3: 8.20s, 1H; 7.80d, 1H; 7.65d, 1H; 7.60d, 1H; 7.15s, 1H; 6.78d, 1H; 4.80bs, 2H, NH2; 4.02s, 3H, COOMe |
| 14.081 | CDCl3: 7.85dd, 1H; 7.70d, 2H; 7.40d, 2H; 7.30s, 1H; 6.70d, 1H; 4.80bs, 2H, NH2; 4.00s, 3H, COOMe |
| 15.061 | CDCl3: 8.50s, 1H; 7.89d, 1H; 7.85d, 1H; 7.50d, 1H; 7.35d, 1H; 7.20s, 1H; 4.80bs, 2H, NH2; 4.03s, 3H, COOMe |
| 16.064 | CDCl₃: 8.10s, 1H; 8.00bs, 1H, NH; 7.60m, 2H; 7.20s, 1H; 7.00s, 1H; 4.70bs, 2H, NH₂; 4.00s, 3H, COOMe; 2.35s, 3H, CH₃ |
| 16.073 | CDCl₃: 8.10bs, 1H, NH; 7.95s, 1 H; 7.50m, 2H; 7.10s, 1H; 6.20s, 1H; 4.75bs, 2H; NH₂; 4.00s, 3H, COOMe; 2.45s, 3H, CH₃ |
| 24.012 | CDCl₃: 8.84s, 1H; 8.35d, 1H; 7.93d, 1H; 7.48d, 1H; 7.42d, 1 H; 5.60bs, 2H, NH₂; 4.00s, 3H; COOMe |
| 30.014 | CDCl₃: 8.65s, 1H; 8.40d, 1H; 7.50d, 1H; 5.60bs, 2H, NH₂; 4.05s, 3H, COOMe; 2.70s, 3H, CH₃ |
| 31.013 | CDCl₃: 9.15s, 1H; 9.05s, 1H; 8.50d, 1H; 8.00d, 1H; 5.60bs, 2H, NH₂; 4.05s, 3H, COOMe |
| 32.013 | CDCl₃: 8.60s, 1H; 8.45d, 1H; 8.18s, 1H; 7.70d, 1H; 5.60bs, 2H, NH₂; 4.05s, 3H, COOMe |
| 33.014 | CDCl₃: 8.85s, 1H; 8.45d, 1H; 7.95d, 1H; 5.60bs, 2H, NH₂; 4.00s, 3H; COOMe; 2.85s, 3H, CH₃ |
| 38.061 | CDCl₃: 8.48s, 1H; 8.30bs, NH; 8.15d, 1H; 7.70d, 1H; 7.30d, 1H; 6.60d, 1H; 5.50bs, 2H, NH₂; 4.00s, 3H, COOMe |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man 75 Gew.-Teile einer Verbindung der allgemeinen Formel (I),

| | |
|---|---|
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der allgemeinen Formel (I),

| | |
|---|---|
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. 1.012, 1.038, 14.061 und 15.061 bei einer Aufwandmenge von 320 Gramm pro Hektar jeweils eine mindestens 90%-ige Wirkung gegen Echinocloa crus galli, Abutilon theophrasti, Amaranthus retroflexus, Matricaria inodora, Stellaria media und Veronica persica

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. 1.012, 14.061 und 15.061 bei einer Aufwandmenge von 80 Gramm pro Hektar jeweils eine mindestens 90%-ige Wirkung gegen Echinocloa crus galli, Abutilon theophrasti, Amaranthus retroflexus, und Matricaria inodora.

## Patentansprüche

1. Verbindungen der Formel (I), deren N-Oxide oder deren Salze 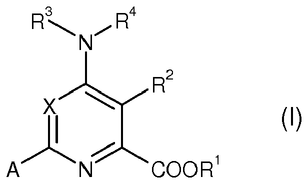 worin
A bedeutet einen Rest der Gruppe bestehend aus A1 bis A20, 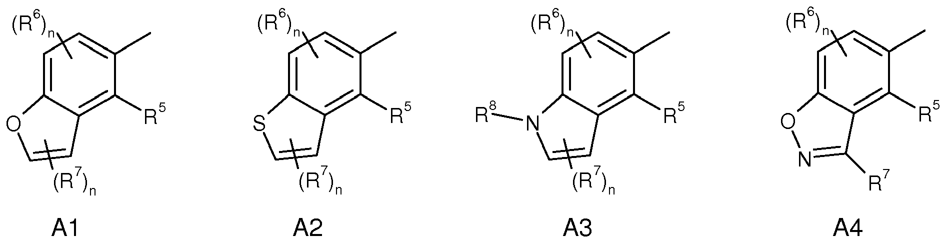 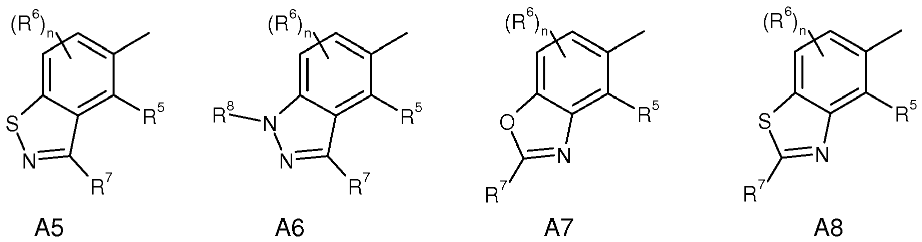 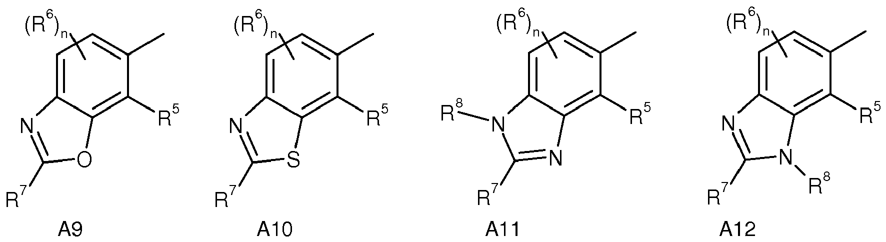 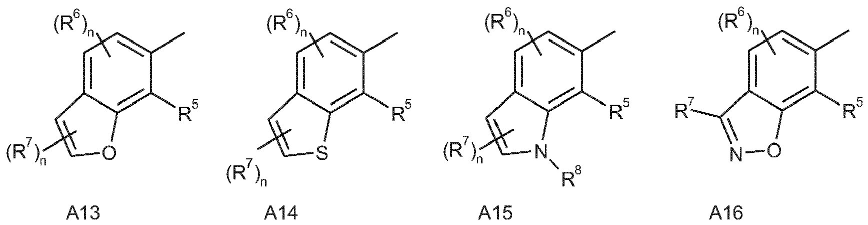 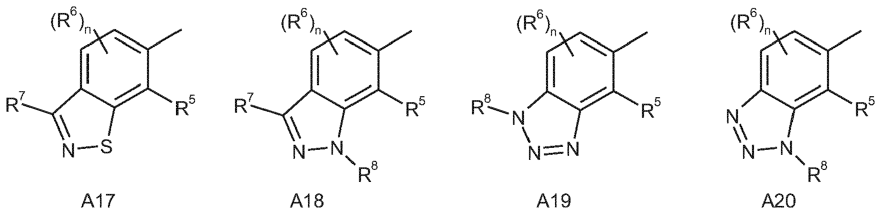
R¹ bedeutet Wasserstoff oder (C₁-C₄)-Alkyl,
R² bedeutet Chlor,
R³ bedeutet Wasserstoff,
R⁴ bedeutet Wasserstoff,
R⁵ bedeutet Wasserstoff, Halogen, OH, NH₂, CN, (C₁-C₃)-Alkyl, (C₁-C₃)-Alkoxy, C₁-C₃)-Alkylamino oder Cyclopropyl,
R⁶ bedeutet Wasserstoff, Halogen, OH, NH₂, CN, (C₁-C₃)-Alkyl, (C₁-C₃)-Alkoxy, Cyclopropyl oder Vinyl,
R⁷ bedeutet Wasserstoff, Halogen, (C₁-C₃)-Alkyl, (C₁-C₃)-Alkoxy, (C₁-C₃)-Alkylthio, Cyclopropyl, (C₁-C₃)-Alkylamino oder Phenyl,
R⁸ bedeutet Wasserstoff, (C₁-C₃)-Alkyl, Phenyl oder (C₁-C₃)-Alkylcarbonyl,
X bedeutet N, CH, CCI, CF oder CBr,
n bedeutet 0, 1 oder 2.

2. Herbizide Mittel, **gekennzeichnet durch** einen herbizid wirksamen Gehalt an mindestens einer Verbindung der Formel (I) gemäß Anspruch 1.

3. Herbizide Mittel nach Anspruch 2 in Mischung mit Formulierungshilfsmitteln.

4. Herbizide Mittel nach Anspruch 2 oder 3 enthaltend mindestens einen weiteren pestizid wirksame Stoffen aus der Gruppe Insektizide, Akarizide, Herbizide, Fungizide, Safenern und Wachstumsregulatoren.

5. Herbizide Mittel nach Anspruch 4 enthaltend einen Safener.

6. Herbizide Mittel nach Anspruch 5 enthaltend ein weiteres Herbizid.

7. Verfahren zur Bekämpfung unerwünschter Pflanzen, **dadurch gekennzeichnet, daß** man eine wirksame Menge mindestens einer Verbindung der Formel (I) gemäß einem Anspruch 1 oder eines herbiziden Mittels nach einem der Ansprüche 2 bis 6 auf die Pflanzen oder auf den Ort des unerwünschten Pflanzenwachstums appliziert.

8. Verwendung von Verbindungen der Formel (I) gemäß Anspruch 1 oder eines herbiziden Mittels nach einem der Ansprüche 2 bis 6 zur Bekämpfung unerwünschter Pflanzen.

9. Verwendung nach Anspruch 8, **dadurch gekennzeichnet, daß** die Verbindungen der Formel (I) zur Bekämpfung unerwünschter Pflanzen in Kulturen von Nutzpflanzen eingesetzt werden.

10. Verwendung nach Anspruch 9, **dadurch gekennzeichnet, daß** die Nutzpflanzen transgene Nutzpflanzen sind.

## Claims

1. Compounds of the formula (I), their N-oxides or their salts 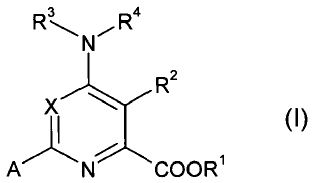 in which
A represents a radical from the group consisting of A1 to A20, 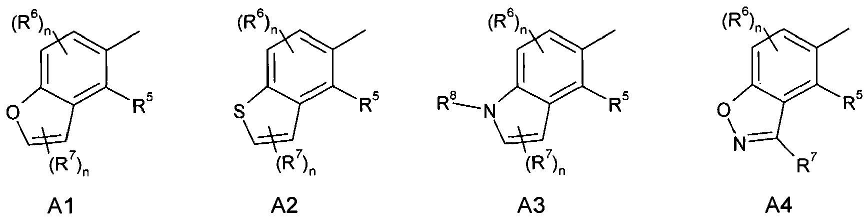 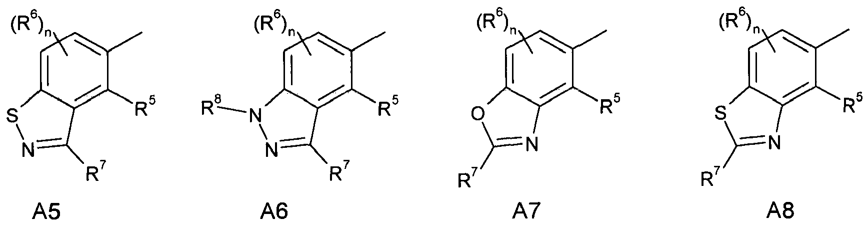 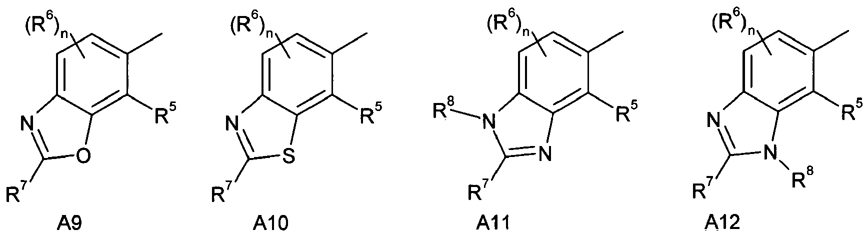 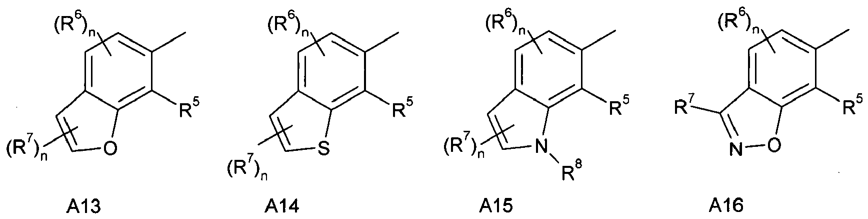 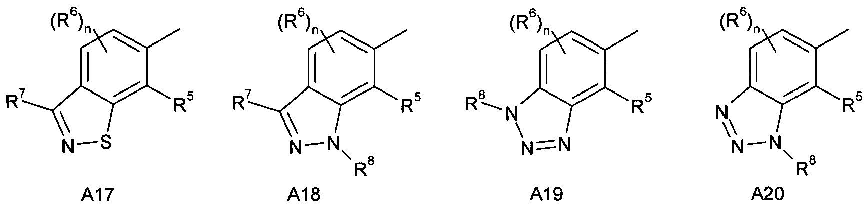 R¹ represents hydrogen or (C₁-C₄)-alkyl,
R² represents chlorine,
R³ represents hydrogen,
R⁴ represents hydrogen,
R⁵ represents hydrogen, halogen, OH, NH₂, CN, (C₁-C₃)-alkyl, (C₁-C₃)-alkoxy, C₁-C₃)-alkylamino or cyclopropyl,
R⁶ represents hydrogen, halogen, OH, NH₂, CN, (C₁-C₃)-alkyl, (C₁-C₃)-alkoxy, cyclopropyl or vinyl,
R⁷ represents hydrogen, halogen, (C₁-C₃)-alkyl, (C₁-C₃)-alkoxy, (C₁-C₃) -alkylthio, cyclopropyl, (C₁-C₃)-alkylamino or phenyl,
R⁸ represents hydrogen, (C₁-C₃) -alkyl, phenyl or (C₁-C₃)-alkylcarbonyl,
X represents N, CH, CC1, CF or CBr,
n represents 0, 1 or 2.

2. Herbicidal compositions, **characterized in that** they comprise a herbicidally effective amount of at least one compound of the formula (I) according to Claim 1.

3. Herbicidal compositions according to Claim 2 in a mixture with formulation auxiliaries.

4. Herbicidal compositions according to Claim 2 or 3, comprising at least one further pesticidally active compound from the group of the insecticides, acaricides, herbicides, fungicides, safeners and growth regulators.

5. Herbicidal compositions according to Claim 4, comprising a safener.

6. Herbicidal compositions according to Claim 5, comprising a further herbicide.

7. Method for controlling unwanted plants, **characterized in that** an effective amount of at least one compound of the formula (I) according to Claim 1 or a herbicidal composition according to any of Claims 2 to 6 is applied to the plants or on the site of the unwanted plant growth.

8. Use of compounds of the formula (I) according to Claim 1 or of a herbicidal composition according to any of Claims 2 to 6 for controlling unwanted plants.

9. Use according to Claim 8, **characterized in that** the compounds of the formula (I) are used for controlling unwanted plants in crops of useful plants.

10. Use according to Claim 9, **characterized in that** the useful plants are transgenic useful plants.

## Revendications

1. Composés de formule (I), leurs N-oxydes ou leurs sels 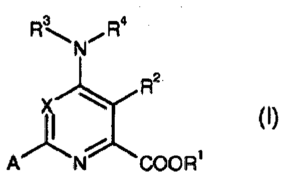 formule dans laquelle
A représente un radical choisi dans le groupe constitué par A1 à A20, 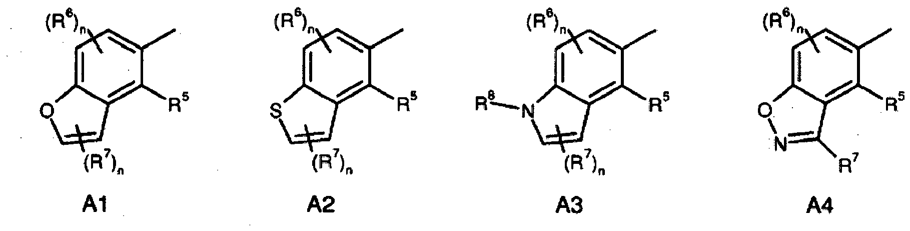 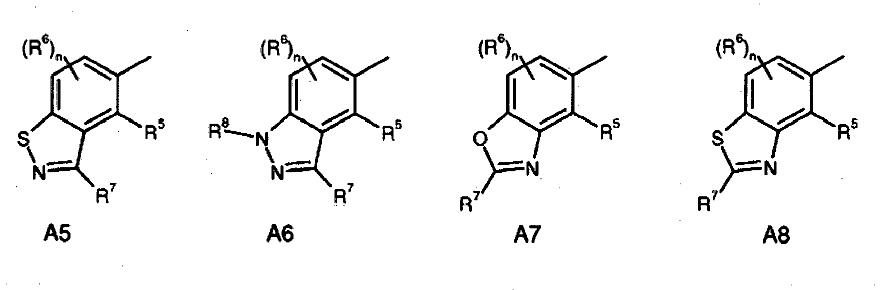 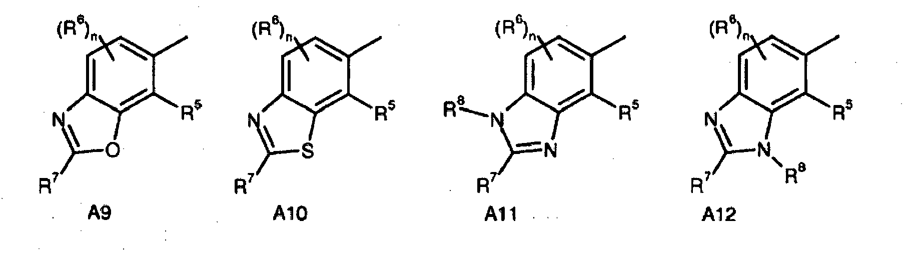 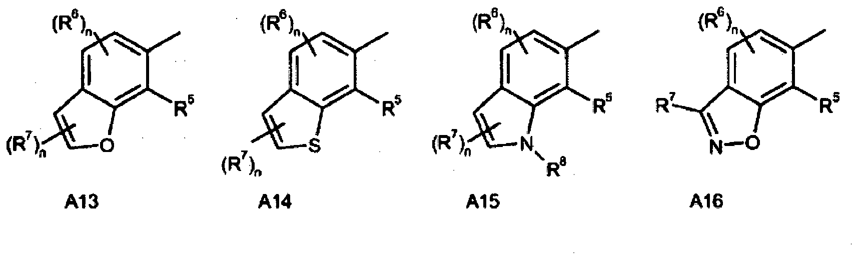 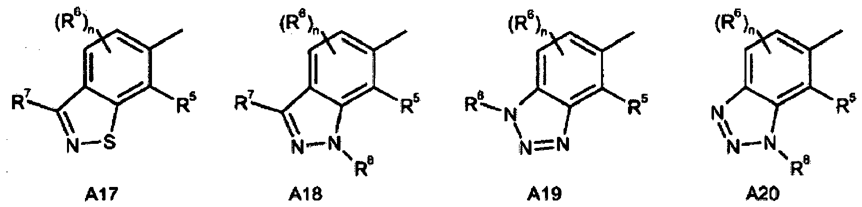
R¹ représente un atome d'hydrogène ou un groupe alkyle en C₁-C₄,
R² représente un atome de chlore,
R³ représente un atome d'hydrogène,
R⁴ représente un atome d'hydrogène,
R⁵ représente un atome d'hydrogène ou d'halogène, un groupe OH, NH₂, CN, alkyle en C₁-C₃, alcoxy en C₁-C₃, alkyl(C₁-C₃)amino ou cyclopropyle,
R⁶ représente un atome d'hydrogène ou d'halogène, un groupe OH, NH₂, CN, alkyle en C₁-C₃, alcoxy en C₁-C₃, cyclopropyle ou vinyle,
R⁷ représente un atome d'hydrogène ou d'halogène, un groupe alkyle en C₁-C₃, alcoxy en C₁-C₃, alkyl(C₁-C₃)thio, cyclopropyle, alkyl(C₁-C₃)amino ou phényle,
R⁸ représente un atome d'hydrogène, un groupe alkyle en C₁-C₃, phényle ou alkyl(C₁-C₃)carbonyle,
X représente N, CH, CCl, CF ou CBr,
n représente 0, 1 ou 2.

2. Compositions herbicides, **caractérisées par** une teneur à activité herbicide en au moins un composé de formule (I) selon la revendication 1.

3. Compositions herbicides selon la revendication 2, en mélange avec des adjuvants de formulation.

4. Compositions herbicides selon la revendication 2 ou 3, contenant au moins une autre substance à activité pesticide, choisie dans le groupe constitué par les insecticides, acaricides, herbicides, fongicides, phytoprotecteurs et régulateurs de croissance.

5. Compositions herbicides selon la revendication 4, contenant un phytoprotecteur.

6. Compositions herbicides selon la revendication 5, contenant un autre herbicide.

7. Procédé pour la lutte contre des plantes indésirables, **caractérisé en ce qu'**on applique sur les plantes ou le lieu de la croissance de plantes indésirables une quantité efficace d'au moins un composé de formule (I) selon la revendication 1 ou d'une composition herbicide selon l'une quelconque des revendications 2 à 6.

8. Utilisation de composés de formule (I) selon la revendication 1 ou d'une composition herbicide selon l'une quelconque des revendications 2 à 6, pour la lutte contre des plantes indésirables.

9. Utilisation selon la revendication 8, **caractérisée en ce que** les composés de formule (I) sont utilisés pour la lutte contre des plantes indésirables dans des cultures de plantes utiles.

10. Utilisation selon la revendication 9, **caractérisée en ce que** les plantes utiles sont des plantes utiles transgéniques.
